# EUROPÄISCHE PATENTSCHRIFT

(11) **EP 3 322 819 B1**
(45) Veröffentlichungstag und Bekanntmachung des Hinweises auf die Patenterteilung: **23.09.2020**
(21) Anmeldenummer: 16744662.4
(22) Anmeldetag: 15.07.2016
(51) Int. Cl.: C12Q 1/68, C12Q 1/70, C12Q 1/6827, C12Q 1/6886

(54) **VERFAHREN ZUR BESTIMMUNG EINER MUTATION IN GENOMISCHER DNA, VERWENDUNG DES VERFAHRENS UND KIT ZUR DURCHFÜHRUNG DES VERFAHRENS**
METHOD FOR DETERMINING A MUTATION IN GENOMIC DNA, USE OF THE METHOD AND KIT TO BE USED IN THE METHOD
METHODE POUR LA DETECTION D'UNE MUTATION DANS DE L'ADN GENOMIC, UTILISATION DE LA METHODE ET KIT POUR REALISER LA METHODE

(30) Priorität: 16.07.2015 DE 102015009187
(43) Veröffentlichungstag der Anmeldung: 23.05.2018
(73) Patentinhaber: Dietrich, Dimo, 10437 Berlin (DE)
(72) Erfinder: Dietrich, Dimo, 10437 Berlin (DE)
(74) Vertreter: Andresen, Heiko
(86) Internationale Anmeldenummer: PCT/EP2016/001237
(87) Internationale Veröffentlichungsnummer: WO 2017/008912

(56) Entgegenhaltungen:
- WO-A1-2013/082043
- WO-A2-2013/084075
- DE-A1- 19 951 189
- US-A1- 2005 064 401
- US-A1- 2008 050 738
- US-A1- 2013 338 032
- YAPING LIU ET AL: "Bis-SNP: Combined DNA methylation and SNP calling for Bisulfite-seq data", GENOME BIOLOGY, BIOMED CENTRAL LTD., LONDON, GB, Bd. 13, Nr. 7, 11. Juli 2012 (2012-07-11), Seite R61, XP021133985, ISSN: 1465-6906, DOI: 10.1186/GB-2012-13-7-R61
- LASKAR RUHINA S ET AL: "Association of HPV with genetic and epigenetic alterations in colorectal adenocarcinoma from Indian population", TUMOR BIOLOGY, KARGER, BASEL, CH, Bd. 36, Nr. 6, 3. Februar 2015 (2015-02-03), Seiten 4661-4670, XP035527559, ISSN: 1010-4283, DOI: 10.1007/S13277-015-3114-Y [gefunden am 2015-02-03]

### SEQUENCE LISTING

<110> Dietrich, Dimo
<120> verfahren zur Bestimmung einer Mutation in genomischer DNA, verwendung des verfahrens und Kit zur Durchführung des verfahrens
<130> DD2015-01-PCT
<150> DE 10 2015 009 187.5
   <151> 2015-07-16
<160> 94
<170> Patentin version 3.5
<210> 1
   <211> 23
   <212> DNA
   <213> Artificial
<220>
   <223> Forward primer to amplify unconverted (SEQ ID NO:3) and bisulfite-converted (SEQ ID NO:4) BRAF V600E locus (vorwärtsprimer für Amplifikation des unkonvertierten (SEQ ID NO:3) und Bisulfit-konvertierten (SEQ ID NO:4) BRAF V600E Lokus)
<400> 1
   tcaattctta ccatccacaa aat 23
<210> 2
   <211> 22
   <212> DNA
   <213> Artificial
<220>
   <223> Reverse primer to amplify unconverted (SEQ ID NO:3) and bisulfite-converted (SEQ ID NO:4) BRAF V600E locus (Rückwärtsprimer für Amplifikation des unkonvertierten (SEQ ID NO:3) und Bisulfit-konvertierten (SEQ ID NO:4) BRAF V600E Lokus)
<400> 2
   agtaaaaata ggtgattttg gt 22
<210> 3
   <211> 105
   <212> DNA
   <213> Human BRAF gene locus comprising the V600E mutation site (BRAF Genlokus,
   der die Position der V600E Mutation umfasst)
<400> 3
   tcaattctta ccatccacaa aatggatcca gacaactgtt caaactgatg ggacccactc 60
   catcgagatt tcactgtagc tagaccaaaa tcacctattt ttact 105
<210> 4
   <211> 105
   <212> DNA
   <213> Artificial
<220>
   <223> Bisulfite-converted (bisulfit strand II) BRAF gene locus comprising the V600E mutation site (Bisulfit-konvertierter (Bisulfit strang II) BRAF Genlokus, der die Position der V600E Mutation umfasst)
<400> 4
   tcaattctta ccatccacaa aataaatcca aacaactatt caaactaata aaacccactc 60
   catcgaaatt tcactataac taaaccaaaa tcacctattt ttact 105
<210> 5
   <211> 25
   <212> DNA
   <213> Artificial
<220>
   <223> qPCR detection probe targeting the bisulfite converted BRAF gene locus (SEQ ID NO:4) comprising the V600E mutation site (qPCR Detektions-Sonde, die auf den Bisulfit-konvertierten BRAF Genlokus (SEQ ID NO:4) zielt, der die V600E Mutation umfasst)
<400> 5
   attcaaacta ataaaaccca ctcca 25
<210> 6
   <211> 112
   <212> DNA
   <213> Human SHOX2 gene locus targeted in the course of methylation analyses
   (SHOX2 Genlokus, welcher im Rahmen von Methylierungsanalysen untersucht wurde)
<400> 6
   gtcccctgga cagccaggta atctccgtcc cgcctgcccg accggggtcg cacgagcaca 60
   ggcgcccacg ccatgttggc tgcccaaagg gctcgccgcc caagccgggc ca 112
<210> 7
   <211> 22
   <212> DNA
   <213> Artificial
<220>
   <223> Forward primer used to amplify the bisulfite-converted (SEQ ID NO:10) SHOX2 gene locus (vorwärtsprimer verwendet für die Amplifikation des des Bisulfit-konvertierten (SEQ ID NO:10) SHOX2 Genlokus)
<400> 7
   gttttttgga tagttaggta at 22
<210> 8
   <211> 20
   <212> DNA
   <213> Artificial
<220>
   <223> Reverse primer used to amplify the bisulfite-converted (SEQ ID NO:10) methylated SHOX2 gene locus (Rückwärtsprimer verwendet für die Amplifikation des des Bisulfit-konvertierten (SEQ ID NO:10) methylierten SHOX2 Genlokus)
<400> 8
   taacccgact taaacgacga 20
<210> 9
   <211> 37
   <212> DNA
   <213> Artificial
<220>
   <223> Blocker oligonucleotide preventing forward primer (SEQ ID NO:7) from binding to the bisulfite-converted unmethylated SHOX2 gene locus (Blockeroligonukleotid um den vorwärtsprimer (SEQ ID NO:7) an der Bindung an den Bisulfit-konvertierten (SEQ ID NO:10)
<400> 9
   taatttttgt tttgtttgtt tgattggggt tgtatga 37
<210> 10
   <211> 112
   <212> DNA
   <213> Artificial
<220>
   <223> Bisulfite-converted (bisulfite strand I) SHOX2 gene locus derived from the genomic sequence SEQ ID NO:6 (Bisulfit-konvertierter (Bisulfit Strang I) SHOX2 Genlokus abgeleited von der genomischen Sequenz SEQ ID NO:6)
<400> 10
   gttttttgga tagttaggta attttcgttt cgtttgttcg atcggggtcg tacgagtata 60
   ggcgtttacg ttatgttggt tgtttaaagg gttcgtcgtt taagtcgggt ta 112
<210> 11
   <211> 18
   <212> DNA
   <213> Artificial
<220>
   <223> qPCR detection probe targeting the bisulfite converted methylated SHOX2 gene locus (SEQ ID NO:10) (qPCR Detektions-Sonde, die auf den Bisulfit-konvertierten methylierten SHOX2 Genlokus (SEQ ID NO:10) zielt)
<400> 11
   ctcgtacgac cccgatcg 18
<210> 12
   <211> 24
   <212> DNA
   <213> Artificial
<220>
   <223> Sequencing primer used for Sanger sequencing of the bisulfite-converted BRAF gene locus SEQ ID NO:4 (sequenzierprimer verwendet für die Sanger-Sequenzierung des Bisulfit-konvertierten BRAF Genlokus SEQ ID NO:4)
<400> 12
   cttaccatcc acaaaataaa tcca 24
<210> 13
   <211> 23
   <212> DNA
   <213> Artificial
<220>
   <223> Forward primer used to amplify the unconverted EGFR exon 21 gene locus SEQ ID NO:15 comprising the L858R mutation site (vorwärtsprimer verwendet um den unkonvertierten EGFR Exon 21 Genlokus SEQ ID NO:15 zu amplfizieren, der die L858R Mutation
<400> 13
   gtttcagggc atgaactact tgg 23
<210> 14
   <211> 22
   <212> DNA
   <213> Artificial
<220>
   <223> Reverse primer used to amplify the unconverted EGFR exon 21 gene locus SEQ ID NO:15 comprising the L858R mutation site (Rückwärtsprimer verwendet um den unkonvertierten EGFR Exon 21 Genlokus SEQ ID NO:15 zu amplifizieren, der die L858R Mutation
<400> 14
   cctggtgtca ggaaaatgct gg 22
<210> 15
   <211> 208
   <212> DNA
   <213> Human unconverted EGFR exon 21 gene locus comprising the L858R mutation
   site (unkonvertierter EGFR Exon 21 Genlokus, der die L858R Mutation umfasst)
<400> 15
   gtttcagggc atgaactact tggaggaccg tcgcttggtg caccgcgacc tggcagccag 60
   gaacgtactg gtgaaaacac cgcagcatgt caagatcaca gattttgggc tggccaaact 120
   gctgggtgcg gaagagaaag aataccatgc agaaggaggc aaagtaagga ggtggcttta 180
   ggtcagccag cattttcctg acaccagg 208
<210> 16
   <211> 24
   <212> DNA
   <213> Artificial
<220>
   <223> Forward primer to amplify bisulfite-converted EGFR exon 21 gene locus SEQ ID NO:18 comprising the L858R mutation site (vorwärtsprimer um Bisulfit-konvertierten EGFR Exon 21 Genlokus SEQ ID NO:18 zu amplifizieren, der die L858R Mutation umfasst)
<400> 16
   gttttagggt atgaattatt tgga 24
<210> 17
   <211> 26
   <212> DNA
   <213> Artificial
<220>
   <223> Reverse primer to amplify bisulfite-converted EGFR exon 21 gene locus SEQ ID NO:18 comprising the L858R mutation site (Rückwärtsprimer um Bisulfit-konvertierten EGFR Exon 21 Genlokus SEQ ID NO:18 zu amplifizieren, der die L858R Mutation umfasst)
<400> 17
   ccctaatatc aaaaaaatac taacta 26
<210> 18
   <211> 209
   <212> DNA
   <213> Artificial
<220>
   <223> Bisulfit-converted EGFR exon 21 gene locus (bisulfite strand I) derived from SEQ ID NO:15 comprising the L858R mutation site (Bisulfite-konvertierter EGFR Exon 21 Genlokus (Bisulfit Strang I) abgeleitet von SEQ ID NO:15, der die L858R Mutation umfasst)
<400> 18
   gttttagggt atgaattatt tggaggatcg tcgtttggtg tatcgcgatt tggtagttag 60
   gaacgtattg gtgaaaatat cgtagtatgt taagattata gattttgggt tggttaaatt 120
   gttgggtgcg gaagagaaag aatattatgt agaaggaggt aaagtaagga ggtggtttta 180
   ggttagttag tatttttttg atattaggg 209
<210> 19
   <211> 24
   <212> DNA
   <213> Artificial
<220>
   <223> Forward primer used to amplify the unconverted EGFR exon 19 gene locus SEQ ID NO:21 (vorwärtsprimer verwendet um den unkonvertierten EGFR Exon 19 Genlokus SEQ ID NO:21 zu amplifizieren)
<400> 19
   tctctgtcat agggactctg gatc 24
<210> 20
   <211> 21
   <212> DNA
   <213> Artificial
<220>
   <223> Reverse primer used to amplify the unconverted EGFR exon 19 gene locus SEQ ID No:21 (Rückwärtsprimer verwendet um den unkonvertierten EGFR Exon 19 Genlokus SEQ ID NO:21 zu amplifizieren)
<400> 20
   cctgaggttc agagccatgg a 21
<210> 21
   <211> 159
   <212> DNA
   <213> Human Unconverted EGFR exon 19 gene locus (unkonvertierter EGFR Exon 19
   Genlokus)
<400> 21
   tctctgtcat agggactctg gatcccagaa ggtgagaaag ttaaaattcc cgtcgctatc 60
   aaggaattaa gagaagcaac atctccgaaa gccaacaagg aaatcctcga tgtgagtttc 120
   tgctttgctg tgtgggggtc catggctctg aacctcagg 159
<210> 22
   <211> 26
   <212> DNA
   <213> Artificial
<220>
   <223> Forward primer used to amplify the bisulfite-converted EGFR exon 19 gene locus SEQ ID NO:24 (Vorwärtsprimer verwendet um den Bisulfit-konvertierten EGFR Exon 19 Genlokus SEQ ID NO:24 zu amplifizieren)
<400> 22
   ttttttgtta tagggatttt ggattt 26
<210> 23
   <211> 27
   <212> DNA
   <213> Artificial
<220>
   <223> Reverse primer used to amplify the bisulfite-converted EGFR exon 19 gene locus SEQ ID NO:24 (Rückwärtsprimer verwendet um den Bisulfit-konvertierten EGFR Exon 19 Genlokus SEQ ID NO:24 zu amplifizieren)
<400> 23
   taaacctaaa attcaaaacc ataaacc 27
<210> 24
   <211> 164
   <212> DNA
   <213> Artificial
<220>
   <223> Bisulfit-converted EGFR exon 19 gene locus (bisulfite strand I) derived from SEQ ID NO:21 (Bisulfite-konvertierter EGFR Exon 19 Genlokus (Bisulfit Strang I) abgeleitet von SEQ ID NO:21)
<400> 24
   ttttttgtta tagggatttt ggattttaga aggtgagaaa gttaaaattt tcgtcgttat    60
   taaggaatta agagaagtaa tattttcgaa agttaataag gaaattttcg atgtgagttt  120
   ttgttttgtt gtgtgggggt ttatggtttt gaattttagg ttta                    164
<210> 25
   <211> 28
   <212> DNA
   <213> Artificial
<220>
   <223> Forward primer used to amplify the unconverted KRAS exon 4 gene locus SEQ ID NO:27 (Vorwärtsprimer verwendet um den unkonvertierten KRAS Exon 4 Genlokus SEQ ID NO:27 zu amplifizieren)
<400> 25
   gagagaaaaa ctgatatatt aaatgaca 28
<210> 26
   <211> 25
   <212> DNA
   <213> Artificial
<220>
   <223> Reverse primer used to amplify the unconverted KRAS exon 4 gene locus SEQ ID NO:27 (Rückwärtsprimer verwendet um den unkonvertierten KRAS Exon 4 Genlokus SEQ ID NO:27 zu amplifizieren)
<400> 26
   tttgccttct agaacagtag acaca 25
<210> 27
   <211> 178
   <212> DNA
   <213> Human unconverted KRAS exon 4 gene locus (unkonvertierter KRAS Exon 4
   Genlokus)
<400> 27
   gagagaaaaa ctgatatatt aaatgacata acagttatga ttttgcagaa aacagatctg       60
   tatttatttc agtgttactt acctgtcttg tctttgctga tgtttcaata aaaggaattc      120
   cataacttct tgctaagtcc tgagcctgtt ttgtgtctac tgttctagaa ggcaaatc        178
<210> 28
   <211> 176
   <212> DNA
   <213> Artificial
<220>
   <223> Bisulfit-converted KRAS exon 4 gene locus (bisulfite strand I) derived from SEQ ID NO:27 (Bisulfite-konvertierter KRAS Exon 4 Genlokus (Bisulfit Strang I) abgeleitet von SEQ ID NO:27)
<400> 28
   gagagaaaaa ttgatatatt aaatgatata atagttatga ttttgtagaa aatagatttg       60
   tatttatttt agtgttattt atttgttttg tttttgttga tgttttaata aaaggaattt      120
   tataattttt tgttaagttt tgagtttgtt ttgtgtttat tgttttagaa ggtaaa          176
<210> 29
   <211> 28
   <212> DNA
   <213> Artificial
<220>
   <223> Forward primer used to amplify the bisulfite-converted KRAS exon 4 gene locus SEQ ID NO:28 (Vorwärtsprimer verwendet um den Bisulfit-konvertierten KRAS Exon 4 Genlokus SEQ ID NO:28 zu amplifizieren)
<400> 29
   gagagaaaaa ttgatatatt aaatgata 28
<210> 30
   <211> 25
   <212> DNA
   <213> Artificial
<220>
   <223> Reverse primer used to amplify the bisulfite-converted KRAS exon 4 gene locus SEQ ID NO:28 (Rückwärtsprimer verwendet um den Bisulfit-konvertierten KRAS Exon 4 Genlokus SEQ ID NO:28 zu amplifizieren)
<400> 30
   tttaccttct aaaacaataa acaca 25
<210> 31
   <211> 168
   <212> DNA
   <213> Artificial
<220>
   <223> Bisulfit-converted KRAS exon 4 gene locus (bisulfite strand II) derived from SEQ ID NO:27 (Bisulfite-konvertierter KRAS Exon 4 Genlokus (Bisulfit Strang II) abgeleitet von SEQ ID NO:27)
<400> 31
   gatttgtttt ttagaatagt agatataaaa taggtttagg atttagtaag aagttatgga 60
   atttttttta ttgaaatatt agtaaagata agataggtaa gtaatattga aataaatata 120
   gatttgtttt ttgtaaaatt ataattgtta tgttatttaa tatattag 168
<210> 32
   <211> 26
   <212> DNA
   <213> Artificial
<220>
   <223> Forward primer used to amplify the bisulfite-converted KRAS exon 4 gene locus SEQ ID NO:31 (vorwärtsprimer verwendet um den Bisulfit-konvertierten KRAS Exon 4 Genlokus SEQ ID NO:31 zu amplifizieren)
<400> 32
   gatttgtttt ttagaatagt agatat 26
<210> 33
   <211> 29
   <212> DNA
   <213> Artificial
<220>
   <223> Reverse primer used to amplify the bisulfite-converted KRAS exon 4 gene locus SEQ ID NO:31 (Rückwärtsprimer verwendet um den Bisulfit-konvertierten KRAS Exon 4 Genlokus SEQ ID NO:31 zu amplifizieren)
<400> 33
   ctaatatatt aaataacata acaattata 29
<210> 34
   <211> 477
   <212> DNA
   <213> Human BRCA2 region of interest (ROI) 1 (BRCA2 "Region of interest" (ROI)
   1)
<400> 34
   tatgccttaa caaaagtaat ccatagtcaa gatcttaagc atttttttcc ttatgatctt 60
   taactgttct gggtcacaaa tttgtctgtc actggttaaa actaaggtgg gatttttttt 120
   ttaaatagat ttaggaccaa taagtcttaa ttggtttgaa gaactttctt cagaagctcc 180
   accctataat tctgaacctg cagaagaatc tgaacataaa aacaacaatt acgaaccaaa 240
   cctatttaaa actccacaaa ggaaaccatc ttataatcag ctggcttcaa ctccaataat 300
   attcaaagag caagggctga ctctgccgct gtaccaatct cctgtaaaag aattagataa 360
   attcaaatta gacttaggta agtaatgcaa tatggtagac tggggagaac tacaaactag 420
   gaatttaggc aaacctgtgt taaaatctta gctcattcat taattgtgtc atgctgg 477
<210> 35
   <211> 555
   <212> DNA
   <213> Human BRCA2 region of interest (ROI) 2 (BRCA2 "Region of Interest" (ROI)
   2)
<400> 35
   tcttagaata ctagaaatgt taataaaaat aaaacttaac aattttcccc tttttttacc    60
   cccagtggta tgtgggagtt tgtttcatac accaaagttt gtgaaggtaa atattctacc   120
   tggtttattt ttatgactta gtaattgaga atttgacaat agcgttatac ctttgccctg   180
   agatttacaa atctgtacct agcattctgc ctcatacagg caattcagta aacgttaagt   240
   gaaataaaga gtgaatgaaa aaataatatc cttaatgatc agggcatttc tataaaaaat   300
   aaactatttt ctttcctccc agggtcgtca gacaccaaaa catatttctg aaagtctagg   360
   agctgaggtg gatcctgata tgtcttggtc aagttcttta gctacaccac ccacccttag   420
   ttctactgtg ctcataggta ataatagcaa atgtgtattt acaagaaaga gcagatgagg   480
   ttgataattg tcatctctaa tacttctgtt aaaaggaaat atgaaaagaa aatattagat   540
   aatgtctttg ataag                                                    555
<210> 36
   <211> 212
   <212> DNA
   <213> Human BRCA2 region of interest (ROI) 3 (BRCA2 "Region of interest" (ROI)
   3)
<400> 36
   tgtctgacaa aaaataagtt tttgcattct agtgataata tacaatacac ataaattttt    60
   atcttacagt cagaaatgaa gaagcatctg aaactgtatt tcctcatgat actactgctg   120
   taagtaaata tgacattgat tagactgttg aaattgctaa caattttgga atgccttgtt   180
   aaattattta tcttacattt ttaatttcct aa                                 212
<210> 37
   <211> 216
   <212> DNA
   <213> Human BRCA2 region of interest (ROI) 4 (BRCA2 "Region of Interest" (ROI)
   4)
<400> 37
   actataattt ttgcagaatg tgaaaagcta tttttccaat catgatgaaa gtctgaagaa    60
   aaatgataga tttatcgctt ctgtgacaga cagtgaaaac acaaatcaaa gagaagctgc   120
   aagtcatggt aagtcctctg tttagttgaa ctacaggttt ttttgttgtt gttgttttga   180
   tttttttttt ttgaggtgga gtcttgctct gtcacc                             216
<210> 38
   <211> 1199
   <212> DNA
   <213> Human BRCA2 region of interest (ROI) 5 (BRCA2 "Region of Interest" (ROI)
   5)
<400> 38
   gttgtgagaa taatataaat tatatggctt ataaaatatt aatgtgcttc tgttttatac      60
   tttaacagga tttggaaaaa catcagggaa ttcatttaaa gtaaatagct gcaaagacca     120
   cattggaaag tcaatgccaa atgtcctaga agatgaagta tatgaaacag ttgtagatac     180
   ctctgaagaa gatagttttt cattatgttt ttctaaatgt agaacaaaaa atctacaaaa     240
   agtaagaact agcaagacta ggaaaaaaat tttccatgaa gcaaacgctg atgaatgtga     300
   aaaatctaaa aaccaagtga aagaaaaata ctcatttgta tctgaagtgg aaccaaatga     360
   tactgatcca ttagattcaa atgtagcaaa tcagaagccc tttgagagtg gaagtgacaa     420
   aatctccaag gaagttgtac cgtctttggc ctgtgaatgg tctcaactaa ccctttcagg     480
   tctaaatgga gcccagatgg agaaaatacc cctattgcat atttcttcat gtgaccaaaa     540
   tatttcagaa aaagacctat tagacacaga gaacaaaaga aagaaagatt ttcttacttc     600
   agagaattct ttgccacgta tttctagcct accaaaatca gagaagccat taaatgagga     660
   aacagtggta aataagagag atgaagagca gcatcttgaa tctcatacag actgcattct     720
   tgcagtaaag caggcaatat ctggaacttc tccagtggct tcttcatttc agggtatcaa     780
   aaagtctata ttcagaataa gagaatcacc taaagagact ttcaatgcaa gtttttcagg     840
   tcatatgact gatccaaact ttaaaaaaga aactgaagcc tctgaaagtg gactggaaat     900
   acatactgtt tgctcacaga aggaggactc cttatgtcca aatttaattg ataatggaag     960
   ctggccagcc accaccacac agaattctgt agctttgaag aatgcaggtt taatatccac    1020
   tttgaaaaag aaaacaaata agtttattta tgctatacat gatgaaacat cttataaagg    1080
   aaaaaaaata ccgaaagacc aaaaatcaga actaattaac tgttcagccc agtttgaagc    1140
   aaatgctttt gaagcaccac ttacatttgc aaatgctgat tcaggtacct ctgtctttt     1199
<210> 39
   <211> 4944
   <212> DNA
   <213> Human BRCA2 region of interest (ROI) 6, preferred for methylation
   analyses (BRCA2 "Region of Interest" (ROI) 6, präferiert für Methylierungsanalysen)
<400> 39
   ttttatgttt aggtttattg cattcttctg tgaaaagaag ctgttcacag aatgattctg      60
   aagaaccaac tttgtcctta actagctctt ttgggacaat tctgaggaaa tgttctagaa     120
   atgaaacatg ttctaataat acagtaatct ctcaggatct tgattataaa gaagcaaaat     180
   gtaataagga aaaactacag ttatttatta ccccagaagc tgattctctg tcatgcctgc     240
   aggaaggaca gtgtgaaaat gatccaaaaa gcaaaaaagt ttcagatata aaagaagagg     300
   tcttggctgc agcatgtcac ccagtacaac attcaaaagt ggaatacagt gatactgact     360
   ttcaatccca gaaaagtctt ttatatgatc atgaaaatgc cagcactctt attttaactc     420
   ctacttccaa ggatgttctg tcaaacctag tcatgatttc tagaggcaaa gaatcataca 480
   aaatgtcaga caagctcaaa ggtaacaatt atgaatctga tgttgaatta accaaaaata 540
   ttcccatgga aaagaatcaa gatgtatgtg ctttaaatga aaattataaa aacgttgagc 600
   tgttgccacc tgaaaaatac atgagagtag catcaccttc aagaaaggta caattcaacc 660
   aaaacacaaa tctaagagta atccaaaaaa atcaagaaga aactacttca atttcaaaaa 720
   taactgtcaa tccagactct gaagaacttt tctcagacaa tgagaataat tttgtcttcc 780
   aagtagctaa tgaaaggaat aatcttgctt taggaaatac taaggaactt catgaaacag 840
   acttgacttg tgtaaacgaa cccattttca agaactctac catggtttta tatggagaca 900
   caggtgataa acaagcaacc caagtgtcaa ttaaaaaaga tttggtttat gttcttgcag 960
   aggagaacaa aaatagtgta aagcagcata taaaaatgac tctaggtcaa gatttaaaat 1020
   cggacatctc cttgaatata gataaaatac cagaaaaaaa taatgattac atgaacaaat 1080
   gggcaggact cttaggtcca atttcaaatc acagttttgg aggtagcttc agaacagctt 1140
   caaataagga aatcaagctc tctgaacata acattaagaa gagcaaaatg ttcttcaaag 1200
   atattgaaga acaatatcct actagtttag cttgtgttga aattgtaaat accttggcat 1260
   tagataatca aaagaaactg agcaagcctc agtcaattaa tactgtatct gcacatttac 1320
   agagtagtgt agttgtttct gattgtaaaa atagtcatat aacccctcag atgttatttt 1380
   ccaagcagga ttttaattca aaccataatt taacacctag ccaaaaggca gaaattacag 1440
   aactttctac tatattagaa gaatcaggaa gtcagtttga atttactcag tttagaaaac 1500
   caagctacat attgcagaag agtacatttg aagtgcctga aaaccagatg actatcttaa 1560
   agaccacttc tgaggaatgc agagatgctg atcttcatgt cataatgaat gccccatcga 1620
   ttggtcaggt agacagcagc aagcaatttg aaggtacagt tgaaattaaa cggaagtttg 1680
   ctggcctgtt gaaaaatgac tgtaacaaaa gtgcttctgg ttatttaaca gatgaaaatg 1740
   aagtggggtt taggggcttt tattctgctc atggcacaaa actgaatgtt tctactgaag 1800
   ctctgcaaaa agctgtgaaa ctgtttagtg atattgagaa tattagtgag gaaacttctg 1860
   cagaggtaca tccaataagt ttatcttcaa gtaaatgtca tgattctgtt gtttcaatgt 1920
   ttaagataga aaatcataat gataaaactg taagtgaaaa aaataataaa tgccaactga 1980
   tattacaaaa taatattgaa atgactactg gcacttttgt tgaagaaatt actgaaaatt 2040
   acaagagaaa tactgaaaat gaagataaca aatatactgc tgccagtaga aattctcata 2100
   acttagaatt tgatggcagt gattcaagta aaaatgatac tgtttgtatt cataaagatg 2160
   aaacggactt gctatttact gatcagcaca acatatgtct taaattatct ggccagttta 2220
   tgaaggaggg aaacactcag attaaagaag atttgtcaga tttaactttt ttggaagttg 2280
   cgaaagctca agaagcatgt catggtaata cttcaaataa agaacagtta actgctacta 2340
   aaacggagca aaatataaaa gattttgaga cttctgatac attttttcag actgcaagtg 2400
   ggaaaaatat tagtgtcgcc aaagagtcat ttaataaaat tgtaaatttc tttgatcaga 2460
   aaccagaaga attgcataac ttttccttaa attctgaatt acattctgac ataagaaaga    2520
   acaaaatgga cattctaagt tatgaggaaa cagacatagt taaacacaaa atactgaaag    2580
   aaagtgtccc agttggtact ggaaatcaac tagtgacctt ccagggacaa cccgaacgtg    2640
   atgaaaagat caaagaacct actctattgg gttttcatac agctagcggg aaaaaagtta    2700
   aaattgcaaa ggaatctttg gacaaagtga aaaacctttt tgatgaaaaa gagcaaggta    2760
   ctagtgaaat caccagtttt agccatcaat gggcaaagac cctaaagtac agagaggcct    2820
   gtaaagacct tgaattagca tgtgagacca ttgagatcac agctgcccca aagtgtaaag    2880
   aaatgcagaa ttctctcaat aatgataaaa accttgtttc tattgagact gtggtgccac    2940
   ctaagctctt aagtgataat ttatgtagac aaactgaaaa tctcaaaaca tcaaaaagta    3000
   tctttttgaa agttaaagta catgaaaatg tagaaaaaga aacagcaaaa agtcctgcaa    3060
   cttgttacac aaatcagtcc ccttattcag tcattgaaaa ttcagcctta gctttttaca    3120
   caagttgtag tagaaaaact tctgtgagtc agacttcatt acttgaagca aaaaaatggc    3180
   ttagagaagg aatatttgat ggtcaaccag aaagaataaa tactgcagat tatgtaggaa    3240
   attatttgta tgaaaataat tcaaacagta ctatagctga aaatgacaaa aatcatctct    3300
   ccgaaaaaca agatacttat ttaagtaaca gtagcatgtc taacagctat tcctaccatt    3360
   ctgatgaggt atataatgat tcaggatatc tctcaaaaaa taaacttgat tctggtattg    3420
   agccagtatt gaagaatgtt gaagatcaaa aaaacactag tttttccaaa gtaatatcca    3480
   atgtaaaaga tgcaaatgca tacccacaaa ctgtaaatga agatatttgc gttgaggaac    3540
   ttgtgactag ctcttcaccc tgcaaaaata aaaatgcagc cattaaattg tccatatcta    3600
   atagtaataa ttttgaggta gggccacctg catttaggat agccagtggt aaaatcgttt    3660
   gtgtttcaca tgaaacaatt aaaaaagtga aagacatatt tacagacagt ttcagtaaag    3720
   taattaagga aaacaacgag aataaatcaa aaatttgcca aacgaaaatt atggcaggtt    3780
   gttacgaggc attggatgat tcagaggata ttcttcataa ctctctagat aatgatgaat    3840
   gtagcacgca ttcacataag gtttttgctg acattcagag tgaagaaatt ttacaacata    3900
   accaaaatat gtctggattg gagaaagttt ctaaaatatc accttgtgat gttagtttgg    3960
   aaacttcaga tatatgtaaa tgtagtatag ggaagcttca taagtcagtc tcatctgcaa    4020
   atacttgtgg gatttttagc acagcaagtg gaaaatctgt ccaggtatca gatgcttcat    4080
   tacaaaacgc aagacaagtg ttttctgaaa tagaagatag taccaagcaa gtcttttcca    4140
   aagtattgtt taaaagtaac gaacattcag accagctcac aagagaagaa aatactgcta    4200
   tacgtactcc agaacattta atatcccaaa aaggcttttc atataatgtg gtaaattcat    4260
   ctgctttctc tggatttagt acagcaagtg gaaagcaagt ttccatttta gaaagttcct    4320
   tacacaaagt taagggagtg ttagaggaat ttgatttaat cagaactgag catagtcttc    4380
   actattcacc tacgtctaga caaaatgtat caaaaatact tcctcgtgtt gataagagaa    4440
   acccagagca ctgtgtaaac tcagaaatgg aaaaaacctg cagtaaagaa tttaaattat    4500
   caaataactt aaatgttgaa ggtggttctt cagaaaataa tcactctatt aaagtttctc 4560
   catatctctc tcaatttcaa caagacaaac aacagttggt attaggaacc aaagtgtcac 4620
   ttgttgagaa cattcatgtt ttgggaaaag aacaggcttc acctaaaaac gtaaaaatgg 4680
   aaattggtaa aactgaaact ttttctgatg ttcctgtgaa aacaaatata gaagtttgtt 4740
   ctacttactc caaagattca gaaaactact ttgaaacaga agcagtagaa attgctaaag 4800
   cttttatgga agatgatgaa ctgacagatt ctaaactgcc aagtcatgcc acacattctc 4860
   tttttacatg tcccgaaaat gaggaaatgg ttttgtcaaa ttcaagaatt ggaaaaagaa 4920
   gaggagagcc ccttatctta gtgg 4944
<210> 40
   <211> 498
   <212> DNA
   <213> Human BRCA2 region of interest (ROI) 7 (BRCA2 "Region of interest" (ROI)
   7)
<400> 40
   atttatatgt gtactagtca ataaacttat atattttctc cccattgcag cacaactaag 60
   gaacgtcaag agatacagaa tccaaatttt accgcacctg gtcaagaatt tctgtctaaa 120
   tctcatttgt atgaacatct gactttggaa aaatcttcaa gcaatttagc agtttcagga 180
   catccatttt atcaagtttc tgctacaaga aatgaaaaaa tgagacactt gattactaca 240
   ggcagaccaa ccaaagtctt tgttccacct tttaaaacta aatcacattt tcacagagtt 300
   gaacagtgtg ttaggaatat taacttggag gaaaacagac aaaagcaaaa cattgatgga 360
   catggctctg atgatagtaa aaataagatt aatgacaatg agattcatca gtttaacaaa 420
   aacaactcca atcaagcagt agctgtaact ttcacaaagt gtgaagaaga acctttaggt 480
   attgtatgac aatttgtg 498
<210> 41
   <211> 271
   <212> DNA
   <213> Human BRCA2 region of interest (ROI) 8 (BRCA2 "Region of Interest" (ROI)
   8)
<400> 41
   aaatatgcat ttttgttttc acttttagat atgatacgga aattgataga agcagaagat 60
   cggctataaa aaagataatg gaaagggatg acacagctgc aaaaacactt gttctctgtg 120
   tttctgacat aatttcattg agcgcaaata tatctgaaac ttctagcaat aaaactagta 180
   gtgcagatac ccaaaaagtg gccattattg aacttacaga tgggtggtat gctgttaagg 240
   cccagttaga tcctcccctc ttagctgtct t 271
<210> 42
   <211> 444
   <212> DNA
   <213> Human BRCA2 region of interest (ROI) 9 (BRCA2 "Region of Interest" (ROI)
   9)
<400> 42
   ccatggaatc tgctgaacaa aaggaacaag gtttatcaag ggatgtcaca accgtgtgga     60
   agttgcgtat tgtaagctat tcaaaaaaag aaaaagattc aggtaagtat gtaaatgctt    120
   tgtttttatc agttttatta acttaaaaaa tgaccttact aacaaaatga ttataaatcc    180
   agataaagta taaagttagt ttatatcaga gaagcaaaat ccactactaa tgcccacaaa    240
   gagataatat aaaagaggat ctgtatttat tttgaaacaa acatttaaat gataatcact    300
   tcttccattg catctttctc atctttctcc aaacagttat actgagtatt tggcgtccat    360
   catcagattt atattctctg ttaacagaag gaaagagata cagaatttat catcttgcaa    420
   cttcaaaatc taaaagtaaa tctg                                           444
<210> 43
   <211> 138
   <212> DNA
   <213> Human BRCA2 region of interest (ROI) 10 (BRCA2 "Region of interest" (ROI)
   10)
<400> 43
   catctaacac atctataata acattctttt cttttttttc cattctagga cttgcccctt      60
   tcgtctattt gtcagacgaa tgttacaatt tactggcaat aaagttttgg atagacctta    120
   atgaggacat tattaagc                                                  138
<210> 44
   <211> 252
   <212> DNA
   <213> Human BRCA2 region of interest (ROI) 11 (BRCA2 "Region of Interest" (ROI)
   11)
<400> 44
   gtgggtttgc aatttataaa gcagcttttc cacttatttt cttagaatat tgacatactt     60
   tgcaatgaag cagaaaacaa gcttatgcat atactgcatg caaatgatcc caagtggtcc    120
   accccaacta aagactgtac ttcagggccg tacactgctc aaatcattcc tggtacagga    180
   aacaagcttc tggtaagtta atgtaaactc aaggaatatt ataagaagta tatatggagg    240
   ccatcgtata tt                                                        252
<210> 45
   <211> 366
   <212> DNA
   <213> Human BRCA2 region of interest (ROI) 12 (BRCA2 "Region of interest" (ROI)
<400> 45
   ttactacata attatgatag gctacgtttt cattttttta tcagatgtct tctcctaatt      60
   gtgagatata ttatcaaagt cctttatcac tttgtatggc caaaaggaag tctgtttcca     120
   cacctgtctc agcccagatg acttcaaagt cttgtaaagg ggagaaagag attgatgacc     180
   aaaagaactg caaaaagaga agagccttgg atttcttgag tagactgcct ttacctccac     240
   ctgttagtcc catttgtaca tttgtttctc cggctgcaca gaaggcattt cagccaccaa     300
   ggagttgtgg caccaaatac gaaacaccca taaagaaaaa agaactgaat tctcctcaga     360
   tgactc                                                                366
<210> 46
   <211> 337
   <212> DNA
   <213> Human BRCA1 region of interest (ROI) 1 (BRCA1 "Region of Interest" (ROI)
   1)
<400> 46
   aggagctccc agggcctgga aaggccactt tgtaagctca ttcttggggt cctgtggctc      60
   tgtacctgtg gctggctgca gtcagtagtg gctgtggggg atctggggta tcaggtaggt     120
   gtccagctcc tggcactggt agagtgctac actgtccaac acccactctc gggtcaccac     180
   aggtgcctca cacatctgcc caattgctgg agacagagaa cacaagcaga gattagtgtc     240
   aattcattct cctggactag gctctaatca atcgactcca gggtcctggt tgtatgagtt     300
   cttaggatta atgaggtaga agctaatttt ttttttt                              337
<210> 47
   <211> 278
   <212> DNA
   <213> Human BRCA1 region of interest (ROI) 2 (BRCA1 "Region of Interest" (ROI)
   2)
<400> 47
   tgtgcagttc tcaaatcctt acccatccct tacagatgga gtcttttggc acaggtatgt      60
   gggcagagaa gacttctgag gctacagtag gggcatccat agggactgac aggtgccagt     120
   cttgctcaca ggagagaata ttgtgtcctc cctctctgac agggcaccca atacttactg     180
   tgccaagggt gaatgatgaa agctccttca ccacagaagc accacacagc tgtaccatcc     240
   attccagttg atctaaaatg gacatttaga tgtaaaat                             278
<210> 48
   <211> 150
   <212> DNA
   <213> Human BRCA1 region of interest (ROI) 3 (BRCA1 "Region of Interest" (ROI)
   3)
<400> 48
   tgcttataat actccactat gtaagacaaa ggctggtgct ggaactctgg ggttctccca 60
   ggctcttacc tgtgggcatg ttggtgaagg gcccatagca acagatttct agccccctga 120
   agatctggaa gaagagagga agagagaggg 150
<210> 49
   <211> 128
   <212> DNA
   <213> Human BRCA1 region of interest (ROI) 4 (BRCA1 "Region of interest" (ROI)
   4)
<400> 49
   aaggggagtg gaatacagag tggtggggtg agatttttgt caacttgagg gagggagctt 60
   tacctttctg tcctgggatt ctcttgctcg ctttggacct tggtggtttc ttccattgac 120
   cacatctc 128
<210> 50
   <211> 704
   <212> DNA
   <213> Human BRCA1 region of interest (ROI) 5 (BRCA1 "Region of Interest" (ROI)
   5)
<400> 50
   atatgactga atgaatatct ctggttagtt tgtaacatca agtacttacc tcattcagca 60
   tttttctttc tttaatagac tgggtcaccc ctaaagagat catagaaaag acaggttaca 120
   tacagcagaa gaacgtgctc ttttcacgga gatagagagg tcagcgattc acaaaagagc 180
   acaggaagaa tgacagagga gaggtccttc cctctaaagc cacagccctt taataaggct 240
   tgtagcagca gtttccttct ggagacagag ttgatgttta atttaaacat tataagtttg 300
   cctgctgcac atggattcct gccgactatt aaataaatcc ctagctcata tgctaacatt 360
   gctaggagca gattaggtcc tattagttat aaaagagacc cattttccca gcatcaccag 420
   cttatctgaa caaagtgata ttaaagataa aagtagttta gtattacaat taaagacctt 480
   ttggtaactc agactcagca tcagcaaaaa ccttaggtgt taaacgttag gtgtaaaaat 540
   gcaattctga ggtgttaaag ggaggagggg agaaatagta ttatacttac agaaatagct 600
   aactacccat tttcctcccg caattcctag aaaatatttc agtgtccgtt cacacacaaa 660
   ctcagcatct gcagaatgaa aaacactcaa aggattagaa gttg 704
<210> 51
   <211> 243
   <212> DNA
   <213> Human BRCA1 region of interest (ROI) 6 (BRCA1 "Region of Interest" (ROI)
   6)
<400> 51
   tacaggcatg cgccaccgtg cctcgcctca tgtggtttta tgcagcagat gcaaggtatt     60
   ctgtaaaggt tcttggtata cctgttttca taacaacatg agtagtctct tcagtaatta    120
   gattagttaa agtgatgtgg tgttttctgg caaacttgta cacgagcatc tgaaattaaa    180
   tcaaatattc cattatcatg agttacctct agcacacagc tcagaatact agttattcca    240
   cca                                                                  243
<210> 52
   <211> 306
   <212> DNA
   <213> Human BRCA1 region of interest (ROI) 7 (BRCA1 "Region of interest" (ROI)
   7)
<400> 52
   acctacataa aactctttcc agaatgttgt taagtcttag tcattaggga gatacatatg     60
   gatacactca caaattcttc tggggtcagg ccagacacca ccatggacat tcttttgttg    120
   accctttctg ttgaagctgt caattctggc ttctccctgc tcacactttc ttccattgca    180
   ttatacccag cagtatcagt agtatgagca gcagctggac tctgggcaga ttctgcaact    240
   ttcaattggg gaactttcaa tgcagaggtt gaagatggta tgttgccaac acgagctgac    300
   tctggg                                                               306
<210> 53
   <211> 346
   <212> DNA
   <213> Human BRCA1 region of interest (ROI) 8 (BRCA1 "Region of interest" (ROI)
   8)
<400> 53
   ttaagtataa caaaagtgtc catgatagac tagtacatct aaaagttggt taaccagaat     60
   atctttatgt aggattcaga gtaaaatcaa agtgtttgtt ccaatacagc agatgaaata    120
   ttacctagat cttgccttgg caagtaagat gtttccgtca aatcgtgtgg cccagactct    180
   tccagctgtt gctcctccac atcaacaacc ttaatgagct cctcttgaga tgggtagttt    240
   ctattctgaa gactcccaga gcaactgtgc atgtaccacc tatcatctaa tgatgggcat    300
   ttagaagggg atgacctaga aagataaatg gaaggagaaa accatc                   346
<210> 54
   <211> 386
   <212> DNA
   <213> Human BRCA1 region of interest (ROI) 9 (BRCA1 "Region of interest" (ROI)
   9)
<400> 54
   tcatgttgta gcttatgtta taggttcaaa aaacctatat aggattaaac aaaagaagta      60
   tcctagagca ataaaagtgt ataaatgcct gtatgcaaaa aactggagaa agtatggtga     120
   aaaaaattaa caatcagagt tcaatataaa taaagatgtc agataccaca gcatctttac     180
   attgatgttt cttacctttc cactcctggt tctttatttt tactggtaga actatctgca     240
   gacacctcaa acttgtcagc agaaaggcct tctggattct ggcttatagg gtattcacta     300
   cttttctgtg aagttaatac tgctttaaat ggaatgagaa aacaaatcta ctttactgct     360
   ttgttctgat agtgataatt caggtt                                          386
<210> 55
   <211> 262
   <212> DNA
   <213> Human BRCA1 region of interest (ROI) 10 (BRCA1 "Region of interest" (ROI)
   10)
<400> 55
   taggtcctta ctcttcagaa ggagataaag gggaaggaaa gaattttgct taagatatca      60
   gtgtttggcc aacaatacac acctttttct gatgtgcttt gttctggatt tcgcaggtcc     120
   tcaagggcag aagagtcact tatgatggaa gggtagctgt tagaaggctg gctcccatgc     180
   tgttctaaca cagcttctag ttcagccatt tcctgctgga gctttatcag gttatgttgc     240
   atggtatccc tctgcttcaa aa                                              262
<210> 56
   <211> 3899
   <212> DNA
   <213> Human BRCA1 region of interest (ROI) 11, preferred for mutation analyses
   (BRCA1 "Region of Interest" (ROI) 11, präferiert für Mutationsanalysen)
<400> 56
   accacacaca cgcatgtgca cacacacaca cgctttttac ctgagtggtt aaaatgtcac      60
   tctgagagga tagccctgag cagtcttcag agacgcttgt ttcactctca cacccagatg     120
   ctgcttcacc ttaaataaca aaaacagagg ttcagatgta aaagcagact ataaacgctg     180
   caacttgctg tgtctttttc ttctcattgg caggactgga tttactttca tgtcacacaa     240
   aatgattaaa ttccttgctt tgggacacct ggatttgctt ttataaaatg aaaccagaag     300
   taagtccacc agtaattagg atgttaaagc tcattcagtc aaagatgacg tcctagctgt     360
   gtgaaggact tttttctatg aaaagcacct taggaggaac atgtttcaag tttaagaagc     420
   agttccttta actatacttg gaaatttgta aaatgtgctc cccaaaagca taaacattta     480
   gctcacttct ataaatagac tggggcaaac acaaaaacct ggttccaata cctaagtttg     540
   aatccatgct ttgctcttct tgattatttt cttccaagcc cgttcctctt tcttcatcat     600
   ctgaaaccaa ttccttgtca ctcagaccaa ctccctggct ttcagactga tgcctcattt     660
   gtttggaaga accaatcaag aaaggatcct gggtgtttgt atttgcagtc aagtcttcca     720
   attcactgca ctgtgaagaa aacaagctag cagaacattt tgtttcctca ctaaggtgat     780
   gttcctgaga tgcctttgcc aatattacct ggttactgca gtcatttaag ctattcttca     840
   atgataataa attctcctct gtgttcttag acagacactc ggtagcaacg gtgctatgcc     900
   tagtagactg agaaggtata ttgtttactt taccaaataa caagtgttgg aagcagggaa     960
   gctcttcatc ctcactagat aagttctctt ctgaggactc taatttcttg gcccctcttc    1020
   ggtaaccctg agccaaatgt gtatgggtga aagggctagg actcctgcta agctctcctt    1080
   tctggacgct tttgctaaaa acagcagaac tttccttaat gtcattttca gcaaaactag    1140
   tatcttcctt tatttcacca tcatctaaca ggtcatcagg tgtctcagaa caaacctgag    1200
   atgcatgact acttcccata ggctgttcta agttatctga aatcagatat ggagagaaat    1260
   ctgtattaac agtctgaact acttcttcat attcttgctt ttttatttca ggatgcttac    1320
   aattacttcc aggaagactt tgtttataga cctcaggttg caaaacccct aatctaagca    1380
   tagcattcaa ttttggccct ctgtttctac ctagttctgc ttgaatgttt tcatcactgg    1440
   aacctatttc attaatactg gagcccactt cattagtact ggaacctact tcattaatat    1500
   tgcttgagct ggcttcttta aaaacatttt ctctaatgtt attacggcta attgtgctca    1560
   ctgtacttgg aatgttctca tttcccattt ctctttcagg tgacattgaa tgttcctcaa    1620
   agttttcctc tagcagattt ttcttacatt tagttttaac aaatgacttg atgggaaaaa    1680
   gtggtggtat acgatatggg ttttgtaaaa gtccatgttt atttggagta atgagtccag    1740
   tttcgttgcc tctgaactga gatgatagac aaaacctaga gcctcctttg atactacatt    1800
   tggcattatc aactggctta tctttctgac caaccacagg aaagcctgca gtgatattaa    1860
   ctgtctgtac aggcttgata ttagactcat tctttccttg attttcttcc ttttgttcac    1920
   attcaaaagt gacttttgga ctttgtttct ttaaggaccc agagtgggca gagaatgttg    1980
   cacattcctc ttctgcattt cctggatttg aaaacggagc aaatgactgg cgctttgaaa    2040
   ccttgaatgt attctgcaaa tactgagcat caagttcact ttcttccatt tctatgcttg    2100
   tttcccgact gtggttaact tcatgtccca atggatactt aaagccttct gtgtcatttc    2160
   tattatcttt ggaacaacca tgaattagtc ccttggggtt ttcaaatgct gcacactgac    2220
   tcacacattt atttggttct gtttttgcct tccctagagt gctaacttcc agtaacgaga    2280
   tactttcctg agtgccataa tcagtaccag gtaccaatga aatactgcta ctctctacag    2340
   atctttcagt ttgcaaaacc ctttctccac ttaacatgag atctttgggg tcttcagcat    2400
   tattagacac tttaactgtt tctagtttct cttctttttc ttctcttgga aggctaggat    2460
   tgacaaattc tttaagttca ctggtatttg aacacttagt aaaagaacca ggtgcatttg    2520
   ttaacttcag ctctgggaaa gtatcgctgt catgtctttt acttgtctgt tcatttggct    2580
   tgttactctt cttggctcca gttgcaggtt ctttaccttc catgagttgt aggtttctgc    2640
   tgtgcctgac tggcatttgg ttgtactttt ttttctttat ctcttcactg ctagaacaac    2700
   tatcaatttg caattcagta caattaggtg ggcttagatt tctactgact actagttcaa    2760
   gcgcatgaat atgcctggta gaagacttcc tcctcagcct attcttttta ggtgcttttg     2820
   aattgtggat atttaattcg agttccatat tgcttatact gctgcttata ggttcagctt    2880
   tcgttttgaa agcagattct ttttcgagtg attctattgg gttaggattt ttctcattct    2940
   gaatagaatc accttttgtt ttattctcat gaccactatt agtaatattc atcacttgac     3000
   cattctgctc cgtttggtta gttccctgat ttatcatttc aggagtcttt tgaactgcca    3060
   aatctgcttt cttgataaaa tcctcaggat gaaggcctga tgtaggtctc cttttacgct    3120
   ttaatttatt tgtgagggga cgctcttgta ttatctgtgg ctcagtaaca aatgctccta    3180
   taattagatt ttcagttaca tggcttaagt tggggaggct tgccttcttc cgataggttt    3240
   tcccaaatat tttgtcttca atattactct ctactgattt ggagtgaact ctttcacttt    3300
   tacatattaa agcctcatga ggatcactgg ccagtaagtc tattttctct gaagaaccag    3360
   aatattcatc tacctcattt agaacgtcca atacatcagc tactttggca tttgattcag    3420
   actccccatc atgtgagtca tcagaaccta acagttcatc acttctggaa aaccactcat    3480
   taactttctg aatgctgcta tttagtgtta tccaaggaac atcttcagta tctctaggat    3540
   tctctgagca tggcagtttc tgcttattcc attcttttct ctcacacagg ggatcagcat    3600
   tcagatctac ctttttttct gtgctgggag tccgcctatc attacatgtt tccttacttc     3660
   cagcccatct gttatgttgg ctccttgcta agccaggctg tttgctttta ttacagaatt    3720
   cagccttttc tacattcatt ctgtctttag tgagtaataa actgctgttc tcatgctgta    3780
   atgagctggc atgagtattt gtgccacatg gctccacatg caagtttgaa acagaactac    3840
   cctgatactt ttctggatgc ctctcagctg cacgcttctc agtggtgttc aaatcatta     3899
<210> 57
   <211> 1100
   <212> DNA
   <213> Human BRCA1 region of interest (ROI) 12 (BRCA1 "Region of Interest" (ROI) 12)
<400> 57
   accccgtctc tactaaaaat acaaaaatta gcctggcatg gtggcgcgtg cctgtaatcc 60
   cagctactaa gggggctaag gcaggaggac tgcttctagc ctgggccaca gagcaagact 120
   ccatctcaaa aaaaaaaaag aaaaaaaaaa gaaaagaaga agaagaagaa gaagaaaaca 180
   aatggtttta ccaaggaagg attttcgggt tcactctgta gaagtctttt ggcacggttt 240
   ctgtagccca tactttggat gatagaaact tcatctttta gatgttcagg agagttattt 300
   tccttttttg caaaattata gctgtttgca tctgtaaaat acaagggaaa acattatgtt 360
   tgcagttaga gaaaaatgta tgaattataa tcaaagaaac caagagaaac cctatgtatg 420
   ctctttgttg tgttaagaca atgcattaag gttacctgtg atttttttta aaaatcaaat 480
   tttaaaaaat caactggaaa ttttactcct aggaaacaaa aacaaataca cagaccatca 540
   aagttattta gagtccttgt tttctacctt aacactaaga aatttgccta tcaatactgt 600
   atttcactaa agctaagaca ccaacaatgt aagttgcact attattttct gtatcactaa 660
   gaaagaaagc acacaaatta aacaaatgac acaccctcaa tagtaaaatg ttcccaattt 720
   cagagatgtt aagatgtgaa aaatgtgcac cttacgattg ataaaataag gtgtgagacc 780
   agtgggagta attttttaaa aatagattac agatacagaa ctaaaattaa cctagactaa 840
   aaggtcttat caccacgtca tagaaagtaa ttgtgcaaac ttcctgagtt ttcatggaca 900
   gcacttgagt gtcattcttg ggatattcaa cacttacact ccaaacctgt gtcaagctga 960
   aaagcacaaa tgattttcaa tagctcttca acaagttgac taaatctcgt actttcttgt 1020
   aggctcctga aattaaattg tttgagaaac acactcagca agtgattatc aaccttttaa 1080
   ggacactaaa ataagaaaag 1100
<210> 58
   <211> 101
   <212> DNA
   <213> Human BRCA1 region of interest (ROI) 13 (BRCA1 "Region of Interest" (ROI)
   13)
<400> 58
   tcctactgtg gttgcttcca acctagcatc attaccaaat tatatacctt ttggttatat 60
   cattcttaca taaaggacac tgtgaaggcc ctttcttctg g 101
<210> 59
   <211> 1229
   <212> DNA
   <213> Human BRCA1 region of interest (ROI) 14 (BRCA1 "Region of Interest" (ROI)
   14)
<400> 59
   tgtaaaacac cataaaaatt aatcttaagg ccgggcgcgg tggctcacgc ctgtaatccc 60
   agcactttgg gaggccgagg tgggcggatc acgaggtcag gaagtggaga ccatcctggc 120
   taacacggtg aaaccccgtc tctactaaaa atacaaaaaa ttagccgggc gtggtggtgg 180
   acgcctgtag tcccagctac ttggggggcc gaggcaggag aatggcgtga acccgggagg     240
   cggagcttgc agtgagccga gatggcgcca ctgcactccg gcctgggtga aagagcgaga     300
   ctccgtctca aaaacaaaac aaacaaaaat taatcttaag ccaggcgcag tggctcacgc     360
   cagcactttg gaaggccgag gcgggtggat cacgagatca ggacttcaag accagcctga     420
   ccaacgtgat gaaaccctat ctctactaaa aatacaaaat tagccggcca cggtggcgtg     480
   cgcctataat cccagctact caggaggctg aggcaggaga agcgcttgaa cttgaacctg     540
   gcaggcggag gttgcagtga gccaagatgg cgccactgca ctccagcctg ggcgacagag     600
   ccagactcca accccccacc ccgaaaaaaa aaggtccagg ccgggcgcag tggctcagga     660
   ctgtaatccc agcactttgg aaggctgagg cgggtggatc acaaggtcag gagatcgaga     720
   ccatcttggc taacatggtg aaaccccgtc tctactaaaa atacaaaaaa ttagccgggc     780
   atagtggtgg gcgcctgtag tcccagctac tcgggaggct gaggcaggag aatggcctga     840
   acccgggagg cggagctggc agtgagccaa gatcgtgcca ctgcactcca gcctaggcag     900
   cagagcgaga ccgtgtctca aaaaaacaaa acaaaacaaa acaaaaagtc tgggagcggt     960
   ggctcacgcc tgtaatccca gcactttcgg aggccaaggc aggaggatca cctgaggtca    1020
   ggagttcgag accaacctga ccaatatgga gaaaccctgt ctctactaaa aatacaaaat    1080
   tagctggtgt gatggcacat gcctgcaatc ccaggtactc cggaggctga ggcagcagaa    1140
   ttgcttgaac ccgggaggtg gaggttgtag tgagccgaga ttgtgccact gcactccagc    1200
   ctgggcaaca agagccaaag tctgtctca                                       1229
<210> 60
   <211> 1369
   <212> DNA
   <213> Human BRCA1 region of interest (ROI) 15 (BRCA1 "Region of Interest" (ROI)
   15)
<400> 60
   agcatggcca acatagcaaa accctatctc tacaacagaa aaatacaaga atggctggac      60
   gcagtggctt atgcctgtaa tcctagcact ttgggaggcc caggcgggtg gatcacaagg     120
   tcaggagatc aagactatcc tggctaacac ggtgaaatcc cgcctctact aaaaaagaaa     180
   aaaaaataca aaaaattagc cgggcgtggt agtgggtgct tgtagtccca gctattcagg     240
   aggctcaggc agaagaatgg catgaacccg ggaggcagag tttgcagtga gctgagatcg     300
   cgccactgca ctccagcctg ggcaacagag caagactcca tctcaaaaaa agaaaaaaaa     360
   atacaaaaat tagctgggca tggtggtgca cacctatcgt ccctgctact ctggaggctg     420
   aggtgggagg attgcttgag cctgacgagg ttgaggctgc agtgagctgt gatagcacca     480
   ctgcactcca gcctctcgac agagatccta tataaaaaaa aaacctctgc atttcattgt     540
   atgtaaataa gtatgtaatt tcattgtatg tacagagcca gtttcaaaca aaggttcttc     600
   caaataccta tcctctcaac gacaccgatc atccatgttt tttttttttt tttttttttt     660
   ttgagatgga gtttagctct gtcgctggag ttcagtggtg ccatattggc tcacagcaac     720
   atctgcctcc tggttcaagt gattctcctg cctcagcctc ctgagtagct gggattacag 780
   gcacatgcca ctacgcccag ctaatttttg tatttttagt ggagaggggg tttcaccatg 840
   ttggccagga tggtctcgat ctcctgacct cgtgatccta ccaccttggc ctcccaaagt 900
   gctgggatta caggcataag ccaccgccct cggcctcatc catgatttta ttttgccatt 960
   tcaagtgatg gagcttgttt tagagctgga agaaaagcca aaatgccagt taatctaaac 1020
   tagattcctg ccccagtgca gaaccaatca agacagagtc cctgtctttc ccggaccaca 1080
   ggatttgtgt tgaaaaggag aggagtggga gaggcagagt ggatggagaa caaggaatca 1140
   ttttctatat ttttaaagtt cttcagttaa gaaaatcagc aattacaata gcctaatctt 1200
   actagacatg tcttttcttc cctagtatgt aaggtcaatt ctgttcattt gcataggaga 1260
   taatcatagg aatcccaaat taatacactc ttgtgctgac ttaccagatg ggacactcta 1320
   agattttctg catagcatta atgacatttt gtacttcttc aacgcgaag 1369
<210> 61
   <211> 1308
   <212> DNA
   <213> Human BRCA1 region of interest (ROI) 16, preferred for methylation
   analyses (BRCA1 "Region of Interest" (ROI) 16, präferiert für Methylierungsanalysen)
<400> 61
   aatcaacaac gaccaaacca acaccaatca aggcctcccc gcccctaacc tttcccagtg 60
   acctgctctc atctctggat cctcctcaag cacatccctg ccggcagcat ctgttactac 120
   tgacgctcct ctacttccct cttgcgcttt ctcaatggcg caaatggatc cagttcttaa 180
   gttctccctc ccacaaaatc ctgtctcctc cccttcccag acatattcct ggcacctctt 240
   cttccacaag gtcccatcct ctcatacata ccagccggtg ttttttgttt tgttttgttt 300
   tgttttgttt tgagacagtc tcgctctgtc gcccaggctg gagtgcaatg gcgcgatctc 360
   ggctcactgc aacctccgcc tcccgggttc tagcgattct cctgcctcag cctcctgagt 420
   agctggagcg gcaccacgcc cggctaattt ttgtattttt agtagagacg gagtttcacc 480
   acgttggtca ggctggtctg gaactcctga cctcatgacc agccgacgtt tttaaagaca 540
   tagtgtcccc ctcaaggcat attccagttc ctatcacgag gattccccca cggacactca 600
   gtgccccctt cctgatcctc agcgcttccc tcgcgaccta caaactgccc ccctccccag 660
   ggttcacaac gccttacgcc tctcaggttc cgcccctacc ccccgtcaaa gaatacccat 720
   ctgtcagctt cggaaatcca ctctcccacg ccagtacccc agagcatcac ttgggccccc 780
   tgtccctttc ccgggactct actaccttta cccagagcag agggtgaagg cctcctgagc 840
   gcaggggccc agttatctga gaaaccccac agcctgtccc ccgtccagga agtctcagcg 900
   agctcacgcc gcgcagtcgc agttttaatt tatctgtaat tcccgcgctt ttccgttgcc 960
   acggaaacca aggggctacc gctaagcagc agcctctcag aatacgaaat caaggtacaa 1020
   tcagaggatg ggagggacag aaagagccaa gcgtctctcg gggctctgga ttggccaccc 1080
   agtctgcccc cggatgacgt aaaaggaaag agacggaaga ggaagaattc tacctgagtt 1140
   tgccataaag tgcctgccct ctagcctcta ctcttccagt tgcggcttat tgcatcacag    1200
   taattgctgt acgaaggtca gaatcgctac ctattgtcca aagcagtcgt aagaagaggt    1260
   cccaatcccc cactctttcc gccctaatgg aggtctccag tttcggta                 1308
<210> 62
   <211> 1411
   <212> DNA
   <213> Human BRCA1 region of interest (ROI) 17 (BRCA1 "Region of Interest" (ROI) 17)
<400> 62
   atgcgttgcg gaatgaaagg tcttcgccac agtgttcctt agaaactgta gtcttatgga      60
   gaggaacatc caataccaga gcgggcacaa ttctcacgga aatccagtgg atagattgga     120
   gacctgtgcg cgcttgtact tgtcaacagt tatggactgg agtgttatgt tttcgtattt     180
   tgaaagcaga aactaggcct taaaaagata cgtacaactc tttagggaga ctacaattcc     240
   catccagccc caggagtctg gggcaagtag tcttgtaagg tcagtggcct gcggggacgc     300
   agtgagcgcc gaatttgcct ggggcagggg aaatgcgctc tggcccatgt ctgcgcactc     360
   gtagttccac ccctcagccc cagtgtttgt tatttttcgg gttcagcttg cttttgcccc     420
   gtctccgtcg acgcaatcgc caccagtcaa tggggtggtc gttttgaggg acaagtggta     480
   agagccaatc ttcttggcga aaacgcggag aaacgggact agttactgtc tttgtccgcc     540
   atgttagatt caccccacag agatagcggc agagctggca gcggacggtc tttgcattgc     600
   cgcctcccca gggggcggga agctggtaag gaagcagcct gggttagcta ggggtggggt     660
   cacgtcacac taagagggtt tggagaagtt caagggagga atcctgcaaa gaagaggggc     720
   gactttttcc gtgtctccgg acagctaatc gttttagtga caggatgaga gagcccttcg     780
   tgttctgagg gaccgagtgg gcgaaaagcg ccggagagtt ggagagtctg tggttcagaa     840
   tgcgaggtga caacgtgcta gcagccctcg ctcgctctcg gcgcctcctc ggccttggcg     900
   tccattctgg ccgtgctgga ggagcccttc agcccgccac tgcgctgtgg gggcccctct     960
   ctgggctggc cgaagccaga gccggctccc tctgcttgcg gggaagtgtg gagggagagg    1020
   cgggtgtggg aactggggct gcgcgcagcg ctcgccagcc agcgcgagtt ccaggtgggc    1080
   gcgggctcag cgggccccgc acccccggcc ccgggcagtc aggggcctag cacccgggcc    1140
   agcagctgca gagggtgcgc cgggtccccc agcactgccg gcccgcctgc accccgcttg    1200
   aattctcacc gggccccagc cgccctgcac agggcaaggc tcaggacctg cagcccgcca    1260
   tgcccgagcc ccctcccaac ccctgtgagc tccagcgtgg cctgagcctc cccgacgggc    1320
   accgccccct gctcctcagc gcccggtccc atcgactgcc caagggctga gaggagtgca    1380
   ggcgcccggc acagccctgc gcaggatcca c                                   1411
<210> 63
   <211> 2166
   <212> DNA
   <213> Human BRCA1 region of interest (ROI) 18 (BRCA1 "Region of Interest" (ROI) 18)
<400> 63
   ggatcacgag gtcaagagat cgagaccatc ctggctaaca cagtgaaacc ccgactctac      60
   taaaaagaca aaatattagc tgggtgcggt ggtgggtgcc tgtaatcccc tctactgggg     120
   aggttgaggc aggagaatgg cgtgaacccg ggaggcggag cttgcagtga gcccagattg     180
   caccactgca ttccagcctg ggtgacagag ggagactcca tctcaaaaaa aaaaaaaaaa     240
   aaaaaaaaaa atgcaggctg cctgagccag cagcagcaac ccgctctggt ctccttccac     300
   gctgtggaag ctttgttctt gtgctctttg caataaatct tgctgctgct cactctttgg     360
   gtccgcatag catttatctg ctggtaacac cgaccgcaga ggtctgcagc ttcactcctg     420
   aagccagcga gaccacgaac ccaccaggag gaatgaacaa ctccagacgg gaggaacgaa     480
   caaactccag acatgccgcc ttaagagctg taacactcac cgcaaaggtc tgcagcttca     540
   ctcctgaagc cagcgagacc acgaatccac cagaaggagg taactctgaa cacgtccgaa     600
   catcagaagg aacaaactct ggacacatca tctttaagaa ctgtaccact caccgtgagg     660
   gtccgcaact tcattcttga agtcagtgag accaagaacc caccaatttc ggacacaaga     720
   acatccggct tctactgatg gaaggccacg actcctcagt agaaagaggg cccaggcaaa     780
   gaaaatgaga gagagtcctt ggggtggtga cactatcttg ggaacatgga gtgctaggaa     840
   acgataactg gaagactagg atgaaatctg caagttaaaa aatgacacta cccatatatc     900
   cttctcatcc cattctcctt ttttcgagac cccgttaaag agatcttggt tagggacctt     960
   tggttttgga aaggcactgt ttctcctttt tgtcaaacac tcaactttca ggccacactt    1020
   tttttttttt tttcgagaca aagtctcgct ttctcaaaga ccagcccagg ctggtcttga    1080
   actcctgagc tcaagcgatc ctcctgcctt ggcctcccaa agtgctggga ttacaggcat    1140
   gagccaccac tcccggcctc agcttggata acttggcttg tacaggcctc tggtggagac    1200
   agtaaccaaa gttcgggttg ccaggtttgt ttcattaaag ggattttgtt gaggctgaaa    1260
   gaaaaaaaaa tcgatgtgga ggaaaagcta acttgtgcca agacaaggtg agtcagtcac    1320
   atggacttaa caataatgtc aattatgaaa gctgaaggcc taggacccag ctatctgact    1380
   ctactggctg tagacctaag gagcaggtag ggttaaccat agctttcgtg tttacacacg    1440
   ttcaagatta atgctcttgc tgaaagaatt tttttacatg ccagccaaag agagttcctt    1500
   atggagacct ggatatggtc cagctaagct agaatctccc acttcctgtt ttctcttagt    1560
   tggcccacca gctggaggag ctccagaata tctaaattaa tttccaattg tgtaatttga    1620
   gaaaaagaga cgaagattaa tttccctaac gagtagggaa gtgtgtttct tttttttctt    1680
   ttttttttga gactgagtct cgctctgtcg cccagcctga agtgcagtgg cacgatcgcg     1740
   gctagtgcac tgcgaactct gtctcccggg ttcacgccat tctcctgcct cagcctcccg    1800
   agtagctggg actacaggca cccgccatca cgcccagcta attttttgtg tttttagtag    1860
   agatgggggt ttcaccgcgt tagccaggat gacctcgatc tcctgacctc gtgatctgcc    1920
   ttcctcggcc tcccaaagtg ctgggattat aggcgtgagc caccgcacct ggcaggaagt    1980
   gtttcttaaa gttggaaagt ggtatttcag aatatccgct tttggcagac tgagtcagtc 2040
   acttcatttt cctgattttt tgcctaaggt cctggtgaat tgacaaagat tctgcccaaa 2100
   ttttaacgac cccagtaatt acatgtcata tgaatgaata cttgacgtca gcaggactgc 2160
   gttttg 2166
<210> 64
   <211> 99
   <212> DNA
   <213> Human EGFR region of interest (ROI) 1, exon 19 (EGFR "Region of Interest"
   (ROI) 1, Exon 19)
<400> 64
   ggactctgga tcccagaagg tgagaaagtt aaaattcccg tcgctatcaa ggaattaaga 60
   gaagcaacat ctccgaaagc caacaaggaa atcctcgat 99
<210> 65
   <211> 156
   <212> DNA
   <213> Human EGFR region of interest (ROI) 2, exon 21 (EGFR "Region of Interest"
   (ROI) 2, Exon 21)
<400> 65
   ggcatgaact acttggagga ccgtcgcttg gtgcaccgcg acctggcagc caggaacgta 60
   ctggtgaaaa caccgcagca tgtcaagatc acagattttg ggctggccaa actgctgggt 120
   gcggaagaga aagaatacca tgcagaagga ggcaaa 156
<210> 66
   <211> 2728
   <212> DNA
   <213> Human EGFR region of interest (ROI) 3, exon 20 (EGFR "Region of interest"
   (ROI) 3, Exon 20)
<400> 66
   ttgcatcggt actgaacata tacggacttt ttttcttgtc attattccct aaacaataca      60
   gcataacaat tattcacata gcatttgcac tgtattaggt actataggta atcaggagat     120
   gctgtagatg ggaggatgtc tgtaggttac acacaaatgc tgtgccactt tatatcaggg     180
   gcttgagcat cctcacattt tgatatttaa gggaggtcct ggaaccaatt ccccagatac     240
   tgagggtcca ctgtctgtgt cccctcgccc caccttgcct ttgtctcctg tctcctatct     300
   ccaccctgcc tcccgccagc ctgttgctcc tgacctgccc gggcaccctg gagcagcacc     360
   ctatctcaga gcctggctca gtgtgttcac ttctgcagag aaactaactt gcccaagtcc     420
   acactcaaaa cataggcatt gctgagatgt gaaaagcagc tgtggatgct ttctgctaca     480
   gtctgtgtgt tcttttccat atctgaataa aaggtcacca ccatttgtat tttaaagaga     540
   aagagaattt atgggtggaa attggggatt ccctcattct cagtcagaca gaaaagaggg     600
   ccccattgtg tgcctgattg caaataaatt tagcttcctc agcccaagaa tagcagaagg     660
   gttaaaataa agtctgtatt tatggctctg tcaaaggaag gcccctgcct tggcagccag     720
   ccggaattag cagggcagca gatgcctgac tcagtgcagc atggatttcc catagggagc     780
   ctgggggcac agcacagaga gaccacttct ctttagaaat gggtcccggg cagccaggca 840
   gcctttagtc actgtagatt gaatgctctg tccatttcaa aacctgggac tggtctattg 900
   aaagagctta tccagctact ctttgcagag gtgctgtggg cagggtcccc agcccaaatg 960
   cccacccatt tcccagagca cagtcagggc caagcctggc ctgtggggaa gggaggcctt 1020
   tctccctgct ggctcggtgc tccccggatg ccttctccat cgcttgtcct ctgcagcacc 1080
   cacagccagc gttcctgatg tgcagggtca gtcattaccc agggtgttcc ggaccccaca 1140
   cagattccta caggccctca tgatatttta aaacacagca tcctcaacct tgaggcggag 1200
   gtcttcataa caaagatact atcagttccc aaactcagag atcaggtgac tccgactcct 1260
   cctttatcca atgtgctcct catggccact gttgcctggg cctctctgtc atggggaatc 1320
   cccagatgca cccaggaggg gccctctccc actgcatctg tcacttcaca gccctgcgta 1380
   aacgtccctg tgctaggtct tttgcaggca cagcttttcc tccatgagta cgtattttga 1440
   aactcaagat cgcattcatg cgtcttcacc tggaaggggt ccatgtgccc ctccttctgg 1500
   ccaccatgcg aagccacact gacgtgcctc tccctccctc caggaagcct acgtgatggc 1560
   cagcgtggac aacccccacg tgtgccgcct gctgggcatc tgcctcacct ccaccgtgca 1620
   gctcatcacg cagctcatgc ccttcggctg cctcctggac tatgtccggg aacacaaaga 1680
   caatattggc tcccagtacc tgctcaactg gtgtgtgcag atcgcaaagg taatcaggga 1740
   agggagatac ggggagggga gataaggagc caggatcctc acatgcggtc tgcgctcctg 1800
   ggatagcaag agtttgccat ggggatatgt gtgtgcgtgc atgcagcaca cacacattcc 1860
   tttattttgg attcaatcaa gttgatcttc ttgtgcacaa atcagtgcct gtcccatctg 1920
   catgtggaaa ctctcatcaa tcagctacct ttgaagaatt ttctctttat tgagtgctca 1980
   gtgtggtctg atgtctctgt tcttatttct ctggaattct ttgtgaatac tgtggtgatt 2040
   tgtagtggag aaggaatatt gcttccccca ttcaggactt gataacaagg taagcaagcc 2100
   aggccaaggc caggaggacc caggtgatag tggtggagtg gagcaggtgc cttgcaggag 2160
   gcccagtgag gaggtgcaag gagctgacag agggcgcagc tgctgctgct atgtggctgg 2220
   ggccttggct aagtgtcccc ctttccacag gctcgctcca gagccagggc ggggctgaga 2280
   gagcagagtg gtcaggtagc cctgcctggg tgctggagac aggcacagaa caacaagcca 2340
   ggtatttcac agctggtgcg gacccagaaa gacttctgct tttgccccaa acccctccca 2400
   tctccatccc agtcttgcat cagttatttg cactcaactt gctaagtcct atttttttct 2460
   aacaatgggt atacatttca tcccattgac tttaaaggat ttgcaggcag gccctgtctc 2520
   tgagaatacg ccgttgcccg tcatctctct ccgacagcag ggcagggggt ccagagatgt 2580
   gccagggacc agagggaggg agcagacacc cacccggcct gggcaggtcc tcctcattgc 2640
   ttgcatccgc ctggttagca gtggcagtca gtcctgccga gtcattcgtg aggcgctcac 2700
   ccaactccag gcagatgtaa aaggtgac 2728
<210> 67
   <211> 123
   <212> DNA
   <213> Human EGFR region of interest (ROI) 4, exon 18 (EGFR "Region of Interest"
   (ROI) 4, Exon 18)
<400> 67
   cttgtggagc ctcttacacc cagtggagaa gctcccaacc aagctctctt gaggatcttg    60
   aaggaaactg aattcaaaaa gatcaaagtg ctgggctccg gtgcgttcgg cacggtgtat   120
   aag                                                                 123
<210> 68
   <211> 122
   <212> DNA
   <213> Human KRAS region of interest (ROI) 1, exon 2 (KRAS "Region of interest"
   (ROI) 1, Exon 2)
<400> 68
   ctctattgtt ggatcatatt cgtccacaaa atgattctga attagctgta tcgtcaaggc    60
   actcttgcct acgccaccag ctccaactac cacaagttta tattcagtca ttttcagcag   120
   gc                                                                  122
<210> 69
   <211> 179
   <212> DNA
   <213> Human KRAS region of interest (ROI) 2, exon 3 (KRAS "Region of interest"
   (ROI) 2, Exon 3)
<400> 69
   ctataatggt gaatatcttc aaatgattta gtattattta tggcaaatac acaaagaaag    60
   ccctccccag tcctcatgta ctggtccctc attgcactgt actcctcttg acctgctgtg   120
   tcgagaatat ccaagagaca ggtttctcca tcaattacta cttgcttcct gtaggaatc    179
<210> 70
   <211> 671
   <212> DNA
   <213> Human KRAS region of interest (ROI) 3, exon 4 (KRAS "Region of interest"
   (ROI) 3, Exon 4)
<400> 70
   tctgttggct ttctgggtat tgtctttctt taatgagacc tttctccaga aataaacaca 60
   tcctcaaaaa aattctgcca aagtaaaatt cttcaaatac aacaacgttt aacctagaaa 120
   catgacataa tggtttaaaa gtactaccga aatagaaaac gcaaaaattt gttgccctgt 180
   tctttatata tatatatata tatatatata tatatatata tatatatata tatgtaacca 240
   tgggaaaaaa aaatccccac ctcttgaatt ctaaatgtta acatgtgctc agaattgaag 300
   agaaattttc aatgtagaaa gaaaccaaag ccaaaagcag taccatggac actggattaa 360
   gaagcaatgc cctctcaaga gacaaaaaca tttactaaat attgttttat ttcctagtat 420
   agcataattg agagaaaaac tgatatatta aatgacataa cagttatgat tttgcagaaa 480
   acagatctgt atttatttca gtgttactta cctgtcttgt ctttgctgat gtttcaataa 540
   aaggaattcc ataacttctt gctaagtcct gagcctgttt tgtgtctact gttctagaag 600
   gcaaatcaca tttatttcct actaggacca taggtacatc ttcagagtcc ttaactcttt 660
   taatttgttc t 671
<210> 71
   <211> 119
   <212> DNA
   <213> Human BRAF region of interest (ROI) 1, exon 15 (BRAF "Region of Interest"
   (ROI) 1, Exon 15)
<400> 71
   catccacaaa atggatccag acaactgttc aaactgatgg gacccactcc atcgagattt 60
   cactgtagct agaccaaaat cacctatttt tactgtgagg tcttcatgaa gaaatatat 119
<210> 72
   <211> 118
   <212> DNA
   <213> Human BRAF region of interest (ROI) 2, exon 11 (BRAF "Region of Interest"
   (ROI) 2, Exon 11)
<400> 72
   catgccactt tcccttgtag actgttccaa atgatccaga tccaattctt tgtcccactg 60
   taatctgccc atcaggaatc tcccaatcat cactcgagtc ccgtctacca agtgtttt 118
<210> 73
   <211> 287
   <212> DNA
   <213> Human AKT1 region of interest (ROI) (AKT1 "Region of Interest" (ROI))
<400> 73
   gggatactta cgcgccacag agaagttgtt gaggggagcc tcacgttggt ccacatcctg 60
   cggccgctcc ttgtagccaa tgaaggtgcc atcattcttg aggaggaagt agcgtggccg 120
   ccaggtcttg atgtactccc ctacagacgt gcgggtggtg agagccacgc acactctacc 180
   cgtcagaccc tcgccaggca gccaggcagg aactgggtgt gccaggacag atgtgcctgg 240
   gatgcctgag tcccaggggg caggcgcggt acgggagctg tttctta 287
<210> 74
   <211> 135
   <212> DNA
   <213> Human DDR2 region of interest (ROI) 1, exon 19 (DDR2 "Region of interest"
   (ROI) 1, Exon 19)
<400> 74
   acttacctcc ctcaaccagc catttgtcct gactctgtgt ataagctgat gctcagctgc 60
   tggagaagag atacgaagaa ccgtccctca ttccaagaaa tccaccttct gctccttcaa 120
   caaggcgacg agtga 135
<210> 75
   <211> 150
   <212> DNA
   <213> Human DDR2 region of interest (ROI) 2, exon 18 (DDR2 "Region of interest"
   (ROI) 2, Exon 18)
<400> 75
   ggcaagttca ctacagcaag tgatgtgtgg gcctttgggg ttactttgtg ggagactttc 60
   accttttgtc aagaacagcc ctattcccag ctgtcagatg aacaggttat tgagaatact 120
   ggagagttct tccgagacca agggaggcag 150
<210> 76
   <211> 719
   <212> DNA
   <213> Human DDR2 region of interest (ROI) 3, exons 16 and 17 (DDR2 "Region of
   Interest" (ROI) 3, Exone 16 und 17)
<400> 76
   gaatgatttt cttaaggaga taaagatcat gtctcggctc aaggacccaa acatcatcca    60
   tctattagct gtgtgtatca ctgatgaccc tctctgtatg atcactgaat acatggagaa   120
   tggagatctc aatcagtttc tttcccgcca cgagccccct aattcttcct ccagcgatgt   180
   acgcactgtc aggtaaacaa gccaggtctt ccttctcctc cctgtggtca tgagagtaac   240
   ctgggatctg aaaataggga gcagtgagcc ttaaagtgta tcaatgttct gggatggtgg   300
   gggaagtcag tgtgcaggga ataatggagc agctgccctt tggggaaacg caagggattc   360
   atcaagagta gagaaagaat gttgagcttt caaccctagt ttgttgatac cattttagaa   420
   tgtgtacctt tcacatcttc ctgtttccat aggctacctt ctgtcttctt gtctatttcc   480
   tcagttacac caatctgaag tttatggcta cccaaattgc ctctggcatg aagtaccttt   540
   cctctcttaa ttttgttcac cgagatctgg ccacacgaaa ctgtttagtg ggtaagaact   600
   acacaatcaa gatagctgac tttggaatga gcaggaacct gtacagtggt gactattacc   660
   ggatccaggg ccgggcagtg ctccctatcc gctggatgtc ttgggagagt atcttgctg    719
<210> 77
   <211> 186
   <212> DNA
   <213> Human ERBB2 region of interest (ROI) (ERBB2 "Region of interest" (ROI))
<400> 77
   gaagcatacg tgatggctgg tgtgggctcc ccatatgtct cccgccttct gggcatctgc    60
   ctgacatcca cggtgcagct ggtgacacag cttatgccct atggctgcct cttagaccat   120
   gtccgggaaa accgcggacg cctgggctcc caggacctgc tgaactggtg tatgcagatt   180
   gccaag                                                              186
<210> 78
   <211> 211
   <212> DNA
   <213> Human MAP2K1 (MEK1) region of interest (ROI) (MAP2K1 (MEK1) "Region of
   interest" (ROI))
<400> 78
   gaccaacttg gaggccttgc agaagaagct ggaggagcta gagcttgatg agcagcagcg    60
   aaagcgcctt gaggcctttc ttacccagaa gcagaaggtg ggagaactga aggatgacga   120
   ctttgagaag atcagtgagc tgggggctgg caatggcggt gtggtgttca aggtctccca   180
   caagccttct ggcctggtca tggccagaaa g                                  211
<210> 79
   <211> 66
   <212> DNA
   <213> Human NRAS region of interest (ROI) 1, codon 61 (NRAS "Region of
   Interest" (ROI) 1, Kodon 61)
<400> 79
   cctgtcctca tgtattggtc tctcatggca ctgtactctt cttgtccagc tgtatccagt 60
   atgtcc 66
<210> 80
   <211> 66
   <212> DNA
   <213> Human NRAS region of interest (ROI) 2, codon 12 (NRAS "Region of
   Interest" (ROI) 2, Kodon 12)
<400> 80
   tttgttttta tgtattggtt ttttatggta ttgtattttt tttgtttagt tgtatttagt 60
   atgttt 66
<210> 81
   <211> 303
   <212> DNA
   <213> Human PIK3CA region of interest (ROI) 1, exon 9 (PIK3CA "Region of
   Interest" (ROI) 1, Exon 9)
<400> 81
   ataagatatt attttatttt acagagtaac agactagcta gagacaatga attaagggaa 60
   aatgacaaag aacagctcaa agcaatttct acacgagatc ctctctctga aatcactgag 120
   caggagaaag attttctatg gagtcacagg taagtgctaa aatggagatt ctctgtttct 180
   ttttctttat tacagaaaaa ataactgaat ttggctgatc tcagcatgtt tttaccatac 240
   ctattggaat aaataaagca gaatttacat gatttttaaa ctataaacat tgccttttta 300
   aaa 303
<210> 82
   <211> 455
   <212> DNA
   <213> Human PIK3CA region of interest (ROI) 2, exon 20 (PIK3CA "Region of
   interest" (ROI) 2, Exon 20)
<400> 82
   gtctacgaaa gcctctctaa ttttgtgaca tttgagcaaa gacctgaagg tattaacatc 60
   atttgctcca aactgaccaa actgttctta ttacttatag gtttcaggag atgtgttaca 120
   aggcttatct agctattcga cagcatgcca atctcttcat aaatcttttc tcaatgatgc 180
   ttggctctgg aatgccagaa ctacaatctt ttgatgacat tgcatacatt cgaaagaccc 240
   tagccttaga taaaactgag caagaggctt tggagtattt catgaaacaa atgaatgatg 300
   cacatcatgg tggctggaca acaaaaatgg attggatctt ccacacaatt aaacagcatg 360
   cattgaactg aaaagataac tgagaaaatg aaagctcact ctggattcca cactgcactg 420
   ttaataactc tcagcaggca aagaccgatt gcata 455
<210> 83
   <211> 455
   <212> DNA
   <213> Human PTEN region of interest (ROI), exon 7 (PTEN "Region of interest"
   (ROI), Exon 7)
<400> 83
   tcaaactgga gaaaatctta cattgtttat atttttattt catttatttc agttgatttg    60
   cttgagatca agattgcaga tacagaatcc atatttcgtg tatattgctg atattaatca   120
   ttaaaatcgt ttttgacagt ttgacagtta aaggcatttc ctgtgaaata atactggtat    180
   gtatttaacc atgcagatcc tcagtttgtg gtctgccagc taaaggtgaa gatatattcc   240
   tccaattcag gacccacacg acgggaagac aagttcatgt actttgagtt ccctcagccg   300
   ttacctgtgt gtggtgatat caaagtagag ttcttccaca aacagaacaa gatgctaaaa   360
   aaggtttgta ctttactttc attgggagaa atatccaaaa taaggacaga ttaaaagcta   420
   tattttattt tatgacatgt aaggaactat aattt                              455
<210> 84
   <211> 593
   <212> DNA
   <213> Human IDH1 region of interest (ROI) (IDH1 "Region of interest" (ROI))
<400> 84
   tatggtataa ctgaataaaa ggataaagga aaaaaaacag catgtttgaa aaaaaatgtg    60
   ttgagatgga cgcctatttg taagtttatt tgtatttgcc tttagctaaa tgtgtgtaaa   120
   tatacagtta tacatatatg catttctcaa tttcatacct tgcttaatgg gtgtagatac   180
   caaaagataa gaataaaaca catacaagtt ggaaatttct gggccatgaa aaaaaaaaca   240
   tgcaaaatca cattattgcc aacatgactt acttgatccc cataagcatg acgacctatg   300
   atgataggtt ttacccatcc actcacaagc cgggggatat ttttgcagat aatggcttct   360
   ctgaagaccg tgccacccag aatatttcgt atggtgccat ttggtgattt ccacatttgt   420
   ttcaacttga actcctcaac cctcttctca tcaggagtga tagtggcaca tttgacgcca   480
   acattatgct tctttatagc ttctgcagca tccttggtga cttggtcgtt ggtggcatca   540
   cgattctcta tgcctaaatc atagcttgaa agagaaaaat tagaagcaaa gtt          593
<210> 85
   <211> 377
   <212> DNA
   <213> Human IDH2 region of interest (ROI) (IDH2 "Region of interest" (ROI))
<400> 85
   ggaagttgta cacttcccac tccttgacac cactgccatc ttttggggtg aagaccattt       60
   tgaaagtgcc ggcccggtct gccacaaagt ctgtggcctt gtactgcaga gacaagagga      120
   tggctaggcg aggagctcca gtcggggggt gcccaggtca gtggatcccc tctccaccct       180
   ggcctacctg gtcgccatgg gcgtgcctgc caatggtgat gggcttggtc cagccaggga      240
   ctaggcgtgg gatgtttttg cagatgatgg gctcccggaa gacagtcccc cccaggatgt      300
   tccggatagt tccattggga cttttccaca tcttcttcag cttgaactct gtgaggacag      360
   agataatagt ggtccca                                                     377
<210> 86
   <211> 22
   <212> DNA
   <213> Artificial
<220>
   <223> Forward primer (F1) suitable for BRCA1 methylation analyses (vorwärtsprimer (F1) geeignet für BRCA1 Methylierungsanalysen)
<400> 86
   ccaatacccc aaaacatcac tt 22
<210> 87
   <211> 22
   <212> DNA
   <213> Artificial
<220>
   <223> Reverse primer (R1) suitable for BRCA1 methylation analyses (Rückwärtsprimer (R1) geeignet für BRCA1 Methylierungsanalysen)
<400> 87
   ggggtagatt gggtggttaa tt 22
<210> 88
   <211> 24
   <212> DNA
   <213> Artificial
<220>
   <223> Forward primer (F2) suitable for BRCA1 methylation analyses (Vorwärtsprimer (F2) geeignet für BRCA1 Methylierungsanalysen)
<400> 88
   ctaaacgcaa aaacccaatt atct 24
<210> 89
   <211> 28
   <212> DNA
   <213> Artificial
<220>
   <223> Reverse primer (R2) suitable for BRCA1 methylation analyses (Rückwärtsprimer (R2) geeignet für BRCA1 Methylierungsanalysen)
<400> 89
   ggtttttttt gtttttttta ttttttga 28
<210> 90
   <211> 1791
   <212> DNA
   <213> Human PITX2 region of interest (ROI) 1 (promoter A), preferred for
   methylation analyses (PITX2 "Region of Interest" (ROI) 1 (Promotor A), präferiert für Methylierungsanalysen)
<400> 90
   cttccagtag ctgaaacttt gtacccagcc ctttatcttg agaatgctaa tccttggccc 60
   gaggatttgt tcctgcagtg ttggcaccga gatttaaggg aagatacctc gttttaaatg 120
   ccagccacgg tctggcttcc ctctcgactt cagcaccctg tagattgtta gtgtctgtgg 180
   cgggggacga aaggaacagg gctttgcaag gtctgtttgc cgactgcgtt accttgggcg 240
   aaacttagcc ccaaaagcca caaatcacct acggtgaaga ttctccgaag tggaacaaat 300
   ttccagactc gcattatctc acatccctgc gggatagatg gcctccactt accggctacc 360
   gggagagagc tgctgtctcc gcgtcccact gcttcccggg gcgatttcca gcgagccgag 420
   cctccggctg cacggcaagc gcccgaaagc cgggcctgag aggactgcag ggctcctgag 480
   ggtgccaagt tccgaaggag tccacgggtg cactggggcc tccgaaatct agccgccact 540
   ggcagtttct ttctgctcct ctccagcttt ctcgctcggt ctcgcactct ctctcctctc 600
   cctccctctc atccctctct cttccctctg ctcctactcc gtgtggggag tgacgtgacg 660
   tcagcagaga ttccaccaaa ctccactgca cagtggcgcg cgggcggccg gccgagcccg 720
   gctgcgcggc tggcgatcca ggagcgagca cagcgcccgg gcgagcgccg gggggagcga 780
   gcaggggcga cgagaaacga ggcaggggag ggaagcagat gccagcgggc cgaagagtcg 840
   ggagccggag ccgggagagc gaaaggagag gggacctggc ggggcactta ggagccaacc 900
   gaggagcagg agcacggact cccactgtgg aaaggaggac cagaagggag gatgggatgg 960
   aagagaagaa aaagcaatct gcgccaaccc ggcagcccta ataaatcaaa gggggagcgc 1020
   cagggcagcg gggagacaga aacgtacttt tggggagcaa atcaggacgg gctgggagga 1080
   agcgacaggg aaagtggccc aagagacgga acaaaggaca atgttcatgg ggttgtttgg 1140
   gacgaggcgt gtggagtgtg ggtgtgagcg tgcgtgtgtg accttctttc aggcctgcag 1200
   agttgaggaa agaggtcaca gcaaagaggg actgcggagg gaggaaagtg agagaccggt 1260
   agagggcggg agtggaggtg ggcgcggtgg ggatgggaga ggatgagtga agagaaatct 1320
   agaagaatgg agtgagctag tgggagaggg tgggagggcc acagccggga gcgaacgagc 1380
   taggcttgtc agctggggaa ggccgggacg ctgggcccag cttagctggg acaccgcgcc 1440
   cgaggtcaag gcgggtggac caggcatgct gagagtgtcg gcgcacaggt gggcacggcc 1500
   acgcactgac ccagtgttca cgaagggttt gcactggaca aggctcagac gctcatagag 1560
   tctagaattt cctctgctgt acctacattc aacaagttca ccctgggtca cggatatctc 1620
   attttttaaa atgacgaggt taaggttcct ggcgaggatg gtattaaatt gcacgggata 1680
   gaagtggggg tgggggagag agtttccctc aagtccacat ttgctcctgc aaagcaaaga 1740
   gtatgtgaaa ttacagggca tattctcact cgaaaagtgt gccttacttc t 1791
<210> 91
   <211> 6265
   <212> DNA
   <213> Human PITX2 region of interest (ROI) 2 (promoter C), preferred for
   methylation analyses (PITX2 "Region of interest (ROI) 2 (Promotor C), präferiert für Methylierungsanalysen)
<400> 91
   tattactaac aactgctttt ataaaattaa tttgacattt cgatatatat acatcctttc 60
   agtcatttaa atgttaacaa tgctaaactt aaaaaataac aagcttatag taatgttaaa 120
   atgtcatatc cagtcaaaca tttgtttgtg tatgtgtcct tgcaactgtt agaaatactt 180
   gtagtgaaag atgtcagaca ctgaggacat ccctttgaaa tcaaaggagc tctctctttg 240
   attcagtggt ttccttttct ctatatagct tctctttctc tccctttctt tagtgcccac 300
   gaccttctag cataattccc agtctttcaa gggcggagtt gccccatccg gcaaggtcct 360
   aggatcccgg cgctgtgggt gcggctcaca cgggccggtc cactgcatac tggcaagcac 420
   tcaggttgga ggccgggttc tgcacgctgg cgtagccgaa gctggagtgc tgctttgctt 480
   tcagtctcag gctggccagg ctcgagttac acgtgtccct ataaacatac ggaggagtcg 540
   gcggcgcgta aggacaggca ggcgtcggca ccgcggaatt cagcgacggg ctactcaggt 600
   tgttcaagtt attcaggctg ttgagactgg agcccgggac gcctgtcact gctgagggca 660
   ccatgctgga cgacatgctc atggacgaga tagagttggg tggggaaaac atgctctgtg 720
   atgacagggg gttgacgttc atagagttga agaaggggaa gctcttggtg gatagggagg 780
   cggatgtaag gcccttggcg gcccagttgt tgtaggaata gcctgggtac atgtcgtcgt 840
   agggctgcat gagcccattg aactgcggcc cgaagccatt cttgcatagc tcggcctgct 900
   ggttgcgctc cctctttctc catttggccc gacgattctt gaaccaaacc tgggggcggt 960
   tggggcaagg gagcaaacag atgccacagt gcagattact aaaacttcca tcggaggcca 1020
   acccccgcct tcccccgaca cacacgctag cgcactcaca caccctggcc tcgcttcact 1080
   gcaccgccct gcacaccaag ataccagggc cagctttcag ttactggccc gggtctccac 1140
   caagcgcagg agacctggtc tgctctggcc tgcgagctgg gactcggagc tacgccacaa 1200
   acctcagccg aacgcatgga gacctgcgga cggtttgatc actcagccag gcgtttctcc 1260
   aggtccaaaa acacttaatg taaaacaaac gcggggcagc aggcttttcc aacccttccc 1320
   ggggcacctt gcaaacttgc ttccattcca aagccacaga cccacggatg aggagaaggg 1380
   gctggaaggg cactagagga tcgctctttc tcccacgcaa ttcctccctt ccttccctga 1440
   cctccactgt cgtcccccac cccctggtac gtgctccctt aacagggact aggccgccaa 1500
   cactctttct cgcctagcaa aacaaccaaa taaagagcaa aagaccacct cttcgtcagc 1560
   tcgttaactc caggagcttg gcatattaaa ctccgggaac ccggaaaggg tagttttgga 1620
   gattccccct tctttcgctc tgcctcttct ttaccctaag cccaccacag gcctgtccgc 1680
   gcgccaggcc cagccgggtc gtttggcttt gcaggcggcc acccaggccg gccggcttcc 1740
   acccgtgtcc ggtggcccag ccgcaacccc gatcccaatc cacatcgggc ctccctgtcg 1800
   ccccagacgg cggcttttgt gtattggaga gaggcctggc ctgagatatc cgagctgaca 1860
   ccagtgatgt ttcacattac acatctccgc cgggcccagc cgtgtaatcc gctttttctc 1920
   tttttccttt cattcttgat ttccttttta tcccccttcc tctttgcacc cgactgctat 1980
   aaaaagcacg cctcactccc acttggctcg acaagcagcc gccctggaag gagaggcagc 2040
   tgcaaggaga gcccagcgcc gcggctacaa agcactaggg tggagctgcg gaatagcggg 2100
   cggggtggga gggcgttttc gaaggatccc agaaaaccca tagactctgt ctttaattac 2160
   ttgccatttc taccctaggc catctaaact ttgctcaggc gagaagagta cgtgagaggc 2220
   ccgttccctt gatgtgcaag agagctaatg aaagactgac cttgctcaaa accacgccgc 2280
   ccaggaccca gctctggctc tggacagtta aactaaaacc attttcaact tcttcccggc 2340
   cttttatcca ccagcatagc ctcatgcctt gcacaaatgc cacccagaga gtgtcttcat 2400
   tccctctgat ttgggagagc attttggtct ttattctttt tatcgttgtt ttcttctttt 2460
   tgtttgctct gctctaaccg ggggctttat tttttctacc cagagcactt aatttttttt 2520
   ttttaacagc aaagcctctg gatgccgctt gatttgcttg attctgtttt ctgcttccag 2580
   aatcctaaca aatttggaat cttccaccga ccagcataaa ccaggacgtt gctattgggt 2640
   tatttatttg agctcatttt tgccaatcca taaagtacag atttgctaca aagttaaggt 2700
   aagccctttt tacaaaacta tgattataat ttagaagagg gggtgtgagt ttcaatttcc 2760
   agagttcaac tcctgagaga agataaataa accaagcaga aaagtctttc ttcttttttt 2820
   ctttctcctt ctaagaggac tagtagttgt gtattaaaac tttgctcccg gagatcacaa 2880
   aactaggaaa tagggtgtgt gggagagacc tgaatggccg aaacaaccgt aaagaaggtg 2940
   taagaagcgc gagcccagga gggaaaaagc tgggccaggg ccgggacaaa ggtttcccag 3000
   ggagggccaa ctcttccgtg tctctggcgg gttttccttg ttaaaggctc acaggttgga 3060
   gcctgttcgc ggctcttggc ctggtaggga ttttattagc tctgctctgg caactgcaag 3120
   ccaggaacac aatgtcctgt gcaggggatt gcccatgcag cccagctcgt gagatcgcgg 3180
   gatggcgggg cagtgagccg gtgccgctct gggagcctga gccagggcgg cagtcctgtc 3240
   ggcctcggag agggaactgt aatctcgcaa ccaggccgcc gcgaggcctt ctgcctttgc 3300
   aaagctgcgc cccaccggcg ccctcccagg cggcgctgcc ttccacattc tctcctggtc 3360
   tacttggcct gtacctccac aacatcctcc ccccatccct cccagactcc gtgctggctc 3420
   ctacccggac tcgggcttcc gtaaggttgg tccacacagc gatttcttcg cgtgtggaca 3480
   tgtccgggta gcggttcctc tggaaagtgg cctccagctc ctggagctgc tggctggtaa 3540
   agtgagtccg ctgccgcctt tgccgcttct tcttagacgg gtcctcggcg cccacgtcct 3600
   cattcttccc ctgctggctt ttatctttct ctgaaaacga aacacacaca ctttcccgtc 3660
   agcatgccca cctgcaacgc ggacgccaac tggaccggcg gcagaagccg tggaagagct 3720
   gggctgcctg gcgccggagg agggtgcgcg cggcggctcc gggccgcgag gagcgctgcg 3780
   cctgtggggt gtgcaggcgc aagtgtgggt gtccgcgccc catttcctcc cctcccccag 3840
   cgccgcacgt tttatttaca tgtttatctc actgcagcgg cacattcact tttatagcct 3900
   gtgctttcaa gtatatttat acacctctgc gcagacacac caaatctcct gggacgcgca 3960
   cacgcgcgtg gtttacagac ccccctcccc ctcgcagaaa gctcagattt ccatgcggtt 4020
   tgggaaggct aggaaaagat gtggggattc ggttgggcac cgaagttcgc cggccctttc 4080
   ccaaaaaaaa aaaaaaaatg cctcttcgcg aagggcattt ctgagtggtt tcaggcaatt 4140
   tcctaacgag tggagctcct cgggagctga aagccgagag gaaaacaggg acagaggtcg 4200
   gcggcctctg aaggtcctcg aatcaagatg ctgggatttt tgtgacccag gaaacagaag 4260
   ggaggccagg gtacgaatag agagggcggc agaattgctc gcgcccttag cgccccagga 4320
   gccgggccgg tcgagggaga actaaaggga tgcggggtag tcaaaattcc ggctcccgga 4380
   agttctgcgg ggagccaggc gaacgaccac tcccaccacg cctccccccg gaggggctga 4440
   cttccttggg gcgagaggga gcgggtggcg cagagcagct gagcgggaat gtctgcaggg 4500
   cggcgcggcg ccttacctgc ggcctccggg ctggaggtgt cggagatggt gtgcacctcc 4560
   agcctgtgct tggaggagtc cagcgaccgg ggctgaccgg gagccagaac cgaagccatg 4620
   gctaacggct ggggatggtg acaggaagat gaggagacgg ccgacagctt ggtccccgct 4680
   gctcggtgct ccaagtgaag cgggcctttc atgcagttca tggacgaggg agcgcgacgc 4740
   tctactagtc cttggctact gccccgccga gcccccgtag ccgccgctgc ccgctccggg 4800
   tcgcgctcta ggcgcggagt ttccccgctg cggggagagc caggggacgc aacccccgcc 4860
   gagttctcaa gccaagctgc ccccgtctcc tccggaaggc tcaagcgaaa aagtccggag 4920
   acggaaagtc agcgggcaaa cgaagacatg ggatgtgggc agaagggcac cactcagagc 4980
   gtctttaggg agcaggcttc caagctccaa agcgaaacaa gagtgggcaa agaccccctt 5040
   cttctctccc tccctccccc aagaacccct ccaataagga aagctaacgc cgaccgcgct 5100
   ctgcccgccc cccccccacg cggcagccct gacagagaag tgtcaagagt gacagggaca 5160
   ggtaggtgat attagatccc ctgcggcggc agcagccgct gcagccacga cgcggccctc 5220
   tgagcgcacc ctccgcaacg cgcacacgca cacccctcgg gcggtcgaac aggagccggg 5280
   ccttgccgca gctcagctcc aggcacccag gcgagcgacg gaccagatct gcggctccgc 5340
   gcttccctgt tggcctaaca tcttaaaacc agaggcgggc ttcctggtgc cgagacgtca 5400
   ctccgccgcg gccctcccca gccctctccg cctccgcctc ctcccagacc cttctccggg 5460
   tgcgactgac gtggctccgc accaatcagg acgccccgag ccgcggtgga gggactgtcc 5520
   tgcctgcacc tatcagcagt gcggggccgg gctactgcct cgccgtgcgc actgggtcta 5580
   cacaggcaag ctcccgggaa ttcagctcct gcccagccca aggcgatccg gcttttagta 5640
   cgaacccaaa ggtgaagaga tgaggctagg agtcgaaggc ttgggagaag agagtggaat 5700
   ggtcaagaag agaaaggtac aaggatcaac aagacaccca ctctttgtgt ctcactacat 5760
   ccatttccaa tcccccaccc catataaaaa ggagacacgt tacttaaaac tagaaaattt 5820
   gaaaaacagc aacaaatcac ctctccgatc ttaaattttc caaacagcct gtcaagtgaa 5880
   tgctgcgcta atctgaagaa gctttaattg caaagaagac agagccctga aaaggcaggc 5940
   taataaatta gaaatcgaga agcaaatgga cccgtcaaaa gaaaattacc ttgactttaa 6000
   acgaacaact gtttggtggt tcactctgga tttatacaag aataaaaagt cgcctcagat 6060
   cacgttctct gtgatgctta ttagtcccca gacagaaaac acacaataga agagaaaccc 6120
   taacccagcg ttttcaaaat gctgaaagct tatccattct acttaacgtt gattaagaca 6180
   catatcctag atctttcaaa ttccttgtac actgtattaa gctcgtccta acccgagaga 6240
   gccacgcttt aaattcgact ctctt 6265
<210> 92
   <211> 2449
   <212> DNA
   <213> Human MGMT region of interest (ROI) 1, preferred for methylation analyses
   (MGMT "Region of Interest" (ROI) 1, präferiert für Methylierungsanalysen)
<400> 92
   ctccaacata gcttctctgg tggacacaat tcaactccta ataacgtcca cacaacccca      60
   agcagggcct ggcaccctgt gtgctctctg gagagcggct gagtcaggct ctggcagtgt     120
   ctaggccatc ggtgactgca gcccctggac ggcatcgccc accacaggcc ctggaggctg     180
   cccccacggc cccctgacag ggtctctgct ggtctggggg tccctgacta ggggagcggc     240
   accaggaggg gagagactcg cgctccgggc tcagcgtagc cgccccgagc aggaccggga     300
   ttctcactaa gcgggcgccg tcctacgacc cccgcgcgct ttcaggacca ctcgggcacg     360
   tggcaggtcg cttgcacgcc cgcggactat ccctgtgaca ggaaaaggta cgggccattt     420
   ggcaaactaa ggcacagagc ctcaggcgga agctgggaag gcgccgcccg gcttgtaccg     480
   gccgaagggc catccgggtc aggcgcacag ggcagcggcg ctgccggagg accagggccg     540
   gcgtgccggc gtccagcgag gatgcgcaga ctgcctcagg cccggcgccg ccgcacaggg     600
   catgcgccga cccggtcggg cgggaacacc ccgcccctcc cgggctccgc cccagctccg     660
   cccccgcgcg ccccggcccc gcccccgcgc gctctcttgc ttttctcagg tcctcggctc     720
   cgccccgctc tagaccccgc cccacgccgc catccccgtg cccctcggcc ccgcccccgc     780
   gccccggata tgctgggaca gcccgcgccc ctagaacgct ttgcgtcccg acgcccgcag     840
   gtcctcgcgg tgcgcaccgt ttgcgacttg gtgagtgtct gggtcgcctc gctcccggaa     900
   gagtgcggag ctctccctcg ggacggtggc agcctcgagt ggtcctgcag gcgccctcac     960
   ttcgccgtcg ggtgtggggc cgccctgacc cccacccatc ccgggcgagc tccaggtgcg    1020
   ccccaagtgc ctcccaggtg ttgcccagcc tttccccggg cctggggttc ctggactagg    1080
   ctgcgctgca gtgactgtgg actggcgtgt ggcgggggtc gtggcagccc ctgccttacc    1140
   tctaggtgcc agccccaggc ccgggccccg ggttcttcct acccttccat gctgccagct    1200
   ttccctccgc cagctgctcc aggaagcttc cagaagcccc tgcgcgggcc ttggcttgca    1260
   gcaacccttt agcatactta ggcagagtcc catatttcct tcctgctgga ggccaagttc    1320
   taggggcctt ctggttacta tggctggtgt ttgtgtacat cataccctaa ctgtattcat     1380
   caacacttag agtaagcaag gctcgctgga gagccacaca cactgggcac cgtaatgtcg    1440
   gttataacac cgcagaggag ttctgaacta tgtatttcgc actcctgggt tcatcatctc    1500
   ctgaaatctc agggtggtgt ttgctctcag ttgcttcagc tgagtagctg gctttctgtc    1560
   ctggaaagca gactttgtac atgtgtgtgc aacctatgcc tgctgagatc atcatcagac    1620
   agggaagcgg cttggtccag agagctgttc tcagtagaat gttaagcaca gagagctgag    1680
   aattagactg gttatttaca tagacatcca aatagaaacc tatagagtat ctgttaagtc 1740
   aggctctccc gtcatctccc ccatccctgg gcaggtgtct aggagatggt tttgttattt 1800
   tcagggccct ctcaatggct aaaacactct gggagatgaa caagatttta aaaacccaaa 1860
   gcttagcgca cctggtgggt gggggtgtga aaactttgaa ggaaaccgcg tcaagagcct 1920
   ggctgattgt taatatcacg ttaactcaga gggccaggat acttgcccag acccggagtc 1980
   tgcctgcaag tagcagagga gagctggcct tgctctgccg cgtgtctttc ttcctgggcc 2040
   ctctgtctcg ggttggaatt tgaatagtgg agtagtgtct gccacagtca ggggcctgga 2100
   tgaagtagac caccagtacg gagctatact caggtacttt tcaatgtata tatcttcatt 2160
   aaaaaaaaaa aatctgggcc aggcacagtg ggtcacgcct gtaatctcag cactttggga 2220
   ggccgaggtg ggtggatcac ctgaagtcag gagtttgaga ccagcctggc caacatggtg 2280
   aaacccagtt tctactaaaa acacaaaaat tatccaggcg tggtggtgca tgctggtaat 2340
   cccagttact tgggagactg aggcaggaga attgcttgaa cctgggaggc ggaggttgca 2400
   gtgagccgag attgcgccat tgcactccaa cctgggcgac gagagtgaa 2449
<210> 93
   <211> 2928
   <212> DNA
   <213> Human SEPT9 region of interest (ROI) 1, preferred for methylation
   analyses (SEPT9 "Region of interest" (ROI) 1, präferiert für Methylierungsanalysen)
<400> 93
   tttcagaggg ggtggggagg ggcaagtggg cagcgagcga cctcagaccc aggatgagct 60
   gtcaggcgct ccccggccac acattcaagg gaccggagtg cagttgtagc gttgcggcct 120
   gctgcttcgg gggtgggggt gttgttccat gctgtgaatt ctcacatggc ccctgactct 180
   gggcagaggc cgagggtcta agggacgggg tgacagggag agcatgcagg agtgggtttc 240
   tggctttcca gggcgagtgg aagaagcgcc tctctctctt gtaggtgaca gacctggggg 300
   gcccttcttg aggatgagag cctgttgctt ctcaagttct gtgtctaacc caggtcccca 360
   ggtctacccc agcccctcgg ccctgcctgc cttgtggatg atatagttta agggtagaga 420
   ccgctggcct ggagggaagg ctaggcctca ggttagggcc cagaagggag ggagaagccc 480
   ttggggcagc tccctttctg ctcactcact gcctagctcc ttccttcaca ccttccttcg 540
   gaaacgtctg ctcctgacaa ggtctacttc ctgctctcag gaggccctta ttgtggagga 600
   agggaggcgt cgcccgtccc tggcttctct gacagccgtg ttccatcccc gccctgtgcc 660
   ccttctcccg gacagtgcct tctccagggc tcacccagga gggtgcagcg gtggcccccg 720
   gggcggtggt cgtggtgggg gtgttagctg caggggtgcc ctcggtgggt gggagttggt 780
   ggcctctcgc tggtgccatg ggactcgcat gttcgccctg cgcccctcgg ctcttgagcc 840
   cacaggccgg gatcctgcct gccagccgcg tgcgctgccg tttaaccctt gcaggcgcag 900
   agcgcgcggc ggcggtgaca gagaactttg tttggctgcc caaatacagc ctcctgcaga 960
   aggaccctgc gcccggggaa ggggaggaat ctcttcccct ctgggcgccc gccctcctcg 1020
   ccatggcccg gcctccacat ccgcccacat ctggccgcag cggggcgccc ggggggaggg 1080
   gctgaggccg cgtctctcgc cgtcccctgg gcgcgggcca ggcggggagg aggggggcgc 1140
   tccggtcgtg tgcccaggac tgtcccccag cggccactcg ggccccagcc ccccaggcct 1200
   ggccttgaca ggcgggcgga gcagccagtg cgagacaggg aggccggtgc gggtgcggga 1260
   acctgatccg cccgggaggc gggggcgggg cgggggcgca gcgcgcgggg aggggccggc 1320
   gcccgccttc ctcccccatt cattcagctg agccaggggg cctaggggct cctccggcgg 1380
   ctagctctgc actgcaggag cgcgggcgcg gcgccccagc cagcgcgcag ggcccgggcc 1440
   ccgccggggg cgcttcctcg ccgctgccct ccgcgcgacc cgctgcccac cagccatcat 1500
   gtcggacccc gcggtcaacg cgcagctgga tgggatcatt tcggacttcg aaggtgggtg 1560
   ctgggctggc tgctgcggcc gcggacgtgc tggagaggac cctgcgggtg ggcctggcgc 1620
   gggacggggg tgcgctgagg ggagacggga gtgcgctgag gggagacggg acccctaatc 1680
   caggcgccct cccgctgaga gcgccgcgcg cccccggccc cgtgcccgcg ccgcctacgt 1740
   gggggaccct gttaggggca cccgcgtaga ccctgcgcgc cctcacagga ccctgtgctc 1800
   gttctgcgca ctgccgcctg ggtttccttc cttttattgt tgtttgtgtt tgccaagcga 1860
   cagcgacctc ctcgagggct cgcgaggctg cctcggaact ctccaggacg cacagtttca 1920
   ctctgggaaa tccatcggtc ccctcccttt ggctctcccc ggcggctctc gggccccgct 1980
   tggacccggc aacgggatag ggaggtcgtt cctcacctcc gactgagtgg acagccgcgt 2040
   cctgctcggg tggacagccc tcccctcccc cacgccagtt tcggggccgc caagttgtgc 2100
   agcccgtggg ccgggagcac cgaacggaca cagcccaggt cgtggcaggg tctagagtgg 2160
   gatgtcccat ggcccccatc caggcctggg gatatcctca tccgcctccc agaatcgggc 2220
   cgtgggggac agaaggggcc tgcgtgcggg cagggagagt attttggctc tctcctgtct 2280
   tcggggttta caaagtgtgt tgggacttgc ggggctgctc tgtccaagcc tgggtctggc 2340
   gtccgcgtct ctgagcctgt gagtgcgtgc gctttcctgc gtcctcttga ctgccggtgc 2400
   tggggctctg cgtcctgcgt ccgcgggagt aaatacagca ggcgaagggg aagctcacac 2460
   aatggtctcc agcgctctgg ggcagggctt ctgaggggcg ggcctgcctc tgccgggacc 2520
   tggagccccc gcccctcgga gaggctccta ggctgacttg ggcagagccc tctggtgggc 2580
   cgggaggggg aaaggctgtg ttgaaatgag caaactgtcc aggtgtcagg ccaagctggg 2640
   aggtgaccag cctgaggtcc tccccgctcc atggccagaa ccagggctga catctgggtg 2700
   tcctgagccc agctgcccac acggcccacc tggggtcagc cctatctgag tgggggaggc 2760
   ggggcctcct gggggaccag aactttggct ggacgccaag cagagtgcca gtggctgttc 2820
   ttcagggctg ggcctgagga gggtgtgggg cggcgaaggg acgggagggg gttgtgatcc 2880
   agtggccact ggcgctgtgc agagtgtgag ctggaaacat cgtagtta 2928
<210> 94
   <211> 8485
   <212> DNA
   <213> Human TP53 region of interest (ROI) (TP53 "Region of interest" (ROI))
<400> 94

## Beschreibung

### Bezugnahme auf frühere Anmeldung

Diese Patentanmeldung beansprucht die Priorität der deutschen Patentanmeldung mit dem Aktenzeichen 10 2015 009 187.5, deren Offenbarungsgehalt hiermit durch Rückbezug aufgenommen wird.

### Sequenzprotokoll

Die Anmeldung beinhaltet ein elektronisches Sequenzprotokoll im txt-Format nach WIPO ST.25 Standard mit 94 Sequenzen als Teil der Beschreibung.

### Gebiet der Erfindung

Die Erfindung betrifft molekulardiagnostische Verfahren im Bereich der Onkologie zur Bestimmung von Mutationen in genomischer DNA. Die Erfindung betrifft weiterhin die Verwendung solcher molekulardiagnostischer Verfahren im Zusammenhang mit der Diagnose, Prognose, Prädiktion und Verlaufskontrolle von malignen Erkrankungen. Ferner betrifft die Erfindung ein Kit zur Durchführung der angegebenen Verfahren beziehungsweise für die angegebenen Verwendungen. Insbesondere handelt es sich bei den erfindungsgemäßen Verfahren um *in vitro* Verfahren.

### Hintergrund der Erfindung

Im Zeitalter der personalisierten Medizin spielen zielgerichtete Therapien eine übergeordnete Rolle. Im Bereich der Onkologie sind bereits einige Medikamente in erfolgreicher Anwendung, die gezielt Proteine inhibieren, welche in Tumoren mutiert sind. Ein Beispiel hierfür ist das Medikament Vemurafenib (Trivialname: Zelboraf), welches selektiv das Onkogen *BRAF* inhibiert. Bei etwa 70% der malignen Melanomtumoren ist dieses Gen mutiert. Das resultierende Genprodukt hat durch diese Mutation eine erhöhte Aktivität. Patienten, deren Tumoren eine solche Mutation aufweisen, sprechen daher gut auf eine Therapie mit dem BRAF Inhibitor Vemurafenib an. Die Mutation von *BRAF* ist somit ein prädiktiver Biomarker für das Ansprechen auf eine Vemurafenib-Behandlung. Daher ist heutzutage die Bestimmung einer Mutation von *BRAF* als Routinediagnostik etabliert, um Patienten zu identifizieren, bei denen Vemurafenib angewendet werden kann.

Ein weiteres Beispiel für eine solche zielgerichtete Therapie, bei welcher der Mutationsstatus eines Gens relevant ist, ist das Medikament Cetuximab (Trivialname: Erbitux). Cetuximab inhibiert selektiv den epidermalen Wachstumsfaktorrezeptor (EGFR) und wird beispielsweise zur Behandlung von metastasiertem Darmkrebs verwendet. Allerdings wirkt dieses Medikament nicht, wenn ein anderes Gen (*KRAS*) in dem Signalweg eine Mutation aufweist. Daher ist die Bestimmung der *KRAS* Mutation prädiktiv für das NichtAnsprechen des Patienten auf eine Behandlung mit Cetuximab.

Die Bestimmung einer Mutation der entsprechenden Gene spielt demnach eine entscheidende Rolle. Sie erfolgt in der Regel durch Sequenzierung der DNA. Üblicherweise wird zu diesem Zweck ein operativ entfernter Tumor oder eine Biopsie vom Tumor zunächst pathologisch beurteilt und das in der Gewebeprobe enthaltene Tumorgewebe markiert. Ist in dem vom Pathologen identifizierten Tumormaterial im Anschluss die Sequenzierung negativ, so ist gezeigt, dass die Mutation nicht vorliegt. Ergibt die Sequenzierung ein positives Resultat, dann ist das Vorliegen der Mutation bewiesen.

Problematisch ist, dass in jedem Stadium dieser Diagnosekette Probenmaterial falsch identifiziert, behandelt oder analysiert werden und auf diese Weise zu falsch-negativen Resultaten führen kann. Möglich ist zum Beispiel, dass der Histopathologe gesundes Normalgewebe als Tumormaterial identifiziert oder dass zuviel gesundes Normalgewebe in den weiteren analytischen Arbeitsablauf mitgeführt wird. Unter solchen Voraussetzungen ist eine Mutation auf molekularer Ebene in der Regel nur noch schwer oder gar nicht mehr erkennbar. Unter Umständen kann die Beurteilung des Probenmaterials durch mehrere Pathologen Abhilfe schaffen. Dies führt jedoch zu einer erhöhten Bearbeitungszeit und erhöhtem Personalaufwand und folglich gesteigerten Kosten. Auch ist damit nicht die zusätzliche Gefahr gebannt, dass der Tumor nur einen geringen Anteil an neoplastischen Zellen enthält oder die Tumorzellen stark zerstreut im Normalgewebe vorliegen und somit die molekulare Analyse trotz sorgfältiger Beurteilung falsch-negative Ergebnisse produzieren kann.

DE 19951189 A1 offenbart ein Verfahren zur Unterscheidung von Cytosin-zu-Thymin Mutationen und zum Nachweis von Single Nucleotide Polymorphisms (SNPs) oder Punktmutationen in genomischer DNA.

In US 2005/064401 A1, US 2013/338032 A1 und US 2008/0050738 A1 werden genetische bzw. epigenetische Parameter anhand des Methylierungszustandes von Cytosin in chemisch umgewandelter DNA bestimmt.

WO 2013/084075 A2 offenbart ein weiteres Verfahren zur Bestimmung von Mutationen und epigenetischen Veränderungen in chemisch modifizierten Nukleinsäureproben.

Yaping et al. (Genome Biology 13, 2012, R61) beschreiben ein Verfahren zur Detektion von SNPs in Bisulfit-konvertierter DNA.

In Laskar et al. (Tumor Biology 36, 2015, 4661-4670) wird die Assoziation von HPV mit genetischen und epigenetischen Veränderungen im Darmkrebs untersucht.

In WO 2013/082043 A1 wird die Promotormethylierung von *RBP1* als molekularer Biomarker zur Vorhersage des Überlebens und Ansprechens auf die Behandlung bei Gliomen untersucht.

Falsche molekulardiagnostische Resultate können fatale Folgen haben. Im schlimmsten Fall erhalten therapierbare beziehungsweise therapiebedürftige Patienten keine oder nicht die passende Therapie. Andererseits ist aber auch möglich, dass Patienten unnötige Therapien erhalten oder die Therapie zu einem falschen Zeitpunkt erhalten.

Es ist daher eine Aufgabe dieser Erfindung, Verfahren und deren Verwendungen sowie Kits bereitzustellen, welche eine robuste, insbesondere sensitivere und/oder spezifischere, und kosteneffiziente molekulare Diagnose von Mutationen beziehungsweise malignen Erkrankungen ermöglichen und eine differenzierte klinische Entscheidungsfindung unterstützen und auf diese Weise die vorgenannten Probleme zumindest teilweise verringern oder lösen.

Diese Aufgabe wird durch die Gegenstände der unabhängigen Ansprüche 1 und 5 bis 8 gelöst.

Bevorzugte Varianten der Erfindung ergeben sich aus der Beschreibung und den abhängigen Ansprüchen.

### Definitionen und allgemeine Erläuterungen

In dieser Beschreibung werden verschiedene Dokumente zitiert, um einen allgemeinen technischen Hintergrund in Bezug auf die vorliegende Erfindung zu vermitteln. Auf die Offenbarung und Lehre dieser Dokumente wird in Ergänzung nachfolgender Beschreibung vollinhaltlich Bezug genommen, um Wiederholungen zu vermeiden.

Die nachfolgenden Definitionen und allgemeinen Erläuterungen sollen den fachkundigen Leser beim Verständnis, in der Auslegung und bei der Ausübung der vorliegenden Erfindung anleiten und unterstützen. Vorbehaltlich anderer Angaben sollen alle technischen und wissenschaftlichen Begriffe diejenige Bedeutung haben, welche dem üblichen Begriffsverständnis eines Durchschnittsfachmanns auf dem Gebiet der vorliegenden Erfindung entspricht.

Die verschiedenen Aspekte und Varianten der Erfindung involvieren Techniken und Methoden aus der molekularbiologischen Routinepraxis. Zweckdienliche Laborhandbücher für diese Techniken und Methoden stehen dem Fachmann ohne Weiteres zur Verfügung, beispielsweise "Molecular Cloning, A Laboratory Manual" von M.R. Green und J. Sambrook, 4th Edition, 2012, Cold Spring Harbor Laboratory Press; "Next-Generation Sequencing: Current Technologies and Applications" von Jianping Xu, 2014, Caister Academic Press; "Next-Generation DNA Sequencing Informatics" von Stuart M. Brown, 2nd Edition, 2015, Cold Spring Harbor Laboratory Press.

Das Design von Primern und Oligonukleotid-Sonden gehört zu den Fachkenntnissen eines Molekularbiologen oder -genetikers. Ein geeigneter Algorithmus, welcher das Design von Primern und Sonden für umgewandelte (Bisulfit-konvertierte) DNA ermöglicht, ist MethPrimer (Li, L.C. and Dahiya, R., Bioinformatics, 2002, 18, 1427-31). BiSearch (Tusnády G.E. et al., Nucleic Acids Research, 2005, 33, e9) ist ein weiterer geeigneter Primerdesign-Algorithmus, der sowohl für umgewandelte (Bisulfit-konvertierte) als auch für genomische, nicht-umgewandelte DNA geeignet ist.

MethBlast (Pattyn, F. et al., BMC Bioinformatics, 2006, 7, 496) ist ein Suchprogramm für die Analyse von *in silico* umgewandelter (Bisulfit-konvertierter) DNA, sowohl in methyliertem als auch unmethyliertem Zustand. Dieses Programm ist vor allem für das Finden von Primer-Bindestellen und damit die Optimierung der Spezifität von PCR-Amplifikationen entwickelt.

So, wie sie hier verwendet werden, schließen unbestimmte Artikel wie "ein" oder "eine" die Möglichkeit mit ein, dass auch zwei oder mehrere dieser Merkmale vorhanden sein können.

Als "Gen" wird hier ein Abschnitt auf der DNA bezeichnet, der regulatorische, transkribierte und/oder funktionelle Sequenzregionen umfasst und somit die Grundinformationen zur Herstellung einer biologisch aktiven RNA enthält.

Die Nomenklatur für die Bezeichnung von Genen und deren Nukleotiden erfolgt entsprechend der Empfehlung des "Human Genome Organisation Gene Nomenclature Committee" (HGNC) mit Stand vom 30. Juni 2015. Ein Genstamm wird beispielsweise mit kursiven lateinischen Großbuchstaben bezeichnet (z.B. *EGFR, BRAF*). Gegebenenfalls folgen dem Stammsymbol eine oder mehrere arabischen Zahlen oder einer Kombination von arabischen Zahlen und lateinischen Buchstaben zur Bezeichnung eines Familienmitglieds des Genstamms (z.B. *BRCA1, BRCA2, SHOX2) .*

Die Beschreibung von Genen auf DNA-Ebene, also beispielsweise die Benennung von Nukleotiden, Sequenzvariationen und Mutationen, folgt den Empfehlungen der "Human Genome Variation Society" (HGVS) zur Beschreibung von Sequenzvarianten mit Stand vom 30. Juni 2015 (den Dünnen, J.T. and Antonarakis, S.E., Human Mutation, 2000, 15, 7-12).

Die hier beschriebenen Gene sind über die "GenBank" des National Institute of Health, USA, mit Stand vom 30. Juni 2015 öffentlich verfügbar (Benson D.A. et al., Nucleic Acids Research, 2013, 41, D36-42).

Wenn im Folgenden Bezug auf "mindestens 95% Sequenzidentität" genommen wird, schließt das höhere Sequenzidentitäten wie zum Beispiel mindestens 96%, mindestens 97%, mindestens 98%, mindestens 99% oder 100% Sequenzidentität mit ein. Die Sequenzidentität von zwei Nukleinsäuresequenzen kann zum Beispiel mit dem Algorithmus ClustalW bestimmt werden (Thompson et al., Nucleic Acids Research, 1994, 22, 4673-4680) .

Als "genomische DNA" im Sinne der Erfindung ist jeder Teil derjenigen DNA zu verstehen, welcher zumindest teilweise das Genom einer oder mehrere Zellen bildet oder gebildet hat. Insbesondere kann genomische DNA auch als zellfreie DNA vorliegen, beispielsweise zirkulierende DNA, welche aus einer oder mehreren Zellen eines Tumors freigesetzt wurde. Entsprechend schließt "genomische DNA" auch aus Zellen isolierte und gegebenenfalls aufgereinigte DNA mit ein.

Eine "Mutation" umfasst eine dauerhafte Veränderung im Erbgut, deren Ursprung in einer einzelnen Zelle liegt und die anschließend an die Tochterzellen weitergegeben wird. In verschiedenen Varianten der Erfindung umfasst "Mutation" eine Genmutation, beispielsweise eine Punktmutation, Deletion, Insertion, Duplikation, Amplifikation, Translokation, Fusion oder Inversion. In gewissen Varianten der Erfindung kann eine Mutation durch eine Allelhäufigkeit von kleiner gleich 1% in der Weltbevölkerung charakterisiert sein und ist somit abzugrenzen von einem Polymorphismus, welcher definitionsgemäß mehr als 1% Allelhäufigkeit in der Weltbevölkerung aufweist.

So, wie der Begriff hier verwendet wird, umfasst eine Mutation auch die Integration von DNA viraler Herkunft in die genomische DNA im Sinne einer Insertionsmutation. Nicht beschränkende Beispiele für solche Viren, welche das Vermögen haben, sich in das Genom der infizierten Zelle zu integrieren und Insertionsmutationen herbeizuführen, sind das Epstein-Barr Virus (EBV), das Hepatitis B Virus (HBV), Humane Papillomaviren (HPV), das Humane T-lymphotrope Virus 1 (HTLV-1), und das Merkelzell-Polyomavirus (MCPyV).

Entsprechend umfasst "Mutationsanalyse" eine Untersuchung, ob und gegebenenfalls in welcher Form beziehungsweise in welchem Ausmaß eine derartige Veränderung im Erbgut vorliegt. In verschiedenen Varianten der Erfindung kann dies beispielsweise anhand einer Abweichung der Basensequenz, des Molekulargewichts, der Hybridisierungsstärke oder einer anderen geeigneten Eigenschaft eines Abschnitts der zu analysierenden genomischen DNA von einem Normwert festgestellt werden. Als Normwert ist zum Beispiel die entsprechende Eigenschaft, insbesondere die Basensequenz, eines entsprechenden Abschnitts einer genomischen DNA geeignet, welche die Veränderung nicht aufweist, nachfolgend auch als Wildtyp, wildtypischer Zustand oder auch Referenz-DNA bezeichnet. Als Wildtyp ist beispielsweise genomische DNA aus Normalgewebe, insbesondere gesundem Gewebe, desselben Individuums geeignet. Dies kann zum Beispiel der bevorzugte Wildtyp in Bezug auf die Bestimmung einer somatischen Mutation sein. Als Wildtyp kann auch ein Referenzgenom verwendet werden. Ein geeignetes Referenzgenom umfasst die humane Genomversion des Genome Reference Consortium Genome Reference Consortium Human Build 38 patch release 2 (GRCh38.p2) mit Stand vom 16. Juli 2015. Ein Referenzgenom kann zum Beispiel der bevorzugte Wildtyp in Bezug auf die Bestimmung einer Keimbahnmutation und/oder somatischen Mutation sein. In bestimmten Varianten wird unter "Mutationsanalyse" die Feststellung verstanden, ob die zu analysierende genomische DNA eine Abweichung in der Basensequenz von einer wildtypischen DNA, insbesondere von einem bestimmten Gen oder einer bestimmten Sequenz der wildtypischen DNA, aufweist. Diese Varianten schließen ausdrücklich mit ein, dass auch nur eine Abweichung in einem Teil des Gens oder der bestimmten Sequenz von der wildtypischen DNA von der Mutationsanalyse umfasst ist, um die Mutation zu bestimmen.

Unter einem "Polymorphismus" wird das Auftreten mehrerer aus Sequenzvariationen resultierenden Genvarianten innerhalb einer Population verstanden. Ein Polymorphismus betrifft die Keimbahn, kann von einem Individuum an ein anderes vererbt werden und tritt in allen Zellen eines Organismus gleichermaßen auf. Die seltenere Genvariante innerhalb einer Population muss im Unterschied zu einer Sequenzvariation oder einer Mutation der Keimbahn definitionsgemäß eine Auftretenshäufigkeit (Allelfrequenz) von über einem Prozent haben.

Unter der "Keimbahn" versteht man diejenige Zellenfolge (Genealogie), aus der die Keimzellen (generative Zellen, Gameten) hervorgehen. Die von der Keimbahn abzweigenden somatischen Zellinien bilden den Körper (das Soma). Unter einer "Keimbahnmutation" wird demnach eine Mutation verstanden, die in den Zellen der Keimbahn eines Organismus auftritt, alle Zellen des Organismus gleichermaßen betrifft und von einem Individuum an ein anderes vererbt werden kann. Dagegen wird unter einer "somatischen Mutation" eine Mutation verstanden, die außerhalb der Keimbahn auftritt, das heißt in somatischen Zellen (Somazellen). Diese Mutation betrifft nur einen Teil der Zellen eines Organismus, zum Beispiel Tumorzellen. Diese Mutation kann nicht von einem Individuum an ein anderes vererbt werden.

Unter einer "rekurrenten Mutation" wird eine Mutation verstanden, welche sehr häufig in einer bestimmten malignen Erkrankung auftritt. Insbesondere liegt eine rekurrente Mutation vor, wenn diese bei mindestens 1%, mindestens 3% oder mindestens 10% der Fälle dieser bestimmten malignen Erkrankung auftritt. Unter einem "rekurrent mutierten Gen" wird ein Gen verstanden, welches sehr häufig in einer bestimmten malignen Erkrankung mindestens eine Mutation aufweist. Insbesondere liegt ein rekurrent mutiertes Gen vor, wenn dieses bei mindestens 5%, mindestens 10% oder mindestens 20% der Fälle dieser bestimmten malignen Erkrankung mindestens eine Mutation aufweist.

Ein "CpG-Dinukleotid" ist ein DNA-Motiv, welches in allgemein gültiger Leserichtung von 5' nach 3' die Nukleosidfolge Cytidin-Phosphat-Guanosin aufweist. Guanosin besteht aus der Nukleinbase Guanin und dem Zucker β-D-Ribose. Cytidin besteht aus der Nukleinbase Cytosin und dem Zucker β-D-Ribose.

Eine "Methylierungsanalyse" im Sinne der vorliegenden Erfindung umfasst die Bestimmung des Methylierungszustands eines CpG-Dinukleotids oder mehrerer CpG-Dinukleotide aus einem bestimmten Sequenzkontext. In verschiedenen Varianten der Erfindung wird unter "Methylierungsanalyse" die Feststellung verstanden, ob das Cytosin in dem oder den CpG-Dinukleotid(en) methyliert vorliegt. Die Methylierungsanalyse kann eine einzelne Kopie des CpG-Dinukleotids umfassen. Die Methylierungsanalyse kann auch eine Vielzahl von Kopien des CpG-Dinukleotids umfassen, beispielsweise wenn die DNA einer Vielzahl von Zellen in der genomischen DNA enthalten ist. In diesem Fall kann die Methylierungsanalyse einen Methylierungsstatus beziehungsweise einen Methylierungswert des CpG-Dinukleotids liefern, das heißt einen Querschnittswert, welcher den Methylierungszustand der Vielzahl von Kopien des CpG-Dinukleotids umfasst.

Ein CpG-Dinukleotid kann in einem Gewebe oder einem Zelltyp "aberrant methyliert" sein beziehungsweise einen "aberranten Methylierungszustand" haben, womit hier ein CpG-Dinukleotid gemeint ist, welches in der genomischen DNA im Vergleich zu einem Normwert hypermethyliert oder hypomethyliert vorliegt. Als Normwert kann zum Beispiel der Methylierungszustand des entsprechenden CpG-Dinukleotids in der genomischen DNA des gleichen Gewebe- oder Zelltyps dienen, wobei dieser gleiche Gewebe- oder Zelltyp sich in einer Eigenschaft unterscheidet, bezogen auf die das CpG-Dinukleotid "aberrant methyliert" ist. "Aberrant methyliert" sind beispielsweise Gene, die in Tumorzellen eine höhere oder niedrigere Methylierung aufweisen als in dem Gewebe, aus dem der Tumor entstanden ist. Der "aberranten Methylierungszustand" bleibt erhalten, wenn die genomische DNA aus dem Tumor oder aus der Zelle freigegeben wird, zum Beispiel in Form von zirkulierender DNA in das Blut. Geeignete Normwerte können demnach zum Beispiel experimentell ermittelt werden, indem eine Methylierungsanalyse des gleichen Zelltyps oder des gleichen Gewebetyps durchgeführt wird, welcher jedoch nicht die Eigenschaft trägt, bezogen auf die das mindestens eine CpG-Dinukleotid als "aberrant methyliert" bestimmt werden soll. Hypermethyliert und hypomethyliert können demnach eine gegenüber dem Normwert höhere beziehungsweise eine niedrigere Methylierung bezeichnen. Hypermethylierte CpG-Dinukleotide zeigen einen Methylierungswert, der höher ist als der Normwert, insbesondere der mindestens 25% über dem Normwert liegt. Hypomethylierte CpG-Dinukleotide zeigen einen Methylierungswert, der niedriger ist als der Normwert, insbesondere der mindestens 25% unter dem Normwert liegt. Ist der Normwert eines bestimmten CpG-Dinukleotid null, dann ist das bestimmte CpG-Dinukleotid nicht hypomethyliert.

Maligne oder "bösartige" Erkrankungen umfassen Erkrankungen, die durch einen Krankheitsverlauf gekennzeichnet sind, der fortschreitend zerstörerisch ist und möglicherweise auch zum Tod des Patienten führen kann. Maligne Erkrankungen umfassen maligne Gewebeneubildungen, zum Beispiel Neoplasien oder Tumoren, wobei die Malignität durch unkontrolliertes, raumforderndes, verdrängendes, infiltratives und/oder invasives Wachstum gekennzeichnet sein kann. Maligne Tumoren sind in der Regel in der Lage, Tochtergeschwülste (Metastasen) zu bilden. Maligne Tumoren sind zum Beispiel Karzinome, Sarkome, Melanome, Blastome und Teratome. Maligne Erkrankungen umfassen auch hämatologische maligne Erkrankungen, das heißt bösartige Erkrankungen, die das Blutsystem oder das blutbildende System betreffen, beispielsweise Leukämien, Lymphome, myeloproliferative Erkrankungen und myelodysplastische Syndrome. Leukämien umfassen eine Gruppe von malignen Erkrankungen, bei denen unreife hämatopoetische Zellen sich bösartig verändert haben, sich übermäßig stark vermehren und zu einer Anhäufung von Zellen im peripheren Blut führen. Lymphome umfassen Erkrankungen, bei denen Zellen des lymphatischen Systems entartet sind. Myeloproliferative Erkrankungen umfassen eine Gruppe von Erkrankungen, bei denen eine oder mehrere blutbildende Zellreihen stark vermehrt auftreten. Myelodysplastische Syndromen umfassen eine klonale Expansion von Vorläuferzellen aller blutbildenden Zellreihen, wobei eine chronisch verlaufende Differenzierungsstörung der blutbildenden Stammzellen zugrunde liegt.

Biomarker sind charakteristische Indikatoren oder/und biologische Merkmale, die objektiv gemessen werden können und Rückschluss über den Status eines normalen biologischen oder eines krankhaften Prozesses in einem Organismus, beziehungsweise die Antwort eines normalen oder krankhaften Prozesses auf eine Intervention, beispielsweise eine Operation, eine Bestrahlung oder eine medikamentöse Behandlung, zulassen. Biomarker sind häufig (bio-)chemische Substanzen, wie zum Beispiel Proteine, Hormone, Metaboliten, Zucker und Nukleinsäuren, sowie Modifikationen davon.

So, wie die Begriffe hier verwendet werden, beinhaltet "Diagnose" eine Feststellung beziehungsweise Bestimmung einer malignen Erkrankung, "Prognose" eine Aussage über eine Entwicklung einer malignen Erkrankung in der Zukunft, insbesondere in Abwesenheit einer therapeutischen Maßnahme, "Prädiktion" eine Vorhersage eines Ansprechverhaltens einer malignen Erkrankung auf eine bestimmte Therapie und "Verlaufskontrolle" eine Feststellung eines Zustands einer malignen Erkrankung zu verschiedenen Zeitpunkten, beispielsweise vor, während und nach einer Therapie. Insbesondere bezeichnen diese Begriffe deduktive Schritte in Zusammenhang mit einem vorausgehenden *in vitro* Verfahren, sodass kein erfindungswesentlicher technischer Schritt am menschlichen oder tierischen Körper stattfindet.

Sowohl die vorstehende allgemeine Beschreibung als auch die nachfolgende detaillierte Beschreibung sind als Beispiel aufzufassen und sollen zur Erläuterung der beanspruchten Erfindung dienen. Weitere Vorteile und Merkmale der Erfindung sind aus der nachstehenden Beschreibung, den Zeichnungen und den Patentansprüchen ersichtlich. Auch wenn die Erfindung anhand ihrer bevorzugten Ausführungsformen erläutert wird, können viele weitere Variationen vorgenommen werden, ohne über den Umfang der vorliegenden Erfindung hinauszugehen. Daher ist es vorgesehen, dass die beiliegenden Patentansprüche Variationen und Kombinationen von Merkmalen abdecken, die im tatsächlichen Umfang der Erfindung enthalten sind, auch wenn diese nicht ausdrücklich in den Ansprüchen abgebildet sind.

### Kurze Beschreibung der Figuren

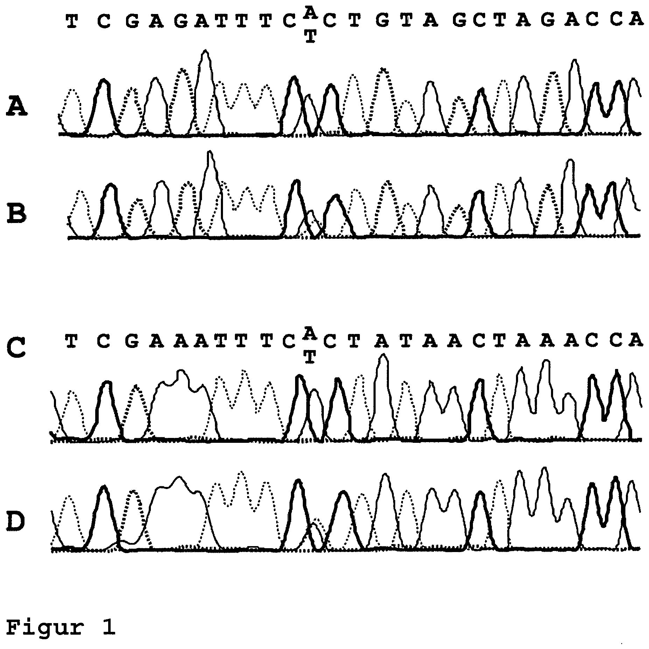

Figur 1 zeigt das Ergebnis einer Referenzanalyse einer Sequenz des *BRAF* Gens mit nicht-umgewandelter genomischer DNA aus Normalgewebe (A) und aus malignem Gewebe (B) sowie das Ergebnis der erfindungsgemäßen Analyse der Sequenz des *BRAF* Gens mit umgewandelter genomischer DNA aus Normalgewebe (C) und aus malignem Gewebe (D).

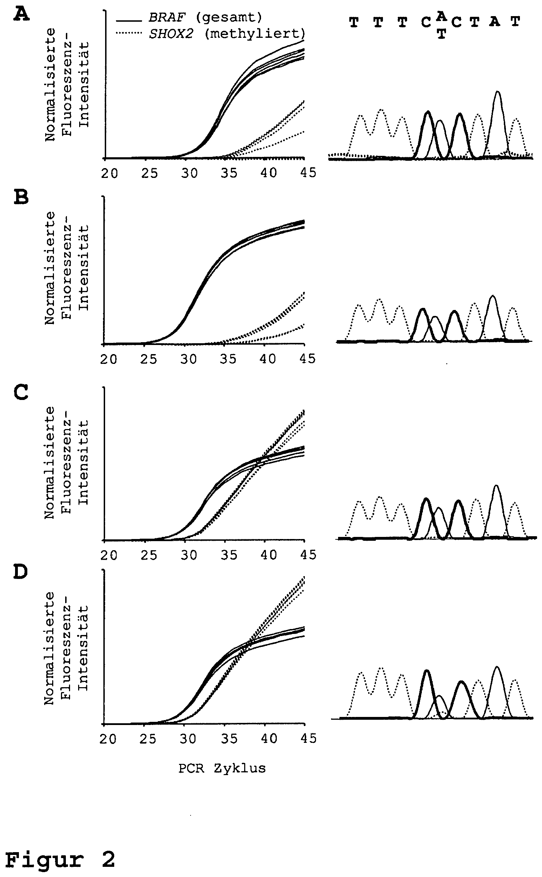

Figur 2 zeigt die Ergebnisse einer Kombination aus Methylierungsanalyse von *SHOX2* und Mutationsanalyse von *BRAF* im Plasma von Melanompatienten. Links: Echtzeit-PCR Resultate für die gesamte Kopienzahl an *BRAF* DNA-Sequenzen (solide Linien) und die Kopienzahl an methylierten *SHOX2* DNA-Sequenzen (gestrichelte Linien). Rechts: Sequenzierung des bei der Echtzeit-PCR Quantifizierung generierten *BRAF* PCR-Amplifikats. A: Patient mit geringer Tumorlast und *BRAF* Wildtyp im Primärtumor. B: Patient mit geringer Tumorlast und *BRAF* V600E Mutation Primärtumor. C: Patient mit hoher Tumorlast und *BRAF* Wildtyp im Primärtumor. D: Patient mit hoher Tumorlast und *BRAF* V600E Mutation Primärtumor.

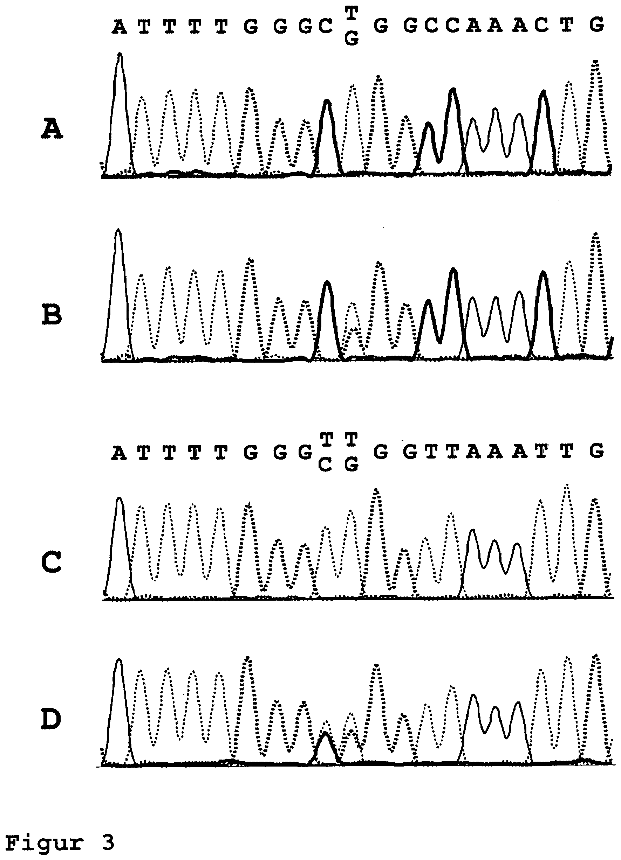

Figur 3 zeigt die Ergebnisse der Sequenzierung des *EGFR* Lokus (Exon 21) mit L858R Mutation. A: Referenzanalyse von nicht-umgewandelter genomischer DNA des gesunden, an den Tumor angrenzenden Gewebes. B: Referenzanalyse von nicht-umgewandelter genomischer DNA des Tumorgewebes. C: erfindungsgemäße Analyse von umgewandelter genomischer DNA des gesunden Gewebes. D: erfindungsgemäße Analyse von umgewandelter genomischer DNA des Tumorgewebes.

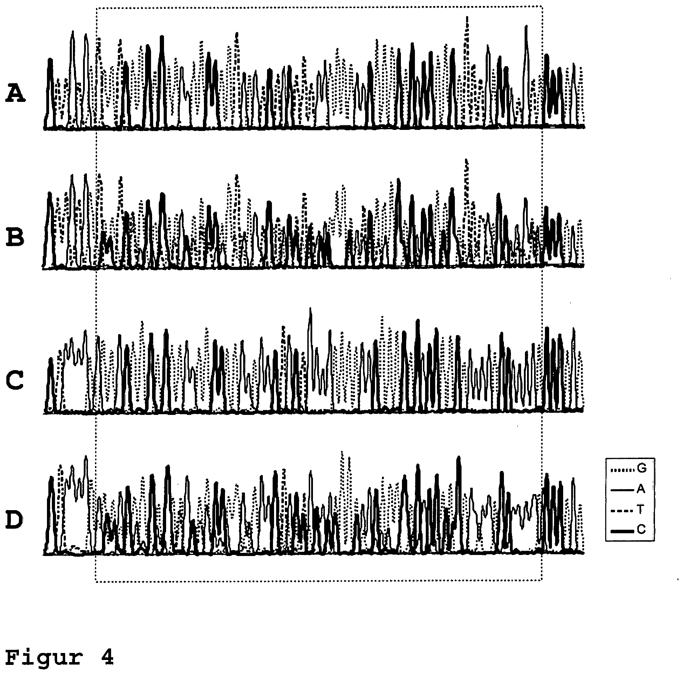

Figur 4 zeigt die Ergebnisse der Sequenzierung des *EGFR* Genlokus (Exon 19) mit einer Deletion. A: Referenzanalyse von nicht-umgewandelter genomischer DNA des gesunden, an den Tumor angrenzenden Gewebes. B: Referenzanalyse von nicht-umgewandelter genomischer DNA des Tumorgewebes. C: erfindungsgemäße Analyse von umgewandelter genomischer DNA des gesunden Gewebes. D: erfindungsgemäße Analyse von umgewandelter genomischer DNA des Tumorgewebes.

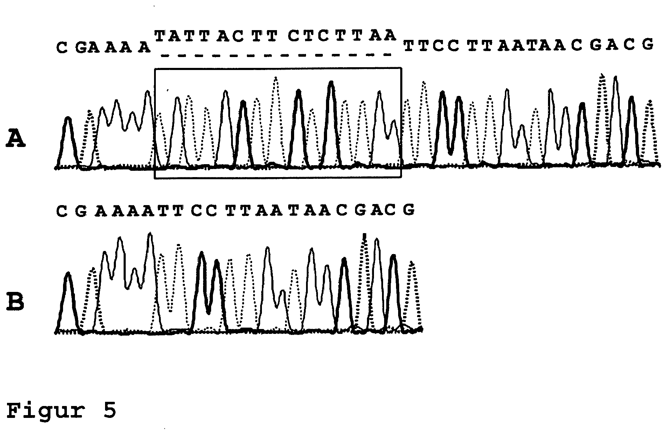

Figur 5 zeigt das Ergebnis einer Klonsequenzierung von beiden Allelen der PCR-Produkte aus Figur 4D. A: Wildtyp-Allel der konvertierten genomischen DNA des mutationstragenden Tumors. B: Mutiertes Allel der konvertierten genomischen DNA des mutationstragenden Tumors mit einer Deletion von 15 Basen.

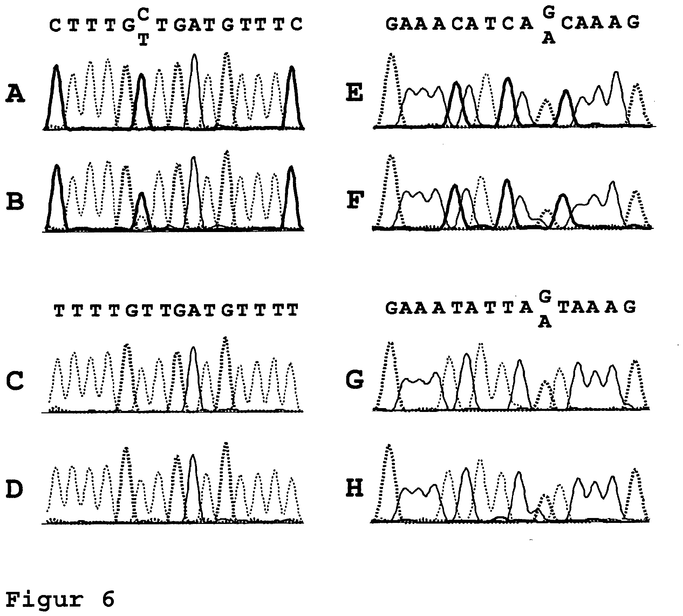

Figur 6 zeigt die Ergebnisse der Sequenzierung des *KRAS* Lokus (Exon 4). A: Referenzanalyse von nicht-umgewandelter genomischer DNA des gesunden, an den Tumor angrenzenden, Gewebes. Sequenzierung des Vorwärtsstrangs. B: Referenzanalyse von nicht-umgewandelter genomischer DNA des Tumorgewebes. Sequenzierung des Vorwärtsstrangs. C: erfindungsgemäße Analyse von umgewandelter genomischer DNA des gesunden Gewebes. Vorwärtssequenzierung des Bisulfit-I Strangs. D: erfindungsgemäße Analyse von umgewandelter genomischer DNA des Tumorgewebes. Vorwärtssequenzierung des Bisulfit-I Strangs. E: Wie A, jedoch Sequenzierung des Rückwärtsstrangs. F: Wie B, jedoch Sequenzierung des Rückwärtsstrangs. G: erfindungsgemäße Analyse von umgewandelter genomischer DNA des gesunden Gewebes. Rückwärtssequenzierung des Bisulfit-II Strangs. H: erfindungsgemäße Analyse von umgewandelter genomischer DNA des Tumorgewebes. Rückwärtssequenzierung des Bisulfit-II Strangs.

### Kurze Beschreibung der Sequenzen

SEQ ID NO:1 Vorwärtsprimer verwendet für die Amplifikation des nicht-umgewandelten (SEQ ID NO:3) und des umgewandelten (SEQ ID NO:4) *BRAF* V600E Lokus.
SEQ ID NO:2 Rückwärtsprimer verwendet für die Amplifikation des nicht-umgewandelten (SEQ ID NO:3) und des umgewandelten (SEQ ID NO:4) *BRAF* V600E Lokus.
SEQ ID NO:3 *BRAF* Genlokus, der die Position der V600E Mutation umfasst.
SEQ ID NO:4 umgewandelter *BRAF* Genlokus (Bisulfit-II Strang), der die Position der V600E Mutation umfasst.
SEQ ID NO:5 qPCR Detektions-Sonde, die auf den Bisulfitkonvertierten *BRAF* Genlokus (SEQ ID NO:4) zielt, der die V600E Mutation umfasst.
SEQ ID NO:6 *SHOX2* Genlokus, welcher im Rahmen von Methylierungsanalysen untersucht wurde.
SEQ ID NO:7 Vorwärtsprimer verwendet für die Amplifikation des umgewandelten *SHOX2* Genlokus (SEQ ID NO:10).
SEQ ID NO:8 Rückwärtsprimer verwendet für die Amplifikation des umgewandelten *SHOX2* Genlokus (SEQ ID NO:10).
SEQ ID NO:9 Blocker-Oligonukleotid verwendet, um den Vorwärtsprimer (SEQ ID NO:7) an der Bindung an den umgewandelten *SHOX2* Genlokus (SEQ ID NO:10) zu hindern, wenn dieser unmethyliert ist.
SEQ ID NO:10 umgewandelter (Bisulfit-I Strang) *SHOX2* Genlokus abgeleitet von der genomischen Sequenz SEQ ID NO:6.
SEQ ID NO:11 qPCR Detektions-Sonde, die auf den umgewandelten methylierten *SHOX2* Genlokus (SEQ ID NO:10) zielt.
SEQ ID NO:12 Sequenzierprimer verwendet für die Sanger-Sequenzierung des umgewandelten *BRAF* Genlokus (SEQ ID NO:4) .
SEQ ID NO:13 Vorwärtsprimer verwendet, um den nicht-umgewandelten *EGFR* Exon 21 Genlokus (SEQ ID NO:15) zu amplifizieren, welcher die L858R Mutation umfasst.
SEQ ID NO:14 Rückwärtsprimer verwendet, um den nicht-umgewandelten *EGFR* Exon 21 Genlokus (SEQ ID NO:15) zu amplifizieren, welcher die L858R Mutation umfasst.
SEQ ID NO:15 unkonvertierter *EGFR* Exon 21 Genlokus, welcher die L858R Mutation umfasst.
SEQ ID NO:16 Vorwärtsprimer verwendet, um den umgewandelten *EGFR* Exon 21 Genlokus (SEQ ID NO:18) zu amplifizieren, welcher die L858R Mutation umfasst.
SEQ ID NO:17 Rückwärtsprimer verwendet, um den umgewandelten *EGFR* Exon 21 Genlokus (SEQ ID NO:18) zu amplifizieren, welcher die L858R Mutation umfasst.
SEQ ID NO:18 umgewandelter *EGFR* Exon 21 Genlokus (Bisulfit-I Strang) abgeleitet von SEQ ID NO:15, der die L858R Mutation umfasst.
SEQ ID NO:19 Vorwärtsprimer verwendet, um den nicht-umgewandelten *EGFR* Exon 19 Genlokus (SEQ ID NO:21) zu amplifizieren.
SEQ ID NO:20 Rückwärtsprimer verwendet, um den nicht-umgewandelten *EGFR* Exon 19 Genlokus (SEQ ID NO:21) zu amplifizieren.
SEQ ID NO:21 nicht-umgewandelter *EGFR* Exon 19 Genlokus.
SEQ ID NO:22 Vorwärtsprimer verwendet, um den umgewandelten *EGFR* Exon 19 Genlokus (SEQ ID NO:24) zu amplifizieren.
SEQ ID NO:23 Rückwärtsprimer verwendet, um den umgewandelten *EGFR* Exon 19 Genlokus (SEQ ID NO:24) zu amplifizieren.
SEQ ID NO:24 umgewandelter *EGFR* Exon 19 Genlokus (Bisulfit-I Strang) abgeleitet von SEQ ID NO:21.
SEQ ID NO:25 Vorwärtsprimer verwendet, um den nicht-umgewandelten *KRAS* Exon 4 Genlokus (SEQ ID NO:27) zu amplifizieren.
SEQ ID NO:26 Rückwärtsprimer verwendet, um den nicht-umgewandelten *KRAS* Exon 4 Genlokus (SEQ ID NO:27) zu amplifizieren.
SEQ ID NO:27 nicht-umgewandelter *KRAS* Exon 4 Genlokus.
SEQ ID NO:28 umgewandelter *KRAS* Exon 4 Genlokus (Bisulfit-I Strang) abgeleitet von SEQ ID NO:27.
SEQ ID NO:29 Vorwärtsprimer verwendet, um den umgewandelten *KRAS* Exon 4 Genlokus (SEQ ID NO:28) zu amplifizieren.
SEQ ID NO:30 Rückwärtsprimer verwendet, um den umgewandelten *KRAS* Exon 4 Genlokus (SEQ ID NO:28) zu amplifizieren.
SEQ ID NO:31 umgewandelter *KRAS* Exon 4 Genlokus (Bisulfit-II Strang) abgeleitet von SEQ ID NO:27.
SEQ ID NO:32 Vorwärtsprimer verwendet, um den umgewandelten *KRAS* Exon 4 Genlokus (SEQ ID NO:31) zu amplifizieren.
SEQ ID NO:33 Rückwärtsprimer verwendet, um den umgewandelten *KRAS* Exon 4 Genlokus (SEQ ID NO:31) zu amplifizieren.
SEQ ID NO:34 *BRCA2,* "Region of Interest" (ROI) 1.
SEQ ID NO:35 *BRCA2,* ROI 2.
SEQ ID NO:36 *BRCA2,* ROI 3.
SEQ ID NO:37 *BRCA2,* ROI 4.
SEQ ID NO:38 *BRCA2,* ROI 5
SEQ ID NO:39 *BRCA2,* ROI 6, präferiert für Methylierungsanalysen.
SEQ ID NO:40 *BRCA2,* ROI 7.
SEQ ID NO:41 *BRCA2,* ROI 8.
SEQ ID NO:42 *BRCA2,* ROI 9.
SEQ ID NO:43 *BRCA2,* ROI 10.
SEQ ID NO:44 *BRCA2,* ROI 11.
SEQ ID NO:45 *BRCA2,* ROI 12.
SEQ ID NO:46 *BRCA1,* "Region of Interest" (ROI) 1.
SEQ ID NO:47 *BRCA1,* ROI 2.
SEQ ID NO:48 *BRCA1,* ROI 3.
SEQ ID NO:49 *BRCA1,* ROI 4.
SEQ ID NO:50 *BRCA1,* ROI 5.
SEQ ID NO:51 *BRCA1,* ROI 6.
SEQ ID NO:52 *BRCA1,* ROI 7.
SEQ ID NO:53 *BRCA1,* ROI 8.
SEQ ID NO:54 *BRCA1,* ROI 9.
SEQ ID NO:55 *BRCA1,* ROI 10.
SEQ ID NO:56 *BRCA1,* ROI 11, präferiert für Mutationsanalysen.
SEQ ID NO:57 *BRCA1,* ROI 12.
SEQ ID NO:58 *BRCA1,* ROI 13.
SEQ ID NO:59 *BRCA1,* ROI 14.
SEQ ID NO:60 *BRCA1,* ROI 15.
SEQ ID NO:61 *BRCA1,* ROI 16, präferiert für Methylierungsanalysen.
SEQ ID NO:62 *BRCA1,* ROI 17.
SEQ ID NO:63 *BRCA1,* ROI 18.
SEQ ID NO:64 *EGFR,* "Region of Interest" (ROI) 1, Exon 19.
SEQ ID NO:65 *EGFR,* ROI 2, Exon 21.
SEQ ID NO:66 *EGFR,* ROI 3, Exon 20.
SEQ ID NO:67 *EGFR,* ROI 4, Exon 18.
SEQ ID NO:68 *KRAS,* "Region of Interest" (ROI) 1, Exon 2.
SEQ ID NO:69 *KRAS,* ROI 2, Exon 3.
SEQ ID NO:70 *KRAS,* ROI 3, Exon 4.
SEQ ID NO:71 *BRAF,* "Region of Interest" (ROI) 1, Exon 15.
SEQ ID NO:72 *BRAF,* ROI 2, Exon 11.
SEQ ID NO:73 *AKT1,* "Region of Interest" (ROI) 1.
SEQ ID NO:74 *DDR2,* "Region of Interest" (ROI) 1, Exon 19.
SEQ ID NO:75 *DDR2,* ROI 2, Exon 18.
SEQ ID NO:76 *DDR2,* ROI 3, Exone 16 und 17.
SEQ ID NO:77 *ERBB2 (HER2),* "Region of Interest" (ROI) 1.
SEQ ID NO:78 *MAP2K1* (*MEK1*), "Region of Interest" (ROI) 1.
SEQ ID NO:79 *NRAS,* "Region of Interest" (ROI) 1, Codon 61.
SEQ ID NO:80 *NRAS,* ROI 2, Codon 12.
SEQ ID NO:81 *PIK3CA,* "Region of Interest" (ROI) 1, Exon 9.
SEQ ID NO:82 *PIK3CA,* ROI 2, Exon 20.
SEQ ID NO:83 *PTEN,* "Region of Interest" (ROI) 1, Exon 7.
SEQ ID NO:84 *IDH1,* "Region of Interest" (ROI) 1.
SEQ ID NO:85 *IDH2,* "Region of Interest" (ROI) 1.
SEQ ID NO:86 Vorwärtsprimer (F1) geeignet für *BRCA1* Methylierungsanalysen.
SEQ ID NO:87 Rückwärtsprimer (R1) geeignet für *BRCA1* Methylierungsanalysen.
SEQ ID NO:88 Vorwärtsprimer (F2) geeignet für *BRCA1* Methylierungsanalysen.
SEQ ID NO:89 Rückwärtsprimer (R2) geeignet für *BRCA1* Methylierungsanalysen.
SEQ ID NO:90 *PITX2,* "Region of Interest" (ROI) 1, Promotor A, präferiert für Methylierungsanalysen.
SEQ ID NO:91 *PITX2,* ROI 2, Promotor C, präferiert für Methylierungsanalysen.
SEQ ID NO:92 *MGMT,* "Region of Interest" (ROI) 1, präferiert für Methylierungsanalysen.
SEQ ID NO:93 *SEPT9,* "Region of Interest" (ROI) 1, präferiert für Methylierungsanalysen.
SEQ ID NO:94 *TP53* "Region of Interest" (ROI) 1.

### Beschreibung der Erfindung

Der erste Aspekt der vorliegenden Erfindung betrifft ein Verfahren zur Diagnose, Prognose, Prädiktion und/oder Verlaufskontrolle einer malignen Erkrankung, umfassend eine Bestimmung mindestens einer Mutation in genomischer DNA. Das Verfahren umfasst die folgenden Schritte: A) Umwandeln zumindest eines Teils der in der genomischen DNA enthaltenen Cytosine in Uracil oder eine andere Base mit einem von Cytosin unterscheidbaren Basenpaarungsverhalten und/oder Molekulargewicht, B) Durchführen einer Mutationsanalyse mit der aus Schritt A) erhaltenen genomischen DNA, um die mindestens eine Mutation zu bestimmen. Die aus Schritt A) erhaltene genomische DNA wird nachfolgend auch als "umgewandelte DNA" oder "konvertierte DNA" bezeichnet. Demzufolge bezeichnet "nicht-umgewandelte DNA" solche genomische DNA, welche Schritt A) nicht durchlaufen hat.

Die vorliegende Erfindung beruht unter anderem auf der Erkenntnis des Erfinders, dass viele klinisch relevante Proben, wie zum Beispiel Biopsien, Feinnadelaspirate, Lasermikrodissezierte Zellen, Blutplasma oder freizirkulierende Tumorzellen nur sehr geringe Mengen an genomischer DNA enthalten, gleichzeitig aber in der klinischen Routine eine immer größere Menge an genetischen Parametern für eine personalisierte und optimierte Behandlung des Patienten benötigt werden. Im Blutplasma sind beispielsweise oftmals nur wenige hundert bis einige tausend DNA Kopien freizirkulierender DNA pro Milliliter Plasma enthalten. Zirkulierende Tumorzellen treten sogar nur in einer Größenordnung von wenigen Einzelzellen bis zu einigen Dutzend Tumorzellen pro 10 Milliliter Vollblut auf. Bei Lasermikrodissezierten Zellen ist die Menge der DNA von der Anzahl der mikrodissezierten Zellen abhängig und kann von einer Einzelzelle mit zwei DNA Kopien bis über 1000 Zellen reichen. Oftmals wird die Laser-Mikrodissektion mit Formalin-fixierten und Paraffin-eingebetteten Proben durchgeführt, bei denen der überwiegende Teil der DNA degradiert ist und daher nicht für molekulardiagnostische Verfahren zur Verfügung steht. Problematisch ist dabei, dass die genomische DNA nach Durchlaufen eines herkömmlichen molekulardiagnostischen Verfahrens in der Regel nicht unverändert beziehungsweise gar nicht zurückgewonnen werden kann. Somit sind weitere molekulardiagnostischen Verfahren oder Wiederholungen von molekulardiagnostischen Verfahren nicht möglich.

Ein Beispiel für ein weiteres molekulardiagnostisches Verfahren ist eine Methylierungsanalyse, bei der die genomische DNA zunächst umgewandelt werden muss, beispielsweise durch Bisulfit-Behandlung. Dabei wird unmethyliertes Cytosin zum Beispiel in Uracil umgewandelt, während methyliertes Cytosin (Methylcytosin, meC) unverändert bleibt. Uracil besitzt die gleichen Basenpaarungseigenschaften wie Thymin. Daher sind beide im Rahmen einer anschließenden Vervielfältigung der umgewandelten genomischen DNA nicht mehr voneinander zu unterschieden. Es findet also eine C nach U Konversion statt, die sich aufgrund des gleichen Basenpaarungsverhaltens von T und U wie eine C nach T Konversion verhält. Folglich setzt die Umwandlung des Cytosins die Sequenzkomplexität der DNA stark herab und führt damit in der Regel zu einem erheblichen Verlust an genetischer Information. Aus diesem Grund sah sich ein Molekularmediziner bislang gerade bei kleinen und Kleinstproben gezwungen zu entscheiden, welche Analyse durchgeführt wird, beispielsweise eine Mutationsanalyse oder eine Methylierungsanalyse.

Die beschriebene Mutationsanalyse in umgewandelter DNA löst dieses Problem. Obwohl die Umwandlung von genomischer DNA zum Zweck einer Methylierungsanalyse seit etwa einem Vierteljahrhundert bekannt ist (Frommer, M. et al., Proc. Natl. Acad. Sci. USA, 1992, 89, 1827-1831), konnte in dieser langen Zeit durch die Fachwelt nicht gezeigt werden, dass eine Analyse von Mutationen auch in umgewandelter DNA möglich ist. Vielmehr entsprach es der herrschenden Lehrmeinung, dass der Verlust von genetischer Information durch die Umwandlung der genomischen DNA die Bestimmung einer Mutation erschwert bis unmöglich macht, insbesondere wenn Cytosine von Mutationen betroffen sind.

Diese in den Fachkreisen weit verbreitete Ansicht wurde im Rahmen der vorliegenden Erfindung überwunden. Es konnte gezeigt werden, dass die Analyse von Mutationen in umgewandelter DNA möglich ist. Diese erfinderische Erkenntnis resultiert in verschiedenen unerwarteten Vorteilen. Beispielsweise erlaubt die erfindungsgemäße Mutationsanalyse in umgewandelter DNA erstmals eine direkte Kombination der Mutationsanalyse mit weiteren Analysen wie zum Beispiel der Methylierungsanalyse innerhalb derselben Probe. Auf diese Weise können verschiedene klinisch relevante genetische und epigenetische Parameter in hohem Maß multiplexiert und parallel analysiert werden. Dies führt nicht nur zu einer kosteneffizienteren molekularen Diagnose von Erkrankungen, sondern ermöglicht auch eine differenziertere klinische Entscheidungsfindung bei gleichzeitig kürzeren Analysezeiten. Überraschenderweise konnte auch festgestellt werden, dass sich bestimmte Mutationen sogar besser analysieren lassen als bei einer herkömmlichen Mutationsanalyse mit nicht-umgewandelter genomischer DNA. Diesbezüglich wird auch auf die unten folgenden Ausführungsbeispiele verwiesen.

Die genomische DNA kann aus verschiedenen Quellen stammen, beispielsweise aus Zellen aus operativ oder bioptisch entnommenem Gewebe. Die Zellen können auch von Abstrichen und aus Aspiraten wie zum Beispiel Spülflüssigkeiten, Feinnadelaspiraten oder Sputum stammen. Die genomische DNA kann auch aus Blut, Blutserum und Blutplasma stammen, beispielsweise in Form von frei zirkulierender DNA, exosomaler DNA, oder in Form von frei zirkulierenden Zellen, aus denen die genomische DNA gewonnen wird. Die genomische DNA kann auch aus weiteren Körperflüssigkeiten wie zum Beispiel Urin, Pleuraergüssen oder Aszites stammen, beispielsweise in Form von freier DNA oder in Form von Zellen, aus denen die genomische DNA gewonnen wird. Die DNA kann aus nicht-konservierten (frischen) Zellen, Geweben und Körperflüssigkeiten, sowie aus fixierten Zellen, Geweben und Körperflüssigkeiten gewonnen werden. Die Fixierung der Zellen, Gewebe und Körperflüssigkeiten kann durch präzipitierende Fixative wie zum Beispiel Ethanol und andere Alkohole oder durch quervernetzende Fixative wie zum Beispiel Formaldehyd erreicht werden. Die genomische DNA kann auch aus beliebigen Kombinationen dieser Quellen stammen. Es kann sich auch um extrahierte DNA aus den vorgenannten Quellen handeln. Es ist auch möglich, die genomische DNA anzureichern, zum Beispiel durch Fällung oder Extraktion. Dies kann sich zum Beispiel bei frei zirkulierender genomischer DNA aus den genannten Körperflüssigkeiten anbieten. Es ist auch möglich, die Zellen anzureichern, zum Beispiel durch Größenfiltration oder über oberflächengebundene Antikörper (zum Beispiel an Magnetpartikeln, Membranen oder Polymeren), deren Antigene sich auf der Oberfläche der anzureichernden Zellen befinden, wie beispielsweise ein anti-EpCAM Antikörper. Dies kann sich zum Beispiel bei frei zirkulierenden Zellen aus den genannten Körperflüssigkeiten anbieten. Eine geeignete Vorrichtung zur Anreicherung frei zirkulierender Zellen aus der Blutbahn eines Patienten ist zum Beispiel in WO 2010/145824 A1 beschrieben, auf welche zur Ergänzung des technischen Hintergrundes dieser Erfindung vollinhaltlich Bezug genommen wird. Entsprechende kommerziell erhältliche Vorrichtungen sind beispielsweise der CellCollector Detektor CANCER01 (DC01) und Detektor CANCER02 (DC02) (beide GILUPI GmbH, Potsdam, Deutschland). Weitere geeignete Quellen genomischer DNA sind Lysate oder Homogenisate von Frischgeweben und Lysate von fixierten Geweben.

Die Umwandlung der genomischen DNA in Schritt A) kann im Prinzip mit allen im Stand der Technik für diesen Zweck bekannten und geeigneten Methoden erfolgen. Typischerweise handelt es sich um eine chemische oder enzymatische Umwandlung, beispielsweise durch Kontaktieren der genomischen DNA mit Bisulfit, zum Beispiel Natriumbisulfit oder Ammoniumbisulfit, oder mit Cytidin-Desaminasen.

Erforderlichenfalls kann nach der Umwandlung in Schritt A) und vor der Mutationsanalyse in Schritt B) ein Aufreinigen der aus Schritt A) erhaltenen genomischen DNA erfolgen. Geeignete Aufreinigungsmethoden und Protokolle sind dem Fachmann bekannt und können zum Beispiel eine DNA-Extraktion, eine Fällung oder eine polymergestützte Anreicherung (polymer-mediated enrichment) umfassen.

Die Art der Mutationsanalyse im Schritt B) unterliegt keinen besonderen Beschränkungen. Geeignete Methoden kann ein Fachmann unschwer auf der Grundlage dieser Offenbarung bestimmen. Diesbezüglich wird auch auf die oben genannten Laborhandbücher verwiesen. In einer bevorzugten Variante wird zunächst eine Polymerasekettenreaktion (PCR) mit Oligonukleotiden, sogenannten Primern, durchgeführt, welche dazu ausgelegt ist, einen Abschnitt der umgewandelten genomischen DNA zu amplifizieren, welcher im Verdacht steht, die Mutation zu enthalten. Anschließend erfolgt vorzugsweise eine Sequenzierung zumindest eines Teils des Amplifikats, zum Beispiel eine Sanger-Sequenzierung, Pyrosequenzierung, massenspektrometrische Sequenzierung oder eine Sequenzierung der zweiten oder dritten Generation, welche auch als "Massive Parallel Sequencing", "Next Generation Sequencing" (NGS) oder als Nanoporensequenzierung bezeichnet werden. Es ist auch möglich, im Anschluss an die PCR eine Hybridisierung mit mutationsspezifischen Oligonukleotiden (Sonden) durchzuführen, beispielsweise in Form eines DNA-Microarrays. Die Mutation kann auch durch quantitative Echtzeit-PCR (quantitative real-time PCR, qPCR), gegebenenfalls gefolgt von einer Schmelzkurvenanalyse, bestimmt werden. Die Mutation kann auch durch modifizierte, PCR-basierte Verfahren, wie beispielsweise ARMES (Amplification Refractory Mutation System) bestimmt werden.

In wiederum anderen bevorzugten Varianten kann eine PCR entfallen, beispielsweise bei einem "Whole Genome Shotgun Bisulfite Sequencing" (WGSBS) oder einer direkten Nanoporensequenzierung. Bei der WGSBS wird die DNA fragmentiert, anschließend werden Adapter an die DNA Fragmente ligiert. Über die Adapter ist im Anschluss eine Amplifikation und Sequenzierung möglich. Es ist auch möglich bei der WGSBS den Schritt der Fragmentierung wegzulassen, da die DNA zum Beispiel durch die Umwandlung durch Bisulfit-Behandlung bereits fragmentiert vorliegen kann. Protokolle für die Durchführung einer WGSBS sind dem Fachmann leicht zugänglich (Johnson, M. D. et al., Curr. Protoc. Mol. Biol., 2012, 99, 21.23.1-21.23.28; Lister, R. et al., Nature, 2009, 462, 315-322; Berman, B. P. et al., Nat. Genet., 2011, 44, 40-46).

Bei der Nanoporensequenzierung wird ein DNA Molekül durch eine Pore geschleust. Die Nukleotide lösen bei der Passage ein messbares elektrisches Signal aus, welches charakteristisch für die in der Nanopore befindlichen Nukleotide ist und diesen so zugeordnet werden kann.

In einer anderen bevorzugten Variante kann vor der PCR Amplifikation eine Hybridisierung mit spezifischen Oligonukleotiden (Sonden) erfolgen, die im Falle des Bindens ligiert werden und anschließend mittels PCR amplifiziert werden. Geeignete Methoden und Protokolle, wie zum Beispiel eine "multiplex ligation dependent probe amplification" (MLPA) stehen dem Fachmann ohne Probleme zur Verfügung, zum Beispiel "PCR Mutation Detection Protocols" von B. D. M. Theophilus und R. Rapley, 2nd Edition, 2011, Springer.

In einer anderen bevorzugten Variante wird die Mutationsanalyse mittels quantitativer Echtzeit-PCR durchgeführt.

Die Mutation kann anschließend durch Vergleich der ermittelten Eigenschaft der behandelten genomischen DNA, also beispielsweise der Nukleotidsequenz, des Molekulargewichts oder der Hybridisierungsstärke, mit einem Normwert festgestellt werden. Als Normwert ist zum Beispiel die entsprechende Eigenschaft der wildtypischen DNA geeignet. Gegebenenfalls kann dabei auch eine entsprechenden Umwandlung der wildtypischen DNA wie in Schritt A) berücksichtigt werden, beispielsweise durch entsprechende bioinformatische Methoden oder indem die wildtypische DNA das erfindungsgemäße Verfahren ebenfalls durchläuft, zum Beispiel als Referenzprobe.

Die Mutation kann grundsätzlich eine Keimbahnmutation oder eine somatische Mutation oder auch Kombinationen davon umfassen. In einer bevorzugten Variante des Verfahrens umfasst die Mutation eine somatische Mutation. Während Keimbahnmutationen im Wesentlichen in allen Zellen eines Organismus nachgewiesen werden können, sind somatische Mutationen in der Regel nur in Zellen eines bestimmten Gewebes nachweisbar, zum Beispiel neoplastischen Zellen eines Tumors. Es ist ein überraschender Vorteil der vorliegenden Erfindung, dass auch solche Mutationen mit geringer Abundanz im Organismus mit Hilfe des erfindungsgemäßen Verfahrens mit hoher Spezifizität und Sensitivität nachgewiesen werden können.

In einer bevorzugten Variante umfasst die Mutationsanalyse zumindest einen Teil oder mehrere Teile eines in der genomischen DNA enthaltenen Gens. Alternativ oder zusätzlich kann die Mutationsanalyse auch zumindest einen Teil oder mehrere Teile von jeweils zwei oder mehreren in der genomischen DNA enthaltenen Genen umfassen.

In bevorzugten Varianten umfasst die Mutationsanalyse zumindest einen Teil oder mehrere Teile eines Gens, welches aus der Gruppe *BRAF, EGFR, KRAS, NRAS, BRCA1, BRCA2, AKT1, VGFR, IDH1, IDH2, CRLF2, TSC1, PDGFRA, NF1, GNAQ, GNA11, CTNNB1, ASXL1, BCOR, DNMT3A, ETV6, EZH2, SF3B1, SRSF2, STAG2, TET2, TP53, U2AF1, ZRSR2, HRAS, TERT (hTERT), SMO, FLT3, JAK2, ESR1, BCR, SMAD4, DNMT3A, AR, ERBB2 (HER2), MAP2K1 (MEK1), PIK3CA, PTEN, PALB2, DDR2* und beliebigen Kombinationen davon ausgewählt ist.

In weiteren bevorzugten Varianten ist die Mutationsanalyse dazu ausgelegt, eine Fusion, Translokation und/oder Inversion von zumindest einem Teil oder mehreren Teilen eines Gens zu bestimmen, welches aus der Gruppe *NTRK1 (TRKA), RET, DEK-NUP214, MLL-MLLT3, CBFB-MYH11, RPN1-EVI1, RUNX1-RUNX1T1, PML-RARA, RBM15-MKL, KIT, ALK, ROS1* und beliebigen Kombinationen davon ausgewählt ist.

In weiteren bevorzugten Varianten ist die Mutationsanalyse dazu ausgelegt, eine Amplifikation von zumindest einem Teil oder mehreren Teilen eines Gens zu bestimmen, welches aus der Gruppe der Gene *FGFR1, MET, ERBB2 (HER2), FGFR1, FGFR2, BCR-ABL1, RET, MEK, mTOR und VEGFR* und beliebigen Kombinationen davon ausgewählt ist.

In einer bevorzugten Variante umfasst die Mutationsanalyse zumindest einen Teil oder mehrere Teile des *BRAF* Gens. Insbesondere kann die Mutationsanalyse dazu ausgelegt sein, eine Mutation in zumindest einem Teil einer Sequenz zu bestimmen, welche im wildtypischen Zustand mindestens 95% Sequenzidentität mit SEQ ID NO:71 und/oder SEQ ID NO:72 aufweist. In einer besonders bevorzugten Variante umfasst die Mutationsanalyse die *BRAF* Mutation c.1799T>A (V600E). Beispielsweise sprechen Tumoren, welche die Punktmutation V600E innerhalb des *BRAF* Gens besitzen, besonders gut auf eine Behandlung mit Vemurafenib an. Die Mutationsanalyse kann auch beliebige Kombinationen dieser Varianten umfassen.

In einer weiteren bevorzugten Variante umfasst die Mutationsanalyse zumindest einen Teil oder mehrere Teile des *EGFR* Gens. Insbesondere kann die Mutationsanalyse dazu ausgelegt sein, eine Mutation in zumindest einem Teil einer Sequenz zu bestimmen, welche im wildtypischen Zustand mindestens 95% Sequenzidentität mit SEQ ID NO:64, SEQ ID NO:65, SEQ ID NO:66 oder SEQ ID NO:67 aufweist, sowie Kombinationen davon.

In einer weiteren bevorzugten Variante umfasst die Mutationsanalyse zumindest einen Teil oder mehrere Teile des *KRAS* Gens. Insbesondere kann die Mutationsanalyse dazu ausgelegt sein, eine Mutation in zumindest einem Teil einer Sequenz zu bestimmen, welche im wildtypischen Zustand mindestens 95% Sequenzidentität mit SEQ ID NO:68, SEQ ID NO:69 oder SEQ ID NO:70 aufweist, sowie Kombinationen davon.

In einer weiteren bevorzugten Variante umfasst die Mutationsanalyse zumindest einen Teil oder mehrere Teile des *NRAS* Gens. Insbesondere kann die Mutationsanalyse dazu ausgelegt sein, eine Mutation in zumindest einem Teil einer Sequenz zu bestimmen, welche im wildtypischen Zustand mindestens 95% Sequenzidentität mit SEQ ID NO:79 oder SEQ ID NO:80 aufweist, sowie Kombinationen davon.

Die Mutationsanalyse von *ERBB2 (HER2)* ist vorzugsweise dazu ausgelegt, eine Mutation in zumindest einem Teil einer Sequenz zu bestimmen, welche im wildtypischen Zustand mindestens 95% Sequenzidentität mit SEQ ID NO:77 aufweist. Die Mutationsanalyse von *MAP2K1 (MEK1)* ist vorzugsweise dazu ausgelegt, eine Mutation in zumindest einem Teil einer Sequenz zu bestimmen, welche im wildtypischen Zustand mindestens 95% Sequenzidentität mit SEQ ID NO:78 aufweist. Die Mutationsanalyse von *PIK3CA* ist vorzugsweise dazu ausgelegt, eine Mutation in zumindest einem Teil einer Sequenz zu bestimmen, welche im wildtypischen Zustand mindestens 95% Sequenzidentität mit SEQ ID NO:81 oder SEQ ID NO:82 aufweist, oder Kombinationen davon. Die Mutationsanalyse von *PTEN* ist vorzugsweise dazu ausgelegt, eine Mutation in zumindest einem Teil einer Sequenz zu bestimmen, welche im wildtypischen Zustand mindestens 95% Sequenzidentität mit SEQ ID NO:83 aufweist. Die Mutationsanalyse von *DDR2* ist vorzugsweise dazu ausgelegt, eine Mutation in zumindest einem Teil einer Sequenz zu bestimmen, welche im wildtypischen Zustand mindestens 95% Sequenzidentität mit SEQ ID NO:74, SEQ ID NO:75 oder SEQ ID NO:76 aufweist, oder Kombinationen davon. Die Mutationsanalyse von *AKT1* ist vorzugsweise dazu ausgelegt, eine Mutation in zumindest einem Teil einer Sequenz zu bestimmen, welche im wildtypischen Zustand mindestens 95% Sequenzidentität mit SEQ ID NO:73 aufweist.

In einer weiteren Variante ist die Mutationsanalyse dazu ausgelegt, in die genomische DNA integrierte virale DNA zu bestimmen, insbesondere zumindest einen Teil oder mehrere Teile der DNA eines oder mehrerer Humaner Papilloma-Viren (HPV). Besonders bevorzugt ist die Mutationsanalyse dazu ausgelegt, zumindest einen Teil oder mehrere Teile der DNA eines oder mehrerer Humaner Papilloma-Viren der Subgruppen HPV 16, 18, 31, 33, 35, 39, 45, 51, 52, 56, 58, 59, 68, 73 und/oder 82 in der genomischen DNA zu bestimmen.

In einer weiteren Variante ist die Mutationsanalyse dazu ausgelegt, Amplifikationen und/oder Deletionen zu bestimmen, welche repetitive DNA-Sequenzen betreffen. Insbesondere ist es möglich, die Amplifikation und Deletion von kurzen Tandemwiederholungen (Short Tandem Repeats, STRs) zu bestimmen. Insbesondere ermöglicht dies die Bestimmung einer Mikrosatteliteninstabilität (MSI). Eine Mikrosatteliteninstabilität ist indikativ für ein fehlerhaftes DNA-Reparatursystem in der Zelle und daher prädiktiv für das Ansprechen auf DNA-schädigende Chemotherapien.

In einer bevorzugten Variante umfasst die genomische DNA frei zirkulierende DNA, DNA aus Exosomen und/oder DNA aus frei zirkulierenden Zellen aus einer Körperflüssigkeit, so genannte Flüssigbiopsien oder "liquid biopsies". Flüssigbiopsien stellen zurzeit ein zentrales Gebiet der onkologischen Forschung dar. Es wird dabei nicht das verdächtige Gewebe selbst, also zum Beispiel ein Tumorgewebe, sondern eine Probe einer Körperflüssigkeit analysiert, beispielsweise eine Blutprobe. In dieser Probe können verschiedene Substanzen untersucht werden, die aus dem Tumor stammen, da frei zirkulierende genomische DNA, exosomale DNA, beziehungsweise frei zirkulierende Zellen vom Tumor in die Blutbahn freigesetzt werden. Mit Vorteil wird das erfindungsgemäße Verfahren zur Analyse von Flüssigbiopsien verwendet, wenn der Tumor oder eine Metastase nicht biopsiert werden kann beziehungsweise wenn eine Biopsie für den Patienten im späten Tumorstadium eine zu große Gefahr darstellen würde.

Es ist möglich, mit dem erfindungsgemäßen Verfahren Mutationen in frei zirkulierender genomischer DNA beziehungsweise in frei zirkulierenden Zellen zu analysieren. Insbesondere können auch wenige mutationstragende DNA Moleküle vor einem Hintergrund von frei zirkulierender genomischer DNA gesunden Ursprungs nachgewiesen werden. Es ist beispielsweise möglich, dass die genomische DNA, insbesondere die umgewandelte genomische DNA, einen größeren Anteil DNA gesunden Ursprungs (wildtypische DNA) und einen kleineren Anteil genomischer DNA, welche die mindestens eine Mutation enthält, umfasst. Im Rahmen der vorliegenden Erfindung wurde jedoch erkannt, dass dies zu Problemen hinsichtlich der Sensitivität bei der Bestimmung einer Mutation führen kann, wenn ein herkömmliches Verfahren verwendet wird. Wurde beispielsweise bei einem Patienten mit einer malignen Erkrankung eine konventionelle Mutationsanalyse mit einer Probe frei zirkulierender DNA durchgeführt und ist der Nachweis der Mutation in der frei zirkulierenden DNA negativ, dann kann das zwei Ursachen haben. Entweder trägt die maligne Erkrankung diese Mutation tatsächlich nicht oder es befindet sich zu wenig oder gar keine DNA der malignen Erkrankung in der Blutbahn des Patienten beziehungsweise in der Probe. Im letzteren Fall ist der Nachweis folglich falsch-negativ.

Eine weitere Aufgabe der Erfindung bestand daher in der Verbesserung der Sensitivität der Mutationsanalyse, indem die Anzahl falsch-negativer Ergebnisse reduziert wird. Diese Aufgabe wird erfindungsgemäß gelöst, indem das Verfahrens zusätzlich umfasst C) Durchführen einer Methylierungsanalyse mit der aus Schritt A) erhaltenen genomischen DNA, um einen Methylierungszustand mindestens eines in der genomischen DNA enthaltenen CpG-Dinukleotids zu bestimmen.

DNA Methylierung ist ein wichtiger Prozess der Entstehung und -progression maligner Erkrankungen, zum Beispiel von Tumoren. Diese Methylierung findet vorwiegend an den Cytosinen im CpG-Dinukleotid Sequenzkontext statt. Viele Gene sind in malignen Erkrankungen, zum Beispiel Tumoren, hypermethyliert, das heisst, dass diese Gene CpG-Dinukleotide enthalten, die im Vergleich zu dem entsprechenden Normalgewebe, aus dem der Tumor entstanden ist, häufiger methyliert sind. Der Methylierungszustand eines CpG-Dinukleotids ist daher gut geeignet, um DNA einer malignen Erkrankung, welche in genomischer DNA enthalten ist, zu bestimmen. Dadurch kann anhand des Methylierungszustands von CpG-Dinukleotiden die DNA der malignen Erkrankung von genomischer DNA gesunden Ursprungs unterschieden werden.

Auf diese Weise kann mit dem erfindungsgemäßen Verfahren erstmals innerhalb einer einzigen Analyse ein funktioneller Zusammenhang zwischen einer Mutationsanalyse und einer Methylierungsanalyse hergestellt werden, beispielsweise in Form einer Normalisierung oder auch internen Standardisierung. Es ist zum Beispiel möglich, die An- oder Abwesenheit von DNA einer malignen Erkrankung in genomischer DNA anhand des Methylierungszustands des CpG-Dinukleotids zu bestimmen und mit der An- oder Abwesenheit der Mutation zu korrelieren. Kann beispielsweise durch den Nachweis eines aberrant methylierten CpG-Dinukleotids gezeigt werden, dass DNA einer malignen Erkrankung in der genomischen DNA enthalten ist und kann gleichzeitig gezeigt werden, dass keine Mutation vorliegt, dann ist bewiesen, dass die maligne Erkrankung die Mutation nicht trägt und eine passfähige Behandlung kann gestartet werden. Zeigt die Methylierungsanalyse andererseits, dass keine DNA einer malignen Erkrankung vorliegt, dann kann entsprechend auch die Mutation nicht nachgewiesen werden. Folglich ist nicht ausgeschlossen, dass der Tumor die Mutation doch trägt. Ergänzende Analysen sind notwendig, um die geeignetste Behandlung zu finden.

Durch dieses synergistische Zusammenwirken von Mutationsanalyse und Methylierungsanalyse wird eine signifikante Verbesserung gegenüber herkömmlichen Mutationsanalysen erreicht. Die erfindungsgemäße Durchführung der Mutationsanalyse und der Methylierungsanalyse in einer Reaktion führt beispielsweise zu einer signifikanten Verbesserung der Messgenauigkeit und damit zu einer signifikanten Verbesserung der analytischen Leistungsfähigkeit des Verfahrens zum Beispiel gegenüber herkömmlichen Mutationsanalysen. Die Messgenauigkeit einer molekulardiagnostischen Prüfmethode kann von vielen Parametern abhängen. Qualitätsmerkmale einer diagnostischen Prüfmethode sind zum Beispiel die Präzision unter Zwischenbedingungen und die Robustheit. Die Präzision unter Zwischenbedingungen umfasst die Variabilität, welche bei der Anwendung einer Prüfmethode zu unterschiedlichen Zeitpunkten, mit unterschiedlichen Prüfinstrumenten und unterschiedlichen Reagenzienchargen durch unterschiedliche Anwender, jedoch im gleichen Labor bei Anwendung der gleichen Probe und des gleichen Messverfahrens, auftritt. Die Robustheit eines Analyseverfahrens sagt aus, in welchem Ausmaß ein Analyseverfahren von kleinen, absichtlichen Veränderungen der Verfahrensparameter unbeeinflusst bleibt, und gibt an, wie verlässlich es unter normalen Einsatzbedingungen ist. Durch die erfindungsgemäße Durchführung der Mutationsanalyse und der Methylierungsanalyse in einer Reaktion wird die Präzision unter Zwischenbedingungen und die Robustheit signifikant verbessert, da Varianzen und Veränderungen innerhalb des Messverfahrens durch die interne Korrelation von beiden Analysen aufgefangen und normalisiert beziehungsweise standardisiert werden. Beispielsweise können falsch-negative Diagnosen wirksam vermieden werden, was letztendlich zu einer Erhöhung der Sensitivität und zu einem besseren und effizienteren Einsatz von Therapiemöglichkeiten zum Wohle des Patienten führt. Diese Vorteile betreffen natürlich nicht nur Flüssigbiopsien, sondern auch andere Quellen genomischer DNA. Beispielsweise wird die Gefahr einer Fehldiagnose aufgrund eines hohen Anteils gesunden Normalgewebes im analytischen Arbeitsablauf, aufgrund eines geringen Anteils von neoplastischen Zellen in der malignen Erkrankung oder aufgrund einer starken Streuung der Zellen im Normalgewebe drastisch reduziert.

In einer weiterhin bevorzugten Variante umfasst die Methylierungsanalyse die Bestimmung des Methylierungszustands von zwei oder mehreren CpG-Dinukleotiden innerhalb eines in der genomischen DNA enthaltenen Gens. Alternativ oder zusätzlich kann die Methylierungsanalyse auch mindestens je ein CpG-Dinukleotid in zwei oder mehreren in der genomischen DNA enthaltenden Gene umfassen. Auf diese Weise löst die Erfindung das Problem, dass CpG-Dinukleotide in der DNA einer malignen Erkrankung mitunter heterogen methyliert vorliegen können, sodass ein besonders robuster Nachweis von DNA der malignen Erkrankung erreicht wird. Falsch-negative und falsch-positive Diagnosen werden folglich vermieden und die Spezifität und Sensitivität des Diagnoseverfahrens werden weiter verbessert.

Die Methylierungsanalyse kann zumindest einen Teil eines Gens umfassen, welches aus der Gruppe *SHOX2, SEPT9, BRCA1, LIMK1, APC, VIM, RASSF2, RASSF1, GSTP1, FOXL2, CDKN2A (p16), RARB* und beliebigen Kombinationen davon ausgewählt ist. Der Methylierungszustand von CpG-Dinukleotiden innerhalb dieser Gene wurde als besonders zuverlässig erkannt, um die An- oder Abwesenheit von DNA einer malignen Erkrankung innerhalb genomischer DNA nachzuweisen. In einer bevorzugten Variante umfasst die Methylierungsanalyse zumindest einen Teil von *SEPT9.* Insbesondere kann die Methylierungsanalyse dazu ausgelegt sein, den Methylierungszustand von ein oder mehreren CpG-Dinukleotiden einer Sequenz zu bestimmen, welche im wildtypischen, nicht-umgewandelten Zustand mindestens 95% Sequenzidentität mit SEQ ID NO:93 aufweist. In einer weiteren bevorzugten Variante umfasst die Methylierungsanalyse zumindest einen Teil von *SHOX2.* Insbesondere kann die Methylierungsanalyse dazu ausgelegt sein, den Methylierungszustand von ein oder mehreren CpG-Dinukleotiden einer Sequenz zu bestimmen, welche im wildtypischen Zustand mindestens 95% Sequenzidentität mit SEQ ID NO:6 aufweist.

Die Methylierungsanalyse kann weiterhin mindestens einen Teil eines Genes umfassen, welches zu diesem Zweck in WO 2009/036922 A2 und/oder US 2012/0101023 A1 beschrieben ist. Die Mutationsanalyse kann auch mindestens einen Teil eines Gens umfassen, welches in US 2014/0303001 A1 offenbart ist. Besonders bevorzugt umfasst die Methylierungsanalyse mindestens einen Teil mindestens eines Gens aus der Gruppe *TWIST1, ONECUT2* und *OTX1* und/oder die Mutationsanalyse mindestens ein Teil mindestens eines Gens aus der Gruppe *FGFR3, TERT, KRAS, NRAS* und *PIK3CA.* In dieser Kombination ist das Verfahren beispielsweise zur Detektion des Vorliegens und/oder der Wiederkehr einer Blasenkrebserkrankung gemäß dem nachfolgenden zweiten Erfindungsaspekt vorgesehen. Vorteilhafterweise wird die Detektion von Blasenkrebs mit genomischer DNA durchgeführt, welche frei-zirkulierende DNA aus Urin und/oder genomische DNA aus Zellen des Urinsediments umfasst.

In einer weiteren Ausgestaltung umfasst die Methylierungsanalyse zumindest einen Teil des Gens *SEPT9* und die Mutationsanalyse zumindest einen Teil des Gens *TP53. SEPT9* ist in besonders vielen malignen Erkrankungen aberrant methyliert und dazu in freizirkulierender DNA im Plasma von gesunden Patienten regelmäßig unmethyliert. Daher eignet sich die Methylierungsanalyse dieses Gens besonders für den Nachweis einer malignen Erkrankung im Blut. *TP53* ist rekurrent in verschiedenen malignen Erkrankungen mutiert. Das durch *TP53* kodierte Protein ist mit 393 Aminosäuren vergleichsweise klein. Die 393 Aminosäuren werden durch 1179 Basen kodiert, innerhalb derer sich die bevorzugt analysierten Mutationen befinden. 86% der Mutationen befinden sich innerhalb der Aminosäuren 125 und 300. Die Mutationsanalyse von *TP53* ist daher insbesondere dazu ausgelegt, eine Mutation in zumindest einem Teil einer Sequenz zu bestimmen, welche im nicht-umgewandelten wildtypischen Zustand mindestens 95% Sequenzidentität mit SEQ ID NO:94 aufweist. Insbesondere ist in Bezug auf den nachfolgenden zweiten Erfindungsaspekt die Mutationsanalyse von *TP53* und die Methylierungsanalyse von *SEPT9* für die Diagnose von Darmkrebs, Kopf- und Halstumoren, Lungenkrebs, Melanomen sowie Eierstock- und Speiseröhrenkrebs vorgesehen.

In einer weiteren Variante umfasst die Mutationsanalyse zumindest einen Teil des Gens *TP53* sowie die Bestimmung einer Insertion viraler DNA, insbesondere zumindest eines Teils oder mehrerer Teile der DNA eines oder mehrerer Humaner Papilloma-Viren (HPV), insbesondere in Kombination einer Methylierungsanalyse zumindest eines Teils von *SEPT9.* Beispielsweise sind die häufigsten Ursachen für Kopf- und Halstumoren Rauchen und eine Insertion des HPV Virus. Kopf- und Halstumoren, die durch eine Insertion von HPV induziert wurden, tragen in der Regel keine *TP53* Mutation. Daher wird in einer bevorzugten Variante des Verfahrens die Mutationsanalyse von *TP53* mit einer Analyse der Insertion von HPV durchgeführt, da der Erfinder erkannt hat, dass durch das Vorliegen entweder der einen oder der anderen Mutation der Großteil der Tumoren erfasst wird. *SEPT9* ist besonders häufig aberrant in Kopf- und Halstumoren methyliert. Besonders bevorzugt wird daher eine Kombination einer Methylierungsanalyse von *SEPT9* mit einer Mutationsanalyse von *TP53* und/oder einer Mutationsanalyse einer Insertion von HPV durchgeführt, da sich diese Erkrankungen auf diese Weise mit besonders hoher Präzision nachweisen lassen.

In bevorzugten Varianten des Verfahrens wird die Mutationsanalyse in Schritt B) unter Bedingungen durchgeführt, welche eine quantitative Bestimmung der mindestens einen Mutation erlauben. Alternativ oder zusätzlich kann auch die Methylierungsanalyse in Schritt C) unter Bedingung durchgeführt werden, welche eine quantitative Bestimmung des Methylierungszustands des mindestens einen CpG-Dinukleotids erlauben.

Es ist zum Beispiel möglich, dass die genomische DNA zumindest zu einem Anteil DNA einer malignen Erkrankung umfasst, wobei die DNA der malignen Erkrankung das mindestens eine, gegebenenfalls aberrant methylierte, CpG-Dinukleotid und/oder, soweit diese vorhanden ist, zumindest teilweise die mindestens eine Mutation aufweist. Dann kann der Anteil der DNA der malignen Erkrankung innerhalb der genomischen DNA anhand der Mutationsanalyse in Schritt B) und/oder der Methylierungsanalyse in Schritt C) bestimmt werden.

Es ist auch möglich, die quantitative Bestimmung der Mutation in Schritt B) mit der quantitativen Bestimmung des Methylierungszustands in Schritt C) in Beziehung zu setzen. Auf diese Weise kann zum Beispiel der Anteil mutationstragender genomischer DNA innerhalb der genomischen DNA der malignen Erkrankung ermittelt werden. Dies ist von besonderem Vorteil, da maligne Erkrankungen oft heterogen sind und zum Beispiel nicht alle Zellen der Erkrankung auch die Mutation tragen. Auf diese Weise können neben dem diagnostischen Aspekt auch vorteilhafte Erkenntnisse in Bezug auf Prognose, Prädiktion oder Verlaufskontrolle einer malignen Erkrankung gewonnen werden. Diese funktionelle Wechselwirkung von Mutationsanalyse und Methylierungsanalyse gehört zu den Alleinstellungsmerkmalen der Erfindung. Darüber hinaus kommen auch hier die bereits oben festgestellten Vorteile in Bezug auch die Robustheit und Präzision des Verfahrens sowie die Analysierbarkeit von kleinsten Mengen genomischer DNA zum Tragen.

In einer weiteren Variante umfasst die quantitative Bestimmung mehrere Mutationen verteilt auf ein oder mehrere Gene und/oder den Methylierungszustand mehrere CpG-Dinukleotide in einem oder mehreren Genen. Anschließend kann eine Mittlung der erhaltenen Quantitäten erfolgen. Für die relative Quantifizierung der genomischen DNA der malignen Erkrankung innerhalb der genomischen DNA können zum Beispiel die quantifizierten Methylierungszustände der einzelnen CpG-Dinukleotide gemittelt werden, um einen besonders robusten Wert zu erhalten. Es ist auch möglich, dasjenige CpG-Dinukleotid zu für die Quantifizierung zu verwenden, für welches der höchste Methylierungswert ermittelt wurde, da dieses CpG-Dinukleotid dann in der genomischen DNA der malignen Erkrankung besonders stark methyliert vorliegt und somit einen verlässlichen Maßstab für deren Menge darstellt. Auf diese Weise wird eine besonders hohe Robustheit und Präzision des Verfahrens erreicht.

Wenn der Anteil der genomischen DNA der malignen Erkrankung innerhalb der genomischen DNA anhand der Mutationsanalyse in Schritt B) bestimmt werden soll, umfasst die Mutationsanalyse vorzugsweise die Bestimmung mindestens einer rekurrenten Mutation. Es ist auch möglich, den Anteil der DNA der malignen Erkrankung innerhalb der genomischen DNA anhand mindestens einer Mutation eines rekurrent mutierten Gens zu bestimmen. Geeignete und bevorzugte rekurrente Mutationen und rekurrent mutierte Gene sind in Beispiel 11 aufgeführt.

In bevorzugten Varianten umfasst das Verfahren eine oder mehrere der folgenden Kombinationen: eine Mutationsanalyse umfassend zumindest einen Teil des *BRAF* Gens und eine Methylierungsanalyse umfassend zumindest einen Teil des *SHOX2* Gens; eine Mutationsanalyse umfassend zumindest einen Teil des *BRCA1, BRCA2* und/oder *PALB2* Gens und eine Methylierungsanalyse umfassend zumindest einen Teil des *BRCA1* Gens; eine Mutationsanalyse umfassend zumindest einen Teil des *IDH1, IDH2* und/oder *EGFR* Gens und eine Methylierungsanalyse umfassend zumindest einen Teil des *MGMT* Gens; eine Mutationsanalyse umfassend zumindest einen Teil des *TP53* Gens und eine Methylierungsanalyse umfassend zumindest einen Teil des *PITX2* Gens; eine Mutationsanalyse umfassend zumindest einen Teil des *AR, ESR1, BRCA1, BRCA2, PALB2* und/oder *ERBB2* (*HER2*) Gens und eine Methylierungsanalyse umfassend zumindest einem Teil des *PITX2* Gens; eine Mutationsanalyse umfassend zumindest einen Teil des *FGFR3, TERT, PIK3CA, KRAS, TP53, NRAS* und/oder *HRAS* Gens und eine Methylierungsanalyse umfassend zumindest einen Teils des *ONECUT2, OTX1, SHOX2, SEPT9* und/oder *TWIST1* Gens; eine Mutationsanalyse umfassend zumindest einen Teil des *TP53* Gens und/oder eine Insertion viraler DNA, insbesondere zumindest eines Teils oder mehrerer Teile der DNA eines oder mehrerer Humaner Papilloma-Viren (HPV) und eine Methylierungsanalyse umfassend zumindest einen Teil des *SEPT9* Gens. Es ist auch möglich, diese Kombinationen mit Methylierungs- und/oder Mutationsanalysen von zumindest Teilen weiterer Gene zu ergänzen. Diesbezüglich bevorzugte Gene und Sequenzen sind aus der vorstehenden Beschreibung und den Ausführungsbeispielen sowie dem Sequenzprotokoll ersichtlich.

Die maligne Erkrankung kann insbesondere ein Karzinom, ein Melanom, ein Sarkom, ein Gliom, ein Lymphom und/oder eine Leukämie umfassen. Das Karzinom kann zum Beispiel ein Adenokarzinom, ein Plattenepithelkarzinom, ein kleinzelliges Karzinom, ein neuroendokrines Karzinom, ein Nierenzellkarzinom, ein Urothelkarzinom, ein hepatozelluläres Karzinom, ein Analkarzinom, ein Bronchialkarzinom, ein Endometriumkarzinom, ein cholangiozelluläres Karzinom, ein hepatozelluläres Karzinom, ein Hodenkarzinom, ein kolorektales Karzinom, ein Karzinom des Kopf- und Halsbereichs, ein Karzinom des Ösophagus, ein Magenkarzinom, ein Mammakarzinom, ein Nierenkarzinom, ein Ovarialkarzinom, ein Pankreaskarzinom, ein Prostatakarzinom, ein Schilddrüsenkarzinom und/oder ein Zervixkarzinom umfassen. Ein Sarkom kann beispielsweise ein Angiosarkom, ein Chondrosarkom, ein Ewing-Sarkom, ein Fibrosarkom, ein Kaposi-Sarkom, ein Liposarkom, ein Leiomyosarkom, ein malignes fibröses Histiozytom, ein neurogenes Sarkom, ein Osteosarkom oder ein Rhabdomyosarkom sein. Eine Leukämie kann beispielsweise eine akute myeloische Leukämie (AML), eine akute lymphatische Leukämie (ALL), eine chronische lymphatische Leukämie (CLL), oder eine chronische myeloische Leukämie (CML) sein. Ein Lymphom kann ein Hodgkin-Lymphom oder Non-Hodgkin-Lymphom sein. Ein Non-Hodgkin-Lymphom kann ein B-Zell-Lymphom oder ein T-Zell-Lymphom sein.

Das Verfahren umfasst die Schritte A), B) und C), da durch das erfindungsgemäße synergistische Zusammenwirken von Mutationsanalyse und Methylierungsanalyse zum Beispiel eine signifikante Verbesserung der Sensitivität und Spezifität der Diagnose und eine differenziertere und zuverlässigere Prognose, Prädiktion und Verlaufskontrolle erreicht wird. Diesbezüglich wird auch auf die nachfolgenden Ausführungsbeispiele verwiesen.

In einer bevorzugten Verwendung des Verfahrens zur Prognose wird die Methylierungsanalyse eines Gens als prognostischer Biomarker verwendet. Vorzugsweise umfasst die Methylierungsanalyse zu diesem Zweck ein oder mehrere CpG-Dinukleotide des *PITX2* Gens. Insbesondere kann sie dazu ausgelegt sein, den Methylierungszustand von einem oder mehreren CpG-Dinukleotiden einer Sequenz zu bestimmen, welche im wildtypischen, nicht-umgewandelten Zustand mindestens 95% Sequenzidentität mit SEQ ID NO:90 und/oder SEQ ID NO:91 aufweist. Alternativ oder zusätzlich kann die Methylierungsanalyse zu diesem Zweck auch ein oder mehrere CpG-Dinukleotide eines Gens ausgewählt aus der Gruppe *CDO1, PLAU, POU4F3, TFF1* und *CXCL12,* sowie Kombinationen davon umfassen. Auf diese Weise kann zum Beispiel die maligne Erkrankung nachgewiesen und gleichzeitig eine Aussage über die Aggressivität der Erkrankung getroffen werden.

In einer bevorzugten Verwendung des Verfahrens zur Prädiktion und Prognose wird die Methylierungsanalyse eines Gens als prognostischer Biomarker und die Mutationsanalyse eines Gens als prädiktiver Biomarker verwendet. Vorzugsweise umfasst die Methylierungsanalyse zu diesem Zweck ein oder mehrere CpG-Dinukleotide des *PITX2* Gens, bevorzugt ein oder mehrere CpG-Dinukleotide einer Sequenz, welche im wildtypischen, nicht-umgewandelten Zustand mindestens 95% Sequenzidentität mit SEQ ID NO:90 und/oder SEQ ID NO:91 aufweist. Vorzugsweise umfasst die Mutationsanalyse zu diesem Zweck eine oder mehrere Mutationen der Gene *BRAF, EGFR, KRAS, NRAS, BRCA1, BRCA2, AKT1, VGFR, IDH1, IDH2, CRLF2, TSC1, PDGFRA, NF1, GNAQ, GNA11, CTNNB1, ASXL1, BCOR, DNMT3A, ETV6, EZH2, SF3B1, SRSF2, STAG2, TET2, TP53, U2AF1, ZRSR2, HRAS, TERT (hTERT), SMO, FLT3, JAK2, ESR1, BCR, SMAD4, DNMT3A, AR, ERBB2 (HER2), MAP2K1 (MEK1), PIK3CA, PTEN, PALB2, DDR2, NTRK1 (TRKA), RET, DEK-NUP214, MLL-MLLT3, CBFB-MYH11, RPN1-EVI1, RUNX1-RUNX1T1, PML-RARA, RBM15-MKL, KIT, ALK, FGFR1, MET, ERBB2 (HER2), FGFR1, FGFR2, BCR-ABL1, RET, MEK, mTOR, VEGFR* und beliebige Kombinationen dieser Gene. *Besonders* bevorzugt umfasst die Mutationsanalyse zu diesem Zweck *AR, ERBB2 (HER2), BRCA2, BRCA1* und/oder *ESR1.*

In einer Variante kann das Ansprechen auf eine Therapie mit Inhibitoren des Androgenrezeptors und/oder des Östrogenrezeptors, auf eine Therapie mit PARP Inhibitoren und/oder mit therapeutischen monoklonalen Antikörpern gerichtet gegen ERBB2 (HER2), vorhergesagt werden. In einer anderen Variante erfolgt die Prädiktion der Wirkung von Inhibitoren der Hormonrezeptoren wie zum Beispiel Antiandrogenen und/oder Antiöstrogenen. In einer weiteren Variante erfolgt die Prädiktion in Bezug auf die Wirkstoffe Enzalutamid, Abirateron, Bicalutamid, Flutamid, Tamoxifen und Cyproteronacetat. In noch einer weiteren Variante erfolgt die Prädiktion der Wirkung von PARP Inhibitoren wie zum Beispiel Talazoparib (BMN-673), Olaparib (AZD-2281), Rucaparib (AG014699, PF-01367338), Veliparib (ABT-888), CEP 9722, MK 4827, BGB-290 und/oder der Wirkung von therapeutischen monoklonalen Antikörpern wie zum Beispiel Trastuzumab. In noch einer weiteren Variante erfolgt die Prädiktion der Wirkung von AG-221, AGI-5198, AG-120, AG-881, Vemurafenib, Cetuximab, Crizotinib, Temozolomid, Erlotinib (Tarceva), Gefitinib (Iressa), Afatinib (Gilotrif), Dacomitinib, Neratinib, CO-1686 (Rociletinib), AZD9291 und/oder HM61713. Beliebige Kombinationen dieser Varianten sind auch möglich.

Bestimmte Verwendungen des Verfahrens zur Prädiktion können zum Beispiel eine Mutationsanalyse und Methylierungsanalyse desselben therapeutisch relevanten Gens umfassen. Dabei kann es sich beispielsweise um ein Tumorsuppressorgen, wie zum Beispiel ein DNA-Reparaturgen, und/oder ein Protoonkogen handeln. Beispielsweise kann eine aberrante Methylierung des therapeutisch relevanten Gens und/oder eine Mutation, zum Beispiel eine inaktivierende Mutation, auf das Ansprechen eines Patienten mit einer malignen Erkrankung auf eine bestimmte Therapie hinweisen, während der Wildtyp beziehungsweise der Normwert des therapeutisch relevanten Gens darauf hinweist, dass der Patient nicht auf die Therapie ansprechen wird. Auch der umgekehrte Fall ist möglich, also dass diese Konstellationen auf ein Nichtansprechen des Patienten auf eine Therapie hinweisen.

In bevorzugten Varianten des Verfahrens zur Prädiktion umfasst die Mutationsanalyse und/oder Methylierungsanalyse zumindest einen Teil oder mehrere Teile beziehungsweise ein oder mehrere CpG-Dinukleotide des *BRCA1, BRCA2* und/oder *PALB2* Gens. Für besonders bevorzugte Sequenzen dieser Gene wird auf die in Beispiel 8 genannten Sequenzen Bezug genommen.

Es gehört aber auch zu den Alleinstellungsmerkmalen des erfindungsgemäßen Verfahrens, dass die Wechselwirkung zwischen verschiedenen therapeutisch relevanten Genen erfasst werden kann, die sich gegenseitig in Bezug auf das Ansprechen eines Patienten auf eine Therapie beeinflussen. Dazu kann, alternativ oder zusätzlich zu der vorhergehenden Variante, zum Beispiel die Mutationsanalyse zumindest einen Teil oder mehrere Teile eines ersten therapeutisch relevanten Gens und die Methylierungsanalyse ein oder mehrere CpG-Dinukleotide von einem zweiten, vom ersten unterschiedlichen, therapeutisch relevanten Gen umfassen. Damit wird der erfinderischen Erkenntnis Rechnung getragen, dass ein Methylierungszustand eines ersten therapeutisch relevanten Gens und eine Mutation eines zweiten therapeutisch relevanten Gens gleichlaufende oder entgegengesetzte Wirkungen auf eine bestimmte Therapie haben können. Diese Wechselwirkungen können mithilfe der vorliegenden Erfindung erstmals durch die simultane Mutations- und Methylierungsanalyse funktionell in Zusammenhang gebracht und entschlüsselt werden.

In bevorzugten Varianten des Verfahrens zur Prädiktion umfasst die Methylierungsanalyse daher ein oder mehrere CpG-Dinukleotide des *MGMT* Gens und die Mutationsanalyse zumindest einen Teil oder mehrere Teile der Gene *IDH1, IDH2* und/oder *EGFR.* Für besonders bevorzugte Sequenzen innerhalb dieser Gene wird auf Beispiel 9 Bezug genommen.
In einer bevorzugten Verwendung des Verfahrens zur Prädiktion erfolgt die Vorhersage des Ansprechens eines Patienten auf eine Therapie mit mindestens einem Wirkstoff ausgewählt aus den Gruppen alkylierender Chemotherapeutika, Zytostatika, therapeutischer monoklonaler Antikörper und Inhibitoren, insbesondere Tyrosin-Kinase-Inhibitoren. Besonders bevorzugt erfolgt die Vorhersage in Bezug auf einen Wirkstoff ausgewählt aus Temozolomid, Cetuximab, Bevacizumab, AG-221, AGI-5198, AG-120, AG-881, oder Kombinationen davon.

In einer bevorzugten Variante des Verfahrens zur Verlaufskontrolle einer malignen Erkrankung eines Patienten ist vorgesehen, dass die Schritte B) und C) unter Bedingungen durchgeführt werden, welche eine quantitative Bestimmung der mindestens einen Mutation und/oder des Methylierungszustands des mindestens einen CpG-Dinukleotids erlauben. Vorzugsweise umfasst die Verwendung des Verfahrens zur Verlaufskontrolle einer malignen Erkrankung dann die folgenden Schritte: i) Bereitstellen einer ersten Probe mit genomischer DNA des Patienten von einem ersten Zeitpunkt und Durchführen eines Verfahrens gemäß dem ersten Aspekt, wobei der Anteil der DNA der malignen Erkrankung in der genomischen DNA anhand der Mutationsanalyse in Schritt B) und/oder, falls vorhanden, anhand der Methylierungsanalyse im Schritt C) bestimmt wird, ii) Bereitstellen einer zweiten Probe mit genomischer DNA des Patienten von einem nach dem ersten Zeitpunkt liegenden zweiten Zeitpunkt und Wiederholen des Verfahrens aus Schritt i) mit der zweiten Probe. Der Verlauf der malignen Erkrankung kann dann anhand einer Änderung des Anteils der DNA der malignen Erkrankung in der genomischen DNA in der zweiten Probe im Vergleich zur ersten Probe kontrolliert werden. Zum Beispiel kann der erste Zeitpunkt vor Beginn der Therapie liegen und der zweite Zeitpunkt nach Beginn der Therapie. Eine Erhöhung des Anteils der DNA der malignen Erkrankung in der zweiten Probe kann dann zum Beispiel auf ein Ansprechen des Patienten auf die Therapie hinweisen. Dies ist zum Beispiel möglich, wenn der Tumor abstirbt und im Sterbeprozess vermehrt genomische DNA der malignen Erkrankung in das Blut abgibt. Es ist auch möglich, dass eine Erhöhung des Anteils der DNA der malignen Erkrankung in der zweiten Probe auf ein Nicht-Ansprechen des Patienten auf die Therapie hinweisen. Dies ist zum Beispiel möglich, wenn der Tumor trotz Therapie wächst und im Wachstumsprozess vermehrt genomische DNA der malignen Erkrankung in das Blut abgibt. Es ist auch möglich, dass eine geringe Abnahme des Anteils der DNA der malignen Erkrankung in der zweiten Probe auf ein Nicht-Ansprechen des Patienten auf die Therapie hinweisen. Dies ist zum Beispiel möglich, wenn nur ein Teil des Tumors auf die Therapie anspricht und abstirbt, sodass weiter genomische DNA der malignen Erkrankung in das Blut gelangt.

In einer Variante des Verfahrens zur Verlaufskontrolle liegt in Schritt i) der erste Zeitpunkt 2 bis 12 Tage, vorzugsweise 4 bis 10 Tage, besonders bevorzugt 6 bis 8 Tage nach dem Beginn einer Therapie, zum Beispiel einer Strahlentherapie. In Schritt ii) kann der zweite Zeitpunkt 10 bis 31 Tage, vorzugsweise 14 bis 28 Tage, besonders bevorzugt 18 bis 24 Tage nach dem Beginn der Therapie liegen. In einer weiteren Variante kann der erste oder zweite Zeitpunkt auch 1 bis 5 Tage, vorzugsweise 2 bis 4 Tage nach Beginn einer Therapie oder einem Eingriff, zum Beispiel einer Operation, liegen. Derjenige Tag, an dem die Therapie begonnen wurde oder an dem der Eingriff erfolgte, gilt als Tag 0. Aufgrund der bereits dargelegten Vorteile wird durch das erfindungsgemäße Verfahren ein besonders spezifischer Nachweis von Tumor-DNA im Blut erreicht, sodass zum Beispiel mit hoher Zuverlässigkeit auf den Verbleib eines Residualtumors und/oder das Vorhandensein von okkulten Metastasen nach einer Operation geschlossen werden kann.

Es ist möglich, mit dem erfindungsgemäßen Verfahren auch solche Mutationen in genomischer DNA zu messen, die keinen Ursprung in einer malignen Erkrankung haben. Beispielsweise ist es möglich, Mutationen in fetaler DNA zu bestimmen, die im Blut der Mutter zirkuliert. Der Nachweis von epileptischer Enzephalopathien des ungeborenen Kindes ist zum Beispiel möglich anhand der erfindungsgemäßen Analyse fetaler DNA im Blut der Mutter. Vorzugsweise werden hierzu Mutationen der Gene *ALDH7A1, PNPO, SLC2A1, MECP2, FOXG1, ARX, CDKL5, STXBP1, SPTAN1, SCN1A, EIEE6, KCNQ2, EIEE7, ARHGEF9, EIEE8, PCDH19, PNKP, EIEE10, SCN2A,* und/oder *EIEE11* untersucht. Es ist auch möglich, weitere genetische Erkrankungen des ungeborenen Kindes im Blut der Mutter nachzuweisen. Vorzugsweise ermöglicht die Verwendung des erfindungsgemäßen Verfahrens die Diagnose von Achondroplasie anhand von Mutationen im *FGFR3* Gen; Alpha-1-Antitrypsin-Mangel anhand von Mutationen im *SERPINA1* Gen; Zystischer Fibrose anhand von Mutationen im *CFTR* Gen; Gaucher-Krankheit anhand von Mutationen im *GBA* Gen; Muskeldystrophie Duchenne anhand von Mutationen im *DMD* Gen; Mediterranem Fieber anhand von Mutationen im *MEFV* Gen; Fragilem X-Syndrom anhand von Mutationen im *FMR1* Gen; hereditärer Hämochromatose anhand von Mutationen im *HFE* Gen; Huntington-Krankheit anhand von Mutationen im *HTT* Gen; Marfan-Syndrom anhand von Mutationen im *FBN1* Gen; Myotonischer Dystrophie anhand von Mutationen in den Genen *CNPB* und/oder *DMPK;* Faktor V Leiden Thrombophilie anhand von Mutationen im *F5* Gen; Hämophilie anhand von Mutationen in den Genen *F8* und/oder *F9;* Noonan Syndrom anhand von Mutationen in den Genen *PTPN11, SOS1, RAF1* und/oder *KRAS*; Phenylketonurie anhand von Mutationen im Gen *PAH;* X-Chromosomaler schwerer kombinierter Immundefizienz anhand von Mutationen im Gen *IL2RG;* Sichelzellenanämie und Thalassämie anhand von Mutationen im Gen *HBB;* spinaler Muskelatrophie anhand von Mutationen in den Genen *SMN1* und/oder *VAPB;* Neurofibromatose Typ I und II anhand von Mutationen in den Genen *NF1* und *NF2;* Hypercholesterinämie anhand von Mutationen im *LDLR* Gen; Osteogenesis Imperfecta anhand von Mutationen in den Genen *COL1A1* und/oder *COL1A2;* Tay-Sachs-Krankheit anhand von Mutationen im Gen *HEXA;* Velo-Cardio-Facial Syndrom anhand von Mutationen in den Genen *COMT* und/oder *TBX1;* Morbus Wilson anhand von Mutationen im Gen *ATP7B;* Trimethylaminurie anhand von Mutationen im Gen *FMO3.* Es ist auch möglich, chromosomale Mutationen wir Trisomie 21, Deletion im Chromosom 5 und/oder Mikrodeletionen im Chromosom 15 erfindungsgemäß zur Diagnose von Down-Syndrom, Katzenschrei-Syndrom und Angelman Syndrom nachzuweisen. Beliebige Kombinationen dieser Verwendungen sind auch möglich.

Es ist auch möglich, weitere gutartige und/oder nicht-maligne Erkrankungen durch die Verwendung des erfindungsgemäßen Verfahrens nachzuweisen. Bevorzugt sind *KCNJ5* Mutationen bei Corstisol-produzierende Adenomen der Nebenniere und bei erblichem Bluthochdruck; PRKACA und/oder *KCNJ5* Mutationen bei Corstisol-produzierende Adenomen und Hyperplasien der Nebeniere; und *CACNA1D* Mutationen bei Aldosteroneproduzierenden Adenomen und primären Aldosteronismus. Ferner sind Kombinationen dieser Verwendungen möglich.

Der zweite Aspekt der Erfindung betrifft eine Verwendung eines Kit zur Durchführung des Verfahrens nach dem ersten Aspekt. Das Kit umfasst a) mindestens ein erstes Oligonukleotid-Paar ausgelegt zur Hybridisierung an die aus Schritt A) erhaltene genomische DNA, um zumindest einen ersten Teilbereich der genomischen DNA zu amplifizieren, welcher in Verdacht steht, die mindestens eine Mutation zu enthalten. Es ist auch möglich, dass der erste Teilbereich zusätzlich das mindestens eine CpG-Dinukleotid beinhaltet, dessen Methylierungszustand zu bestimmen ist.

Zusätzlich umfasst das Kit b) mindestens ein zweites Oligonukleotid-Paar ausgelegt zur Hybridisierung an die aus Schritt A) erhaltene genomische DNA, um zumindest einen zweiten Teilbereich der genomischen DNA zu amplifizieren, welcher das mindestens eine CpG-Dinukleotid beinhaltet, dessen Methylierungszustand zu analysieren ist.

Es ist möglich, dass das erste und/oder zweite Oligonukleotid-Paar derart ausgelegt ist, dass derjenige Abschnitt des Teilbereichs, welcher im Verdacht steht, die mindestens eine Mutation zu enthalten, beziehungsweise derjenige Teilbereich, welcher das mindestens eine CpG-Dinukleotid beinhaltet, zumindest teilweise revers-komplementär zu einem der Oligonukleotide des ersten beziehungsweise zweiten Oligonukleotid-Paars ist. Auf diese Weise wird eine besonders spezifische Amplifikation des jeweiligen zu analysierenden Bereichs erreicht. Es ist aber auch möglich, dass der Abschnitt, in dem die Mutation vermutet wird beziehungsweise der Abschnitt, welcher das CpG-Dinukleotid beinhaltet, zwischen den zu den Oligonukleotiden revers-komplementären Sequenzen liegt.

Das Kit kann auch weitere erste und zweite Oligonukleotid-Paare enthalten, um entsprechend weitere erste und/oder zweite Teilbereiche der umgewandelten genomischen DNA zu amplifizieren, welche im Verdacht stehen, eine Mutation zu enthalten oder welche ein CpG-Dinukleotid beinhalten, dessen Methylierungszustand zu analysieren ist.

Vorzugsweise sind das erste und das zweite Oligonukleotid-Paar sowie gegebenenfalls weitere Oligonukleotid-Paar für eine Duplex-PCR beziehungsweise Multiplex-PCR ausgelegt. Vorzugsweise liegt der GC-Gehalt der Oligonukleotide im Bereich von 20 bis 70%, besonders bevorzugt bei 30 bis 60%. Vorzugsweise liegt die Primerlänge zwischen 17 und 35 Nukleotiden, besonders bevorzugt zwischen 18 und 30 Nukleotiden. Vorzugsweise enthält ein Primer eines Primerpaares weniger als vier Cytosine und der andere Primer des Primerpaares weniger als vier Guanine in der DNA bindenden Sequenz. Besonders bevorzugt enthält ein Primer eines Primerpaares kein Cytosin und der andere Primer des Primerpaares kein Guanin in der DNA bindenden Sequenz.

Vorzugsweise enthält die Bindestelle eines Oligonukleotids eines Oligonukleotid-Paares in der aus A) erhaltenen umgewandelten genomischen DNA keine Cytosine und keine Methylcytosine. Vorzugsweise enthält die Bindestelle des einen Oligonukleotids eines Oligonukleotid-Paares beziehungsweise die revers-komplementäre Bindestelle des anderen Oligonukleotids des Oligonukleotid-Paares in der aus A) erhaltenen umgewandelten genomischen DNA kein CpG-Dinukleotid.

In bestimmten Varianten sind die Oligonukleotide des ersten Oligonukleotid-Paars derart ausgelegt, dass sie jeweils zu einer Sequenz der umgewandelten DNA zumindest teilweise komplementär sind, in denen keine Umwandlung von Cytosin stattfindet, insbesondere die kein Cytosin enthält.

In einer bevorzugten Variante umfasst der erste Teilbereich zumindest einen Teilbereich von *BRAF.* In einer weiteren bevorzugten Variante umfasst der erste Teilbereich zumindest einen Teilbereich von *EGFR.* In einer weiteren bevorzugten Variante umfasst der erste Teilbereich zumindest einen Teilbereich von *KRAS.* In einer weiteren bevorzugten Variante umfasst der erste Teilbereich zumindest einen Teilbereich von *BRCA1.* In einer weiteren Variante umfasst der erste Teilbereich zumindest einen Teilbereich von *BRCA2.* In wiederum einer bevorzugten Variante umfasst der erste Teilbereich zumindest einen Teilbereich von *PALB2.* In einer weiteren bevorzugten Variante umfasst der erste Teilbereich zumindest einen Teilbereich von *IDH1.* In noch einer weiteren bevorzugten Variante umfasst der erste Teilbereich zumindest einen Teil von *IDH2.* In einer weiteren bevorzugten Variante umfasst der erste Teilbereich zumindest einen Teil von *TP53.* Darüber hinaus sind beliebige Kombinationen dieser ersten Teilbereiche möglich.

In einer bevorzugten Variante umfasst der zweite Teilbereich zumindest einen Teil von *PITX2.* In einer weiteren bevorzugten Variante umfasst der zweite Teilbereich zumindest einen Teil von *CDO1, PLAU, POU4F3, TFF1* und/oder *CXCL12.* In einer weiteren bevorzugten Variante umfasst der zweite Teilbereich zumindest einen Teil von *MGMT.* In noch einer bevorzugten Variante umfasst der zweite Teilbereich zumindest einen Teil von *SHOX2.* In noch einer bevorzugten Variante umfasst der zweite Teilbereich zumindest einen Teil von *SEPT9.* Beliebige Kombinationen dieser zweiten Teilbereiche sind ebenfalls umfasst.

Ferner sind beliebige Kombinationen eines oder mehrerer erster und zweiter Teilbereiche möglich. In einer bevorzugten Variante umfasst der erste Teilbereich zum Beispiel zumindest einen Teil des *BRAF* Gens und der zweite Teilbereich zumindest einen Teil des *SHOX2* Gens. In einer weiteren bevorzugten Variante umfasst der erste Teilbereich zumindest einen Teil des *BRCA1, BRCA2 oder PALB2* Gens und der zweite Teilbereich zumindest einen, gegebenenfalls zweiten, Teil des *BRCA1* Gens. In einer weiteren bevorzugten Variante umfasst der erste Teilbereich zumindest einen Teil des *IDH1, IDH2* und/oder *EGFR* Gens und der zweite Teilbereich zumindest einen Teil des *MGMT* Gens. In einer weiteren bevorzugten Variante umfasst der erste Teilbereich zumindest einen Teil des *TP53* Gens und der zweite Teilbereich zumindest einen Teil des *PITX2* Gens.

Weitere bevorzugte Teilbereiche und bevorzugte Sequenzen sind aus den vorstehenden Beschreibungen und den Ausführungsbeispielen sowie dem Sequenzprotokoll ersichtlich.

Das Kit umfasst vorzugsweise eine Gebrauchsanweisung für die Durchführung des Verfahrens nach dem ersten Aspekt.

### Detaillierte Beschreibung von Ausführungsbeispielen

Nachfolgend wird die Erfindung anhand von Ausführungsbeispielen und experimentellen Ergebnissen näher beschrieben. Diese Ausführungsbeispiele dienen der Erläuterung und nicht der Beschränkung auf spezifische Details.

### Beispiel 1: Bestimmung einer Punktmutation in nicht-umgewandelter genomischer DNA (Referenz)

Punktmutationen sind solche Mutationen, bei denen nur eine einzelne Nukleinbase von der Veränderung betroffen ist. Die zuverlässige Bestimmung von Punktmutationen stellt demzufolge hohe Anforderungen an die Spezifität und Sensitivität eines molekulardiagnostischen Nachweisverfahrens.

Als Beispiel für eine klinisch hochrelevante Punktmutation wurde die Punktmutation V600E innerhalb des *BRAF* Gens untersucht.

Die zu analysierende genomische DNA kann aus verschiedenen Quellen stammen. Beispielhaft wurde hier fixiertes Gewebe verwendet. Jeweils drei Dünnschnitte von je 10 *µ*m eines Formalin-fixierten und in Paraffin eingebetteten malignen Melanoms sowie ein Gewebestück des an das Melanom angrenzenden Normalgewebes (Haut) wurden in separate 2 ml Reaktionsgefäße überführt. Anschließend wurde die genomische DNA aus den Gewebeschnitten extrahiert. Hierfür eignete sich zum Beispiel die Verwendung des QIAamp DNA FFPE Tissue Kit (Qiagen, Hilden, Deutschland) nach Herstellerangaben.

Die nicht-umgewandelte genomische DNA wurde anschließend mithilfe eines NanoDrop ND-1000 Spektrophotometers (Thermo Fisher Scientific, Waltham, MA, USA) quantifiziert.

In der nicht-umgewandelten genomischen DNA wurde anschließend der Lokus des *BRAF* Gens amplifiziert, in dem die Mutation vermutet wurde. Dazu wurde der Lokus mithilfe einer PCR mit dem Vorwärtsprimer SEQ ID NO:1 und dem Rückwärtsprimer SEQ ID NO:2 amplifiziert, um die resultierende Sequenz mit der nicht-umgewandelten wildtypischen Sequenz SEQ ID NO:3 abzugleichen. Die PCR Reaktionen wurden zum Beispiel in 20 *µ*l Volumina unter folgenden Bedingungen durchgeführt: 2 *µ*l PCR Reaktionspuffer mit 20 mM MgCl₂ (10-fach konzentriert, Roche, Penzberg, Deutschland), 2 U FastStart *Taq* DNA-Polymerase (Roche), 0,4 *µ*M jedes Primers, 0,25 mM jedes dNTP (dTTP, dATP, dGTP, dCTP). Die PCR wurde zum Beispiel mittels eines DNA Engine Tetrad Thermocyclers (Biorad, USA) durchgeführt. Ein geeignetes Temperaturprofil umfasste beispielsweise folgende Schritte: 10 min bei 95 °C gefolgt von 40 Zyklen je 45 s bei 54 °C, 45 s bei 72 °C und 15 s bei 95°C. Das resultierende PCR-Produkt wurde anschließend per Sanger-Sequenzierung sequenziert, wobei der Vorwärtsprimer SEQ ID NO:1 als Sequenzierprimer verwendet wurde. Die Sanger Sequenzierung von PCR Produkten ist eine dem durchschnittlichen Fachmann leicht zugängliche Methode und wird darüber hinaus von vielen Anbietern als Dienstleistung angeboten. Die in diesem Anwendungsbeispiel durchgeführten Sanger-Sequenzierungen wurden beispielsweise durch Beckman Coulter Genomics, Hope End, Takeley, Essex CM22 6TA, Großbritannien, generiert.

Figur 1A zeigt das Ergebnis der als Referenz dienenden Analyse der nicht-umgewandelten genomischen DNA aus dem Normalgewebe. Das Normalgewebe weist ausschließlich die wildtypische Sequenz CAC auf. Es ist demnach an dieser Stelle keine Punktmutation vorhanden. Figur 1B zeigt das Ergebnis der als Referenz dienenden Analyse mit der nicht-umgewandelten genomischen DNA des malignen Gewebes. In der Sequenz der genomischen DNA des Melanoms ist neben der wildtypischen Sequenz CAC auch die V600E Mutation mit der Basenfolge CTC zu finden.

### Beispiel 2: Bestimmung einer Punktmutation in genomischer DNA

Von der extrahierten DNA aus Beispiel 1 wurde jeweils eine Teilportion der genomischen DNA erfindungsgemäß umgewandelt. Die Umwandlung kann zum Beispiel durch Kontaktieren der genomischen DNA mit Bisulfit erfolgen. Vorliegend wurde die Umwandlung zum Beispiel mit dem innuCONVERT Bisulfit All-In-One Kit (Analytik Jena, Jena, Deutschland) durchgeführt. Zu diesem Zweck wurden 2 *µ*g von der extrahierten DNA jeder Probe nach Herstellerangaben umgewandelt. Anschließend wurde die Menge umgewandelter DNA mithilfe eines NanoDrop ND-1000 Spektrophotometers (Thermo Fisher Scientific, Waltham, MA, USA) quantifiziert.

Im Normalzustand ist DNA in Form einer Doppelhelix organisiert, welche aus zwei zueinander revers-komplementären Einzelsträngen besteht. Ein Strang wird als Plus- oder Vorwärtsstrang und der andere Strang als Minus- oder Rückwärtstrang bezeichnet. Nach der Umwandlung der DNA zum Beispiel mit Bisulfit sind der Plus- und der Minusstrang nicht mehr revers-komplementär zueinander. Der Strang, der durch Bisulfit-Umwandlung des Plusstrangs entsteht, wird für die Beschreibung der Erfindung als Bisulfit-I Strang bezeichnet. Der Strang, der durch Bisulfit-Umwandlung des Minusstrangs entsteht, wird als Bisulfit-II Strang bezeichnet.

Im Rahmen der erfindungsgemäßen Verfahrensausführung kann die Mutationsanalyse mit einer Amplifikation von umgewandelter genomischer DNA verbunden sein. Im vorliegenden Beispiel wurde die umgewandelte genomische DNA des Melanoms und des Normalgewebes mithilfe einer quantitativen Echtzeit-PCR (qPCR) amplifiziert und gleichzeitig quantifiziert, siehe unten. Vorzugsweise wird eine PCR beziehungsweise qPCR als Duplex- oder Multiplex-PCR ausgeführt, bei welcher innerhalb der gleichen Reaktion zum Beispiel ein oder mehrere erste Teilbereiche der umgewandelten genomischen DNA amplifiziert werden, welche im Verdacht stehen, eine Mutation zu enthalten, und ein oder mehrere zweite Teilbereiche der umgewandelten genomischen DNA, welche ein CpG-Dinukleotid beinhalten, dessen Methylierungszustand zu analysieren ist.

Zur Quantifizierung der Gesamtmenge an umgewandelter genomischer DNA wurde der Lokus des *BRAF* Gens in der qPCR mit den oben in Beispiel 1 beschriebenen Primersequenzen amplifiziert. Die Primer SEQ ID NO:1 und SEQ ID NO:2 wurden zum Beispiel so ausgelegt, dass sie in der Zielsequenz auf dem Bisulfit-II Strang in Bereichen hybridisieren, in denen keine Umwandlung von Cytosin stattfindet. Diese Sequenz des Bisulfit-II Strangs entspricht im Falle der Abwesenheit einer Mutation der Sequenz SEQ ID NO:4. Durch die Wahl dieser Primer kann gleichzeitig sowohl nicht-umgewandelte genomische DNA als auch umgewandelte genomische DNA amplifiziert und quantifiziert werden.

Die sequenzspezifische Detektion und Quantifizierung eines Amplifikats kann auf verschiedenen Wegen erfolgen. Zum Beispiel kann sie mithilfe einer Sonde stattfinden. Beispielsweise wurde vorliegend die Detektion des *BRAF* Amplifikats mit einer Sonde der SEQ ID NO:5 realisiert, welche ein Fluorophor/Quencher-System Atto-647N/BHQ-2 enthielt.

Die erfindungsgemäße Methylierungsanalyse kann zum Beispiel eine methylierungsspezifische Amplifikationsreaktion umfassen. Beispielsweise können zur Amplifikation Oligonukleotide verwendet werden, welche in Kombination nur dann zu einem Amplifikat führen, wenn das oder die zu untersuchende(n) CpG-Dinukleotid(e) methyliert vorlag(en). Eine geeignete Methode ist zum Beispiel in WO 02/072880 A2 beschrieben, auf welche diesbezüglich vollinhaltlich Bezug genommen wird. Vorzugsweise wird eine solche methylierungsspezifische Amplifikationsreaktion als Duplex- oder Multiplex-PCR in Kombination mit der oben genannten Amplifikation der Mutationsanalyse ausgeführt.

Zum Beispiel wurde für die methylierungsspezifische Amplifikation des *SHOX2* Genlokus, dessen nicht-umgewandelter wildtypischer Zustand der SEQ ID NO:6 entspricht, der Vorwärtsprimer SEQ ID NO:7, der Rückwärtsprimer SEQ ID NO:8 und ein Blocker-Oligonukleotid SEQ ID NO:9 verwendet. Das Blocker-Oligonukleotid trägt wie in WO 02/072880 A2 beschrieben am 3'-Ende anstelle der OH-Gruppe ein Phosphat und kann dadurch nicht durch die Polymerase verlängert werden, verhindert aber die Hybridisierung des Vorwärtsprimers an die umgewandelte genomische DNA, wenn diese unmethyliert vorlag. In dieser beispielhaften Ausgestaltung wurde somit eine methylierungsspezifische Amplifikation des Bisulfit-I Strangs (SEQ ID NO:10) des *SHOX2* Gens in der umgewandelten genomischen DNA erreicht. Für die sequenzspezifische Detektion des Amplifikats wurde das 6-FAM/BBQ-650 dual-markierte Oligonukleotid der SEQ ID NO:11 verwendet.

Es ist möglich, zum Erreichen einer besonders hohen Genauigkeit der quantitativen Methylierungsanalyse eine Kalibrierung durchzuführen. Beispielsweise kann eine Kalibrierung mit einer DNA durchgeführt werden, welche die gleiche Sequenz wie der in der Methylierungsanalyse zu untersuchende Genlokus aufweist und vor der Umwandlung zu einem oder mehreren definierten Anteilen methyliert wurde, beispielsweise zu 50% oder 100%, nachfolgend auch als Standard-DNA bezeichnet. In diesem Beispiel wurde die CpG Methyltransferase *M.Sss*I (New England Biolabs, Ipswich, MA, USA) entsprechend der Herstellerangaben verwendet um, um alle CpG-Dinukleotide in humaner genomischer DNA aus Leukozytenfilm (Roche Applied Science, Penzberg, Deutschland) vollständig zu methylieren. Insbesondere wurden dabei alle CpG-Dinukleotide innerhalb der Sequenz SEQ ID NO:6 methyliert. Anschließend wurde die DNA mittels des innuCONVERT All-In-One Bisulfit Kits entsprechend der Herstellerangaben umgewandelt.

Für die quantitative Methylierungsanalyse wurden anschließend jeweils 5 ng der umgewandelten Standard-DNA beziehungsweise jeweils 50 ng der umgewandelten DNA des malignen Melanoms oder des Normalgewebes in der vorgehend beschriebenen Amplifikationsreaktion eingesetzt.

Im vorliegenden Beispiel wurde die Echtzeit-PCR Quantifizierung in 20 *µ*l PCR Reaktionen in jeweils drei unabhängigen Messungen durchgeführt, wobei sich beispielsweise die folgende Zusammensetzung eignete: 35 mM Tris-HCl, pH 8,4, 6 mM MgCl₂, 50 mM KCl, 4% Glycerin, 0,25 mM jedes dNTP (dTTP, dATP, dGTP, dCTP), 2 U FastStart *Taq* DNA-Polymerase (Roche Applied Science, Penzberg, Deutschland), 0,4 *µ*M jedes Primers, 0,75 *µ*M Blocker-Oligonukleotid, 0,2 *µ*M jeder Detektionssonde. Die qPCR wurde zum Beispiel mittels eines AB 7500 Fast Real-Time PCR System (Life Technologies Corporation, Carlsbad, CA, USA) durchgeführt. Ein geeignetes Temperaturprofils umfasste beispielweise folgende Schritte: 20 min bei 95 °C gefolgt von 45 Zyklen je 45 s bei 56 °C und 15 s bei 95 °C.

Der Methylierungszustand in der umgewandelten DNA des malignen Melanoms beziehungsweise in dem Normalgewebe wurde mittels der DeltaDelta-CT Methode berechnet und prozentual gegenüber der Standard-DNA ausgedrückt, welche hierfür als 100% angenommen wurde. Die in diesem Beispiel verwendeten Vorwärtsprimer SEQ ID NO:7, Rückwärtsprimer SEQ ID NO:8, Blocker-Oligonukleotid SEQ ID NO:9 und die dual-markierte Sonde SEQ ID NO:11 sind beispielsweise so ausgelegt, dass insgesamt neun unterschiedliche CpG-Dinukleotide des *SHOX2* Genlokus von der Methylierungsanalyse umfasst sind. Der nach der DeltaDelta-CT Methode berechnete prozentuale Methylierungswert spiegelt dabei denjenigen Wert wider, den eine entsprechende Mischung aus DNA hätte, die zu diesen prozentualen Teilen aus Sequenzen besteht, die an diesen neun CpG-Dinukleotiden entsprechend methyliert beziehungsweise unmethyliert sind. Beispielsweise entspricht ein gemessener Methylierungswert von 66% dem Verhalten in der qPCR, welches eine Mischung aus Sequenzen hätte, von denen 66% an allen neun analysierten CpG-Dinukleotiden methyliert sind, während 34% der Sequenzen keine Methylierung an den neun CpG-Dinukleotiden aufweist.

Die Methylierungsanalyse mithilfe der qPCR zeigte, dass der Methylierungszustand des *SHOX2* Genlokus in der genomischen DNA aus dem Gewebeschnitt des Melanoms bei 66% lag, während in dem Gewebestück der angrenzenden Haut nur eine Methylierung von weniger als 1% gemessen wurde. Somit konnte gezeigt werden, dass die extrahierte genomische DNA des Melanoms einen hohen Anteil an DNA der malignen Erkrankung enthielt.

Anschließend wurde das aus der qPCR resultierende Amplifikat des *BRAF* Gens per Sanger-Sequenzierung wie in Beispiel 1 beschrieben sequenziert. Hierbei kam statt des Primers SEQ ID NO:1 ein modifizierter Sequenzierprimer SEQ ID NO:12 zum Einsatz, der im Vergleich zu SEQ ID NO:1 eine leicht verschobene Zielsequenz hatte. Auf diese Weise kann vermieden werden, dass unspezifische Amplifikate aus der qPCR sequenziert werden.

Gleichzeitig mit der Bestimmung der genomischen konvertierten DNA der malignen Erkrankung konnte durch die Sanger-Sequenzierung des qPCR-Produkts der umgewandelten DNA das Vorliegen der *BRAF* V600E Mutation in der genomischen DNA des Melanoms zweifelsfrei bestätigt werden (Figur 1D), während das anliegende Normalgewebe keine Mutation aufwies (Figur 1C).

Das erfindungsgemäße Verfahren erlaubt demzufolge eine zuverlässige Bestimmung von Punktmutationen unter Verwendung von umgewandelter genomischer DNA. Gleichzeitig erlaubt das erfindungsgemäße Verfahren demzufolge auch eine simultane Bestimmung des Methylierungszustands mindestens eines CpG-Dinukleotids, beispielsweise als Nachweis für das Vorliegen von DNA einer malignen Erkrankung in einer Probe genomischer DNA, und des Vorliegens einer Mutation. Diese simultane Bestimmung hat die zusätzliche Wirkung, dass über die Korrelation der Mutationsanalyse und der Methylierungsanalyse falsch-negative Resultate einer Mutationsanalyse wirksam vermieden werden können. Solche falsch-negativen Befunde können in herkömmlichen Analyseverfahren zum Beispiel durch die unbemerkte Abwesenheit von DNA einer malignen Erkrankung in einer zu analysierenden Probe mit genomischer DNA entstehen.

Für den fachkundigen Leser ist ersichtlich, dass sich diese Methode leicht für die Bestimmung weiterer Mutation und andere maligne Erkrankungen anpassen lässt. Die Mutationen können dabei einzeln oder in Kombination miteinander simultan analysiert werden. Es können auch andere Methylierungsbiomarker einzeln oder in Kombination miteinander analysiert werden. Der Kombination mehrerer DNA Methylierungsbiomarker und/oder Mutationen sind bei der Verwendung einer Echtzeit-PCR basierten Technologie lediglich durch die Auslesbarkeit verschiedener Farbstoffe Grenzen gesetzt. Eine noch höhere Multiplexierbarkeit lässt sich durch andere Methoden erreichen. Ein bevorzugtes Beispiel für solch eine Methode ist die multiplexierte PCR-Amplifikation mehrerer Loci mit anschließender Analyse der PCR-Amplifikate mittels NGS.

### Beispiel 3: Bestimmung einer Mutation in frei zirkulierender genomischer DNA

Die Mutationsanalyse in frei zirkulierender genomischer DNA aus Körperflüssigkeiten ist besonders attraktiv, da sie keinen chirurgischen Eingriff am Patienten erfordert. Gleichzeitig birgt diese Methode ein erhöhtes Risiko, falsch-negative Ergebnisse zu produzieren, da die Menge an DNA einer malignen Erkrankung stark vom Stadium der Erkrankung abhängen kann. Gerade im Frühstadium zirkulieren oftmals nur sehr geringe Mengen genomischer DNA einer malignen Erkrankung im Körper, welche vor einem Hintergrund gesunder DNA schwer nachzuweisen sind.

Dieses Problem wurde im Rahmen der Erfindung erkannt und mithilfe des erfindungsgemäßen Verfahrens gelöst, wie am nachfolgenden Beispiel gezeigt wird.

Beispielhaft wurde als Körperflüssigkeit das Blutplasma von vier ausgewählten Patienten (A, B, C, D) mit einem malignen Melanom analysiert. Zwei der Patienten (C und D) befanden sich in einem fortgeschrittenen Stadium der Erkrankung und hatten somit eine hohe Tumorlast. Daher war bei diesen Patienten ein hoher Anteil an frei zirkulierender Tumor DNA im Blut zu erwarten. Die beiden anderen Patienten (A und B) litten an einem malignen Melanom in einem früheren Stadium mit sehr geringer Tumorlast. Demzufolge musste bei diesen Patienten von einer geringen Menge frei-zirkulierender Tumor DNA ausgegangen werden.

Zunächst wurden Referenzanalysen mit der nicht-umgewandelten genomischen DNA des malignen Gewebes wie in Beispiel 1 beschrieben durchgeführt. Einer der Patienten (D) im fortgeschrittenen Stadium und ein Patient im Frühstadium (B) zeigten nach Analyse des Tumorgewebes eine V600E *BRAF* Mutation, während die anderen beiden Patienten (A und C) diese Mutation nicht im Tumorgewebe aufwiesen.

Zur Durchführung des erfindungsgemäßen Verfahrens wurde von jedem Patienten genomische DNA aus jeweils 3 ml Blutplasma mithilfe des innuCONVERT Bisulfit Body Fluids Kit (Analytik Jena, Jena, Deutschland) nach Herstellervorgaben zunächst aufkonzentriert und anschließend umgewandelt. Die in diesem Kit angewendete Methode zur Aufkonzentrierung der frei zirkulierenden DNA aus Plasma basiert beispielsweise auf einer polymergestützen Anreicherung. Die Umwandlung erfolgte im Rahmen des Kit-Protokolls durch Kontaktieren mit Bisulfit. Die umgewandelte genomische DNA wurde am Ende in 60 *µ*l eluiert. Diese 60 *µ*l mit umgewandelter genomischer DNA wurden in sechs Ansätzen zu jeweils 10 *µ*l in einer quantitativen Echtzeit-PCR eingesetzt, welche analog Beispiel 2 durchgeführt wurde.

Durch Korrelieren der quantitativen Methylierungsanalyse des *SHOX2* Genlokus und der quantitativen Bestimmung der frei zirkulierenden DNA anhand des *BRAF* Genlokus wurde anschließend der Anteil der DNA der malignen Erkrankung in der zirkulierenden DNA bestimmt.

Figur 2 zeigt die Ergebnisse der kombinierten Methylierungsanalyse von *SHOX2* und Mutationsanalyse von *BRAF* im Plasma der Melanompatienten. Die Diagramme in der linken Spalte zeigen die Resultate der quantitativen Echtzeit-PCR für den Anteil von *BRAF* DNA Kopien unabhängig von Methylierungs- und Mutationszustand als Maß für die Gesamtmenge genomischer DNA und für die Anzahl methylierter *SHOX2* DNA Kopien als Maß für den Anteil der DNA der malignen Erkrankung in der genomischen DNA. Die rechte Spalte zeigt die Resultate der Sequenzierung des bei der Echtzeit-PCR generierten *BRAF* Amplifikats (A: Patient mit geringer Tumorlast und gemäß Referenzanalyse mit *BRAF* Wildtyp im Primärtumor; B: Patient mit geringer Tumorlast und *BRAF* V600E Mutation Primärtumor; C: Patient mit hoher Tumorlast und BRAF Wildtyp im Primärtumor; D: Patient mit hoher Tumorlast und *BRAF* V600E Mutation Primärtumor).

Anhand der erfindungsgemäßen Korrelation der Methylierungsanalyse des *SHOX2* Genlokus mit der Gesamtmenge an frei zirkulierender genomischer DNA gemessen anhand des *BRAF* Genlokus wurde festgestellt, dass bei Patient A 1,7% DNA der malignen Erkrankung und bei Patient B 0,26% DNA der malignen Erkrankung im Plasma vorlagen. Dies entspricht einem relativ frühen Tumorstadium, bei dem im Allgemeinen nur eine geringe Menge an Tumor DNA im Blutplasma zu finden ist.

Bei der anschließenden Sanger-Sequenzierung der generierten *BRAF* Amplifikate konnte anschließend bei Patienten A und B nur wildtypische DNA detektiert werden, obwohl Patient B Mutationsträger ist (Figur 2, rechte Spalte). Isoliert betrachtet würde die Mutationsanalyse in der frei zirkulierenden genomischen DNA folglich für Patient B zu einem falsch-negativen Ergebnis führen. Dieser Befund konnte jedoch durch die erfindungsgemäße Kombination der Mutationsanalyse mit der Methylierungsanalyse auf die zu geringe Menge an DNA der malignen Erkrankung im Plasma zurückgeführt und somit zuverlässig als falsch-negativ identifiziert werden. Eine herkömmliche Mutationsanalyse hätte dagegen zu einer falsch-negativen Diagnose geführt.

Anders ist die Situation bei den Patienten C und D. In beiden Fällen konnte anhand der Methylierungsanalyse gezeigt werden, dass eine hohe Menge an frei-zirkulierender DNA der malignen Erkrankung im Blut vorhanden war (Figur 2, linke Spalte: Patient C: 26%; Patient D: 41%). Obgleich die Mutationsanalyse des Patienten C nur die wildtypische Sequenz zeigte, konnte mithilfe der erfindungsgemäß kombinierte Methylierungs- und Mutationsanalyse ausgeschlossen werden, dass es sich um einen falsch-negativen Befund handelt. Die maligne Erkrankung des Patienten C trägt demnach die *BRAF* V600E Mutation nicht. Diese richtig-negative Diagnose wäre mit einem herkömmlichen Verfahren allein über die Mutationsanalyse nicht von einem falsch-negativen Ergebnis zu unterscheiden gewesen.

Auf diese Weise erhöht das erfindungsgemäße Verfahren die Sensitivität molekulardiagnostischer Mutationsanalysen, da die Anzahl falsch-negativer Ergebnisse reduziert wird, insbesondere auf der Grundlage frei zirkulierender genomischer DNA, exosomaler DNA oder frei zirkulierender Zellen.

### Beispiel 4: Bestimmung einer Mutation, die zur Bildung eines CpGs führt

Der epidermale Wachstumsfaktor-Rezeptor (EGFR) ist eine Tyrosinkinase, die normalerweise durch die Bindung eines Liganden aktiviert wird. Durch Mutationen kommt es zu einer konstitutiven Aktivierung und somit zu einer erhöhten Zellproliferation. EGFR-Tyrosinkinaseinhibitoren (TKI) konkurrieren mit ATP um die Bindung in der Ligandenbindungstasche des Rezeptors und inhibieren auf diese Weise sowohl die Tyrosinkinaseaktivität als auch den EGFR-Signalweg. Bei Patienten mit aktivierenden Mutationen in *EGFR* gelten diese TKI als Frontline-Therapie und können das progressionsfreie Überleben der Patienten sowie die Ansprechraten auf eine Therapie verbessern. *EGFR* Mutationen sind daher starke prädiktive Marker für das Ansprechen auf EGFR Tyrosin-Kinase-Inhibitoren (TKI), wie zum Beispiel die TKIs der ersten Generation Erlotinib (Tarceva) und Gefitinib (Iressa) und TKIs der zweiten Generation wie zum Beispiel Afatinib (Gilotrif), Dacomitinib und Neratinib. TKIs der dritten Generation wie zum Beispiel CO-1686 (Rociletinib), AZD9291 und HM61713 sind zusätzlich so designt, dass sie das mutierte EGFR Protein stärker inhibieren als den EGFR Wildtyp. Aus diesen Gründen ist die Bestimmung des Mutationsstatus von *EGFR* von besonderer Wichtigkeit für eine personalisierte, zielgerichtete Therapie.

In Lungentumoren sind *EGFR* Mutationen sind besonders häufig bei Adenokarzinomen, Frauen und Nichtrauchern zu erwarten. Sie finden sich zu 9% in Exon 18, 51% in Exon 19, 18% in Exon 20 und 22% in Exon 21 des *EGFR*-Gens. Etwa 5% der *EGFR-*Mutationen führen zu einer sekundären Therapieresistenz auf EGFR-TKI. Dabei ist die Mutation c.2369C>T (T790M) der häufigste Mechanismus für eine EGFR-TKI Therapieresistenz.

In diesem Anwendungsbeispiel wurde die c.2573T>G (L858R) Mutation von *EGFR* untersucht. Sie befindet sich im Exon 21 innerhalb der Kinase-Domäne des *EGFR* Gens und tritt bei etwa 43% der EGFR-mutierten Lungentumoren auf.

Jeweils drei Dünnschnitte von je 10 *µ*m eines Formalin-fixierten und in Paraffin eingebetteten Lungentumors sowie ein Gewebestück angrenzenden normalen Lungengewebes wurden in ein 2 ml Reaktionsgefäß überführt. Mittels des QIAamp DNA FFPE Tissue Kit (Qiagen, Hilden) wurde entsprechend des Kithandbuchs genomische DNA aus den Gewebeschnitten extrahiert. Von der extrahierten DNA wurden pro Probe jeweils 2 *µ*g mittels des innuCONVERT Bisulfit All-In-One Kit (Analytik Jena, Jena, Deutschland) umgewandelt.

Der nicht-umgewandelte Teil der extrahierten DNA wurde für die Referenzanalyse verwendet. Dazu wurde die genomische nicht-umgewandelte DNA mithilfe von zwei Primern amplifiziert (Primersequenzen SEQ ID NO:13 und SEQ ID NO:14), welche den genomischen Lokus im Exon 21 des *EGFR* Gens amplifizieren, in dem die Mutation vermutet wurde. Das resultierende Amplifikat wurde anschließend in einer Sanger-Sequenzierung sequenziert und mit der nicht-umgewandelten wildtypischen Sequenz SEQ ID NO:15 verglichen, wobei der Vorwärts-Primer SEQ ID NO:13 als Sequenzierprimer verwendet wurde. Die PCR Amplifikation und die Sanger-Sequenzierung wurde wie in Beispiel 1 beschrieben durchgeführt.

Das Ergebnis der Referenzanalyse des *EGFR* Lokus in Exon 21 ist in Figur 3A und 3B zusammengefasst. Figur 3A zeigt die ermittelte Sequenz des gesunden, an den Tumor angrenzenden Gewebes. Figur 3B zeigt die ermittelte Sequenz des Tumorgewebes. Es wurde festgestellt, dass in der genomischen DNA des angrenzenden Normalgewebes keine Mutation zu finden ist. Dagegen ist in der Sequenz der genomischen DNA aus dem Tumor neben der wildtypischen Sequenz (CTG) auch die L858R Mutation (CGG) zu finden.

Für die erfindungsgemäße Mutationsanalyse wurde die umgewandelte genomische DNA mit den Primern der Sequenzen SEQ ID NO:16 und SEQ ID NO:17 amplifiziert, um eine Abweichung von der wildtypischen Sequenz des Bisulfit-I Strangs mit der Sequenz SEQ ID NO:18 zu bestimmen. Der Bisulfit-I Strang mit der Sequenz SEQ ID NO:18 entspricht der Sequenz, die aus einer Umwandlung des Plusstrangs der wildtypischen genomischen Sequenz SEQ ID NO:15 hervorgeht. Die entstehenden PCR-Produkte wurden anschließend mithilfe des Vorwärtsprimers SEQ ID NO:16 als Sequenzierprimer sequenziert. Die PCR-Amplifikation und die Sanger-Sequenzierung wurden wie in Beispiel 1 beschrieben durchgeführt.

Das Ergebnis des erfindungsgemäßen Verfahrens ist in Figur 3C und 3D gezeigt. Figur 3C zeigt die ermittelte Sequenz des gesunden Gewebes, Figur 3D die ermittelte Sequenz des Tumorgewebes. Es konnte festgestellt werden, dass das aus der Punktmutation resultierende CpG-Dinukleotid im Tumorgewebe methyliert vorliegt. Aufgrund seiner Methylierung wird das Cytosin in diesem CpG-Dinukleotid durch die Bisulfitbehandlung nicht umgewandelt. Dadurch unterscheidet sich die ermittelte Sequenz der umgewandelten Wildtyp-DNA (Figur 3C) vorteilhafterweise in einer zusätzlichen Base von der ermittelten Sequenz der umgewandelten Tumor-DNA (Figur 3D). Da in der genomischen nicht-umgewandelten DNA des Tumors dagegen nur eine Base unterschiedlich ist, lässt sich diese Art von Mutation mithilfe des erfindungsgemäßen Verfahrens deutlich besser nachweisen als in der herkömmlichen Mutationsanalyse. Dies führt zu einer überraschenden Verbesserung der Sensitivität und Spezifität der erfindungsgemäßen Mutationsanalyse auch bei geringem Anteil an DNA der malignen Erkrankung in der Probe.

### Beispiel 5: Bestimmung einer Deletion in genomischer DNA

Deletionen, die zu einem Verlust von Aminosäuren im Protein führen, sind weitere häufig zu findende Mutationen im *EGFR* Gen. Sie finden oftmals im Exon 19 statt, welches einen Teil der Kinase-Domäne kodiert. Diese Mutationen sind ebenfalls prädiktiv für das Therapieansprechen bei der Gabe von auf *EGFR* gerichtete TKIs. In etwa 48% der *EGFR*-mutierten Lungentumoren ist eine Deletion im Exon 19 zu finden.

In dieser Untersuchung war zu zeigen, dass Deletionen in umgewandelter genomischer DNA zuverlässig bestimmt werden können. Zu diesem Zweck wurden jeweils drei Dünnschnitte von je 10 *µ*m eines Formalin-fixierten und in Paraffin eingebetteten Lungentumors sowie ein Gewebestück angrenzenden normalen Lungengewebes in ein 2 ml Reaktionsgefäß überführt. Mittels des QIAamp DNA FFPE Tissue Kit (Qiagen, Hilden) wurde entsprechend des Kithandbuchs genomische DNA aus den Gewebeschnitten extrahiert. Von der extrahierten DNA wurden pro Probe jeweils 2 *µ*g DNA mittels des innuCONVERT Bisulfit All-In-One Kit (Analytik Jene, Jena, Deutschland) umgewandelt.

Von der extrahierten DNA wurde ein Teil nicht umgewandelt und für die Referenzanalyse verwendet. Zu diesem Zweck wurden die Primer SEQ ID NO:19 und SEQ ID NO:20 verwendet, welche dazu ausgelegt sind, den mutationstragenden genomischen Lokus im Exon 21 des *EGFR* Gens zu amplifizieren, um die resultierende Sequenz mit der nicht-umgewandelten wildtypischen Sequenz SEQ ID NO:21 zu vergleichen. Das resultierende Amplifikat wurde anschließend in einer Sanger-Sequenzierung sequenziert, wobei der Rückwärts-Primer SEQ ID NO:20 als Sequenzierprimer verwendet wurde. Die PCR Amplifikation und die Sanger-Sequenzierung wurde wie in Beispiel 1 beschrieben durchgeführt.

Das Ergebnis der Referenzanalyse ist in Figur 4 abgebildet. Figur 4A zeigt, dass in der genomischen nicht-umgewandelten DNA des angrenzenden Normalgewebes keine Mutation zu finden ist. Dagegen ist in der Sequenz der nicht-umgewandelten DNA aus dem Tumor ein Überlappung der wildtypischen Sequenz mit einer mutierten Sequenz, bei der 15 Basen deletiert sind, zu finden (Figur 4B). Durch diese Deletion kommt es zu einer Überlagerung der nachfolgenden Basen, die sich an die Deletion anschließen, mit der wildtypischen Sequenz.

Für das erfindungsgemäße Verfahren wurde die umgewandelte genomische DNA mit den Primern SEQ ID NO:22 und SEQ ID NO:23 amplifiziert, um eine Abweichung von der wildtypischen Sequenz des Bisulfit-I Strangs mit der Sequenz SEQ ID NO:24 zu bestimmen. Die SEQ ID NO:24 entspricht der Sequenz, die aus einer Umwandlung des Plusstrangs der wildtypischen genomischen Sequenz SEQ ID NO:21 hervorgeht. Die entstandenen Amplifikate wurden anschließend mittels des Rückwärts-Primers SEQ ID NO:23 sequenziert. Die PCR Amplifikation und die Sanger-Sequenzierung wurde wie in Beispiel 1 beschrieben durchgeführt.

Die Ergebnisse der Sequenzierung sind in Figur 4C für die umgewandelte genomische DNA des Normalgewebes und in Figur 4D für die umgewandelte genomische DNA des Tumors gezeigt. Es ist zu erkennen, dass die Deletion in einem Allel des Exon 19 zu einem vergleichbaren Überlagerungsmuster wie bei der Sequenzierung der genomischen nicht-umgewandelten Tumor DNA führt. Folglich sind auch Deletionen zuverlässig mit dem erfindungsgemäßen Verfahren zu bestimmen.

Um die erfindungsgemäße Bestimmung der Deletion in umgewandelter genomischer DNA weiter zu bestätigen, wurde das PCR-Produkt der mutationstragenden konvertierten DNA in ein Plasmid ligiert. Die Plasmide wurden in *E. coli* transfiziert und auf einer Agar Platte vereinzelt. Diese einzelnen Zellen wurden über Nacht bei 37 °C zu klonalen Kolonien herangezogen. Zellen einzelner *E. coli* Klone wurden in PCR Reaktionen überführt und anschließend sequenziert. Die Klonierung des PCR-Produkts erfolgte mittels des TOPO-TA Klonierungs-Kit (Life Technologies, Carlsbad, CA, USA) entsprechend der Herstellerangaben. Die PCR-Amplifizierung wurde wie in Beispiel 1 beschrieben durchgeführt. Dazu wurden die Primer SEQ ID NO:22 und SEQ ID NO:23 verwendet. Die Sequenzierung wurde wie in Beispiel 1 beschrieben durchgeführt. Als Sequenzierprimer diente SEQ ID NO:23. Figur 5A zeigt, dass ein Teil der umgewandelten genomischen DNA in der Tumor-DNA tragenden Probe wildtypisch ist, während das in Figur 5B gezeigte Allel eindeutig die Deletion von 15 Basen aufweist.

### Beispiel 6: Bestimmung einer Mutation mit Cytosin-Thymin Transition

In der herrschenden Lehrmeinung wurde bisher davon ausgegangen, dass Mutationen in umgewandelter genomischer DNA sehr schwer oder gar nicht detektierbar sind. Diese verbreitete Ansicht beruhte auch auf dem Umstand, dass unmethyliertes Cytosin zum Beispiel zu Uracil umgewandelt wird und sich Uracil in den Basenpaarungseigenschaften nicht von Thymin unterscheidet. In der Fachwelt galt es daher als unlösbares Problem, zwischen einem durch C nach T Mutation eingeführten Thymin und einem Thymin, welches durch Umwandlung von Cytosin eingeführt wird, zu differenzieren.

Diese falsche Vorstellung konnte im Rahmen der vorliegenden Erfindung überwunden werden. Durch erfinderisches Verständnis wurde erstmals erkannt, dass dieses Problem gelöst werden kann, wenn anstatt oder zusätzlich zum umgewandelten Plusstrang der genomischen Probe auch der umgewandelte Minusstrang der genomischen DNA von der erfindungsgemäßen Mutationsanalyse umfasst ist. Auf dem Minusstrang entspricht eine C nach T Transition des Plusstrangs einer G nach A Transition. Im vorliegenden Beispiel war daher anhand der Mutation c.437C>T (A146V, COSM1360827) im Exon 4 des *KRAS* Lokus zu zeigen, dass die G nach A Transition auf dem Minusstrang nach einer Umwandlung der genomischen DNA gut nachweisbar ist und somit eine zuverlässige Bestimmung einer Punktmutationen, welche allein auf einer C nach T Transition basiert, möglich ist.

Zu diesem Zweck wurden jeweils drei Dünnschnitte von je 10 *µ*m eines Formalin-fixierten und in Paraffin eingebetteten Adenokarzinoms des Dickdarms sowie ein Gewebestück angrenzenden normalen Darmgewebes in ein 2 ml Reaktionsgefäß überführt. Mittels des QIAamp DNA FFPE Tissue Kit (Qiagen, Hilden, Deutschland) wurde entsprechend des Kithandbuchs genomische DNA aus den Gewebeschnitten extrahiert. Von der extrahierten DNA wurden pro Probe jeweils 2 *µ*g DNA mittels des innuCONVERT Bisulfit All-In-One Kit (Analytik Jena, Jena, Deutschland) umgewandelt.

Jeweils ein weiterer Teil der extrahierten DNA wurde nicht umgewandelt und für die Referenzanalyse verwendet. Dazu wurde in einer PCR mithilfe der Primer SEQ ID NO:25 und SEQ ID NO:26 der potenziell mutationstragende genomische Lokus im Exon 4 des *KRAS* Gen amplifiziert, um Abweichungen von der nicht-umgewandelte wildtypischen DNA Sequenz SEQ ID NO:27 zu bestimmen. Das resultierende PCR-Produkt wurde anschließend in einer Sanger-Sequenzierung sequenziert, wobei der Vorwärtsprimer SEQ ID NO:25 als Sequenzierprimer verwendet wurde. Die PCR Amplifikation und die Sangersequenzierung wurde wie in Beispiel 1 beschrieben durchgeführt.

Figur 6A und 6B zeigen das Ergebnis der Referenzanalyse. Figur 6A gibt die Sequenz der nicht-umgewandelten genomischen DNA des gesunden, an den Tumor angrenzenden Gewebes nach Sequenzierung des Vorwärtsstrangs wieder. Figur 6B zeigt die Sequenz der nicht-umgewandelten genomischen DNA des Tumorgewebes nach Sequenzierung des Vorwärtsstrangs. Demzufolge ist in der nicht-umgewandelten genomischen DNA des angrenzenden Normalgewebes keine Mutation zu finden (6A). Dagegen tritt in der Sequenz der genomischen DNA aus dem Tumor neben der wildtypischen Sequenz auch eine mutierte Sequenz mit einer C nach T Transition auf (6B).

Im Rahmen der erfindungsgemäßen Verfahrensausführung wurde zunächst ein PCR-Amplifikation des entsprechenden Genlokus im Exon 4 des *KRAS* Gens mit den Primern mit den Sequenzen SEQ ID NO:29 und SEQ ID NO:30 durchgeführt, um eine Abweichung des Amplifikats von der Sequenz SEQ ID NO:28 zu bestimmen. Die Sequenz SEQ ID NO:28 entspricht der umgewandelten wildtypischen Sequenz des Plusstrangs mit der Sequenz SEQ ID NO:27. Die PCR Amplifikation wurde wie in Beispiel 1 beschrieben durchgeführt. Das Ergebnis der anschließenden entsprechend nach Beispiel 1 ausgeführten Sanger-Sequenzierung über den Vorwärtsprimer SEQ ID NO:29 ist in Figur 6C und 6D gezeigt. Figur 6C gibt die Sequenz der umgewandelten genomischen DNA des gesunden Gewebes nach Vorwärtssequenzierung des Bisulfit-I Strangs wieder, Figur 6D entsprechend die Sequenz der umgewandelten genomischen DNA des Tumorgewebes nach Vorwärtssequenzierung des Bisulfit-I Strangs. Das Ergebnis verdeutlicht, dass die C nach T Transition aufgrund der chemischen Umwandlung von C zu U in dem Bisulfit-I Strang nicht mehr nachweisbar ist. Dies beruht darauf, dass ein U in der umgewandelten genomischen DNA anschließend im Rahmen der PCR-Amplifikation wieder durch ein T ersetzt wird, so dass die ursprünglich mutierte Sequenz nicht mehr von der wildtypischen Sequenz unterscheidbar ist.

Anschließend wurde die Mutationsanalyse des entsprechenden Genlokus im Exon 4 des *KRAS* Gens basierend auf dem Bisulfit-II Strang durchgeführt, um eine Abweichung von der umgewandelten wildtypischen Sequenz SEQ ID NO:31 zu bestimmen. Für die PCR-Amplifikation auf der Grundlage des Bisulfit-II Strangs wurden die Primer SEQ ID NO:32 und SEQ ID NO:33 verwendet. Die Sequenzierung des PCR Produkts wurde mit dem Rückwärtsprimer SEQ ID NO:32 durchgeführt. Die Ergebnisse sind in Figur 6E-H gezeigt. Figuren 6E und 6F zeigen zunächst als Referenz die Wildtypvariante und die Mutation in der nicht-umgewandelten genomischen DNA nach Sequenzierung über den Rückwärtsprimer SEQ ID NO:26. Die Mutation ist in Figur 6F anhand des Auftretens einer teilweisen G nach A Transition erkennbar. Figuren 6G und 6H zeigen die ermittelten Sequenzen in der umgewandelten genomischen DNA des gesunden Gewebes (6G) beziehungsweise des Tumorgewebes (6H) jeweils nach der vorgehend beschriebenen Rückwärtssequenzierung des Bisulfit-II Strangs. Es ist deutlich zu erkennen, dass im Gegensatz zum gesunden Gewebe in dem Tumorgewebe neben der wildtypischen Sequenz auch die mutierte Sequenz mit der G nach A Transition auftritt. Diese G nach A Transition entspricht der gesuchten C nach T Transition auf dem Vorwärtsstrang.

Demzufolge erlaubt das erfindungsgemäße Verfahren entgegen falscher Vorstellungen in Fachkreisen generell eine zuverlässige Bestimmung von Mutationen, welche eine C nach T Transition umfassen oder aus dieser bestehen.

In bestimmten Varianten des erfindungsgemäßen Verfahrens umfasst die Mutationsanalyse daher ein Bestimmen der Mutation auf der Grundlage des umgewandelten Minusstrangs der genomischen DNA.

### Beispiel 7: Multiplexierte Bestimmung von Mutationen in genomischer DNA aus Körperflüssigkeiten und Geweben

In einer bevorzugten Anwendung der Erfindung ist vorgesehen, eine kombinierte Mutations- und Methylierungsanalyse durchzuführen, welche mehrere Gene aus verschiedenen Bereichen des Genoms umfasst, nachfolgend auch als genomweite Mutations- und Methylierungsanalyse bezeichnet.

Vorzugsweise wird die genomweite Mutations- und Methylierungsanalyse mit frei zirkulierender genomischer DNA aus Körperflüssigkeiten durchgeführt. Besonders bieten sich Blutplasma, Blutserum, Urin, Aszites und Pleuraergüsse an. Die Umwandlung der genomischen DNA aus Körperflüssigkeiten kann beispielsweise mittels des innuCONVERT Bisulfit Body Fluids Kits (Analytik Jene, Jena, Deutschland) entsprechend des Kit-Handbuchs erfolgen.

Im Anschluss ist eine kombinierte Mutationsanalyse und Methylierungsanalyse mithilfe einer geeigneten genomweiten Sequenzierungsmethode vorgesehen. In bevorzugten Varianten wird eine Hochdurchsatz-Sequenzierungsmethode wie zum Beispiel eine Next Generation Sequencing Methode verwendet. Besonders bevorzugt wird die Whole Genome Shotgun Bisulfite Sequencing (WGSBS) Methode verwendet. Auf diese Weise lassen sich eine Vielzahl von Mutationen beziehungsweise CpG-Methylierungszuständen nebeneinander bestimmen. Sofern die Bestimmung jeweils quantitativ erfolgt, können die ermittelten Quantitäten anschließend zu den entsprechenden wildtypischen Sequenzen beziehungsweise Methylierungszuständen innerhalb der, gegebenenfalls umgewandelten, genomischen DNA in Beziehung gesetzt werden. Auf diese Weise kann zum Beispiel der relative Anteil an mutationstragender DNA und/oder DNA einer malignen Erkrankung (anhand des Methylierungszustands) innerhalb der genomischen DNA ermittelt werden. Es ist auf diese Weise auch möglich, denjenigen Anteil der DNA einer malignen Erkrankung zu bestimmen, welcher eine Mutation trägt. Dies kann besonders vorteilhaft sein, da maligne Erkrankungen wie zum Beispiel Tumoren oft eine heterogene genetische Zusammensetzung aufweisen oder sich zum Beispiel der relative Anteil mutationstragender DNA in der genomischen DNA einer malignen Erkrankung durch beispielsweise eine Therapie verändern kann und Rückschlüsse auf den Therapie- und/oder Krankheitsverlauf zulässt.

In einer weiteren Ausgestaltung ist vorgesehen, dass die Mutations- beziehungsweise Methylierungsanalyse der umgewandelten genomischen DNA aus Körperflüssigkeiten nicht direkt mittels einer genomweiten Sequenzierungsmethode wie oben beschrieben durchgeführt werden. In diesem Fall ist vorgesehen, zunächst die zu untersuchenden Bereiche der umgewandelten genomischen DNA zu amplifizieren. In einer bevorzugten Anwendung werden diese Bereiche mithilfe einer PCR amplifiziert. Die Primerpaare werden dazu derart ausgelegt, dass sie jeweils ein Amplifikat aus der umgewandelten genomischen DNA erzeugen, welches eine oder mehrere zu analysierenden Mutationsstellen oder CpG-Dinukleotide umfasst. Vorzugsweise werden die Primerpaare auch derart ausgelegt, dass sie mit einer Multiplex-PCR kompatibel sind, in der eine Vielzahl von Primerpaaren zur gleichzeitigen Amplifikation einer Vielzahl von zu untersuchenden Bereichen der umgewandelten genomischen DNA eingesetzt werden. Anschließend erfolgt die Untersuchung der Vielzahl von PCR-Amplifikaten beispielsweise mittels Next Generation Sequencing.

In einer weiteren bevorzugten Ausgestaltung erfolgt die Analyse über eine multiplexierte, ligationsabhängige Sondenamplifikation (MLPA). Die für die MLPA verwendeten Sonden werden dazu in einer Weise ausgelegt, dass sie an den zu bestimmenden Mutationsstellen beziehungsweise Methylierungsstellen binden und beispielsweise bei Vorliegen einer Mutation oder einer Methylierung eines CpG-Dinukleotids ligiert werden. Im Anschluss können die ligierten Sonden zum Beispiel mithilfe einer PCR amplifiziert und gegebenenfalls sequenziert werden.

In einer weiteren bevorzugten Ausführungsform wird DNA aus frischen oder fixierten Geweben oder frei-zirkulierenden Tumorzellen anstelle von frei zirkulierender DNA analysiert.

### Beispiel 8: Prädiktion durch kombinierte Mutations- und Methylierungsanalyse innerhalb eines Gens

Im Rahmen der vorliegenden Erfindung wurde erkannt, dass ein Ansprechen eines Patienten mit einer malignen Erkrankungen auf eine Therapie wie zum Beispiel eine Chemotherapie davon abhängen kann, ob zum Beispiel bestimmte DNA-Reparaturenzyme in dem Gewebe der malignen Erkrankung aktiv oder inaktiv sind. Eine Inaktivierung kann beispielsweise aufgrund des Methylierungszustands oder auch einer inaktivierenden Mutation des entsprechenden, das Reparaturenzym codierenden Gens in der genomischen DNA der malignen Erkrankung vorliegen. In Hinblick auf eine zielgerichtetere Therapie kann es daher vorteilhaft sein, dasselbe Gen sowohl in Bezug auf seinen Methylierungszustand als auch auf das Vorliegen einer Mutation hin zu untersuchen.

Eine derartige Vorgehensweise sieht die Erfindung zum Beispiel in Bezug auf die Therapie mit PARP Inhibitoren vor. PARP Inhibitoren sind eine Gruppe von pharmakologischen Substanzen, die das Enzym poly-ADP-Ribose-Polymerase (PARP) inhibieren. Dieses Enzym ist für die Reparatur von Einzelstrangbrüchen in der DNA wichtig. Werden diese Einzelstrangbrüche nicht effizient repariert, dann können diese zu Doppelstrangbrüchen führen, welche wiederum bei ebenfalls ausbleibender Reparatur zum Zelltod führen können. Dies ist zum Beispiel bei einer Chemotherapie und/oder Strahlentherapie von Tumoren so gewollt. Vorzugsweise erfolgt die erfindungsgemäße Prädiktion in Bezug auf die PARP Inhibitoren, Talazoparib (BMN-673), Olaparib (AZD-2281), Rucaparib (AG014699, PF-01367338), Veliparib (ABT-888), CEP 9722, MK 4827 und/oder BGB-290.

*BRCA1, BRCA2* und *PALB2* kodieren Reparaturenzyme, welche solche resultierenden Doppelstrangbrüche durch homologe Rekombination reparieren. Wenn diese Gene aktiv und funktionsfähig sind, dann können die durch eine PARP Inhibition resultierenden Einzel- und Doppelstrangbrüche effizient repariert werden. Die Tumorzelle kann überleben. Die Therapie mit PARP Inhibitoren wirkt in diesem Fall schlecht.

Bei einigen Tumoren ist die Funktion von *BRCA1, BRCA2* und/oder *PALB2* jedoch eingeschränkt, da die entsprechenden Gene zum Beispiel eine Keimbahnmutation oder eine somatische Mutation tragen oder/und die Gene durch Methylierung inaktiviert sind. In diesen Fällen können die aus der PARP Inhibition resultierenden DNA Schäden nicht repariert werden und die Tumorzelle stirbt. Tumorzellen, bei denen *BRCA1, BRCA2* oder *PALB2* durch Mutation und/oder Methylierung inaktiviert sind, sprechen daher gut auf die Behandlung mit PARP Inhibitoren an.

Es ist vorgesehen, mit Hilfe des erfindungsgemäßen Verfahrens Tumoren dahingehend zu testen, ob diese aufgrund einer Inaktivierung von DNA-Reparaturenzymen durch Methylierung und/oder Mutation des entsprechenden Gens voraussichtlich auf eine Monotherapie oder Kombinationstherapie mit PARP Inhibitoren ansprechen. Für eine Kombinationstherapie kommen dabei beispielsweise, aber nicht ausschließlich, eine Chemotherapie mit Cisplatin, eine Bestrahlung oder weitere Therapien in Frage.

Zur Durchführung des erfindungsgemäßen Verfahrens wird zum Beispiel ein Hämatoxylin-Eosin-Schnitt (H&E-Schnitt) des Formalin-fixierten und in Paraffin eingebetteten Tumors angefertigt. Gleichzeitig werden weitere ungefärbte Leerschnitte von etwa 10 *µ*m angefertigt und auf Glasobjektträger aufgezogen. Durch einen Pathologen wird der Tumor-tragende Bereich des Gewebes auf dem H&E-Schnitt markiert. Das entsprechende Areal der aufgezogenen Leerschnitte wird dann mittels eines Skalpells in ein 2 ml Reaktionsgefäß überführt. Idealerweise wird ein Areal, welches insgesamt etwa 1 bis 3 cm² misst, verwendet. Dieses Areal kann auch aus mehreren Leerschnitten gewonnen werden.

Das Gewebe kann anschließend deparaffiniert und zum Beispiel mittels Proteinase K lysiert werden. Das lysierte Gewebe kann direkt ohne vorhergehende Extraktion beispielsweise in eine Bisulfit-Konversionsreaktion zur Umwandlung der genomischen DNA überführt werden. Alternativ kann die genomische DNA auch vor der Umwandlung aus dem lysierten Gewebe extrahiert werden. Nach der Umwandlung erfolgt eine Aufreinigung der umgewandelten genomischen DNA. Hierfür eignen sich zum Beispiel Silicamembran-Säulen. Die Lyse des Gewebes, die Bisulfit-Konversion der DNA und die anschließende Aufreinigung kann zum Beispiel mit dem innuConvert All-In-One Kit (Analytik Jena, Jena, Deutschland) entsprechend der im Kit enthaltenen Protokolle durchgeführt werden.

Die erfindungsgemäße Bestimmung der Mutation und des Methylierungszustands kann anschließend über verschiedene Verfahrensvarianten durchgeführt werden.

Es ist zum Beispiel möglich, eine multiplex-PCR Amplifikation der umgewandelten genomischen DNA mit Primerpaaren durchzuführen, welche geeignet sind, mindestens einen ersten Teilbereich der umgewandelten genomischen DNA zu amplifizieren, welcher im Verdacht steht, eine Mutation zu enthalten, und einen zweiten Teilbereich, welcher ein CpG-Dinukleotid beinhaltet, dessen Methylierungszustand zu analysieren ist.

Für die Methylierungsanalyse von *BRCA1* werden Primer verwendet, die vorzugsweise, aber nicht ausschließlich, dazu ausgelegt sind, zumindest teilweise in der genomischen Sequenz SEQ ID NO:61 binden, nachdem diese Sequenz umgewandelt wurde. Beispielsweise kann sich das Primerpaar mit SEQ ID NO:86 und SEQ ID NO:87 beziehungsweise das Primerpaar mit SEQ ID NO:88 und SEQ ID NO:89 eignen.
Für die Methylierungsanalyse von *BRCA2* werden Primer verwendet, die vorzugsweise, aber nicht ausschließlich, dazu ausgelegt sind, zumindest teilweise in der genomischen Sequenz SEQ ID NO:39 binden, nachdem diese Sequenz umgewandelt wurde.

Für die Mutationsanalyse von *BRCA1* werden vorzugsweise ein oder mehrere Primerpaare verwendet, deren Zielsequenzen zumindest teilweise in Bereichen der umgewandelten genomischen DNA liegen, welche im nicht-umgewandelten wildtypischen Zustand mindestens 95% Sequenzidentität mit einer der Sequenzen ausgewählt aus SEQ ID NO:46 bis SEQ ID NO:63 oder Kombinationen davon hatten, insbesondere umfassend SEQ ID NO:56.

Für die Mutationsanalyse von *BRCA2* werden vorzugsweise ein oder mehrere Primerpaare verwendet, deren Zielsequenzen zumindest teilweise in Bereichen der umgewandelten genomischen DNA liegen, welche im nicht-umgewandelten wildtypischen Zustand mindestens 95% Sequenzidentität mit einer der Sequenzen ausgewählt aus SEQ ID NO:34 bis SEQ ID NO:45 oder Kombinationen davon hatten. Des Weiteren werden besonders bevorzugt ein oder mehrere Primerpaare verwendet, deren Zielsequenzen in Bereichen der umgewandelten genomischen DNA des *PALB2* Genlokus liegen.

Es ist möglich, die Produkte der PCR Amplifikation anschließend zum Beispiel über NGS zu sequenzieren, wobei die Bestimmung des Methylierungszustands beziehungsweise der An- oder Abwesenheit der Mutation vorzugsweise quantitativ erfolgt. Auf diese Weise kann zum Beispiel über den quantifizierten Methylierungszustand und/oder das Verhältnis von umgewandelter genomischer DNA mit und ohne Mutation der prozentuale Anteil methylierter und mutierter Allele bestimmt werden.

Die Mutations- und Methylierungsanalyse kann auch über MLPA oder WGSBS analog der Beschreibung in Beispiel 7 oder geeigneten Variationen dieser Verfahren erfolgen.

In einer anderen Ausgestaltung wird der Formalin-fixierte Tumor nicht makrodisseziert. Beispielsweise können Schnitte von jeweils 10 *µ*m eines tumortragenden Gewebeblocks direkt in ein 2 ml Reaktionsgefäß übertragen werden. Die anschließende Lyse, Umwandlung und Aufreinigung kann wie oben beschrieben durchgeführt werden.

Die Anwendung dieses Verfahrens bietet sich besonders für Erkrankungen an, bei denen das Vorliegen von *BRCA1* und *BRCA2* Mutationen eine wichtige Rolle spielt, wie zum Beispiel Brust- und Eierstockkrebs, sowie Melanomen und Prostatakrebs.

### Beispiel 9: Prädiktion durch kombinierte Mutations- und Methylierungsanalyse von verschiedenen Genen

Während im vorherigen Beispiel insbesondere eine kombinierte Mutations- und Methylierungsanalyse innerhalb des gleichen Gens eine Vorhersage hinsichtlich des Ansprechens auf eine Therapie erlaubt, ist im Rahmen der Erfindung auch erkannt worden, dass bei bestimmten malignen Erkrankungen eine kombinierte Bestimmung einer Mutation beziehungsweise eines Methylierungszustands von verschiedenen Genen eine besonders vorteilhafte Prädiktion gewährleisten kann.

Ein Beispiel betrifft die Behandlung mit Temozolomid. Temozolomid ist ein alkylierendes Zytostatikum, welches zur simultanen, adjuvanten und palliativen Therapie von Glioblastomen in Kombination mit Strahlentherapie eingesetzt wird. Temozolomid führt zu einer DNA Schädigung, indem die DNA alkyliert wird. Diese DNA Schäden können die Tumorzellen zu Apoptose bewegen und diese so abtöten.

Das Enzym O6-Methylguanin-DNA-Methyltransferase (MGMT) ist an der Reparatur alkylierter DNA beteiligt. Dieses Enzym kann folglich die Wirkung von Temozolomid verringern. MGMT wird in einigen Glioblastomen durch die Methylierung des Gens reprimiert. Patienten, deren Tumoren eine herabgesetzte Expression von MGMT aufweisen, sprechen daher gut auf eine Behandlung mit Temozolomid an. Der Methylierungszustand von *MGMT* ist daher ein prädiktiver Biomarker für das Ansprechen auf eine Temozolomid-Behandlung.

Inzwischen ist bekannt, dass neben der Methylierung von *MGMT* auch einige Mutationen, beispielsweise Mutationen der Gene *IDH1* und *IDH2* sowie Amplifikation von *EGFR,* das Ansprechen auf eine Behandlung mit Temozolomid vorhersagen können. Dazu ist die Anwendung von Medikamenten, die spezifisch die mutierte Variante von *IDH1* und *IDH2* inhibieren, ein vielversprechender Therapieansatz. Tumoren, die eine Amplifikation von *EGFR* aufweisen, können auf eine Behandlung mit gegen EGFR gerichteten therapeutischen monoklonalen Antikörpern ansprechen. Tumoren, die Mutationen in *IDH1* oder *IDH2* aufweisen, sprechen gut auf eine Therapie mit Wirkstoffen an, die die mutierten Varianten inhibieren. Die kombinierte Methylierungsanalyse von *MGMT* und Mutationsanalyse von *IDH1, IDH2* und/oder *EGFR* in umgewandelter genomischer DNA aus Glioblastomen oder anderen Tumoren, bei denen *IDH1, IDH2* und/oder *EGFR* Mutationen vorliegen, ist daher eine klinisch relevante Anwendung der vorliegenden Erfindung. Insbesondere umfasst das erfindungsgemäße Verfahren eine Methylierungsanalyse des *MGMT* Gens, vorzugsweise umfassend zumindest einen Teil einer Sequenz, welche im nicht-umgewandelten wildtypischen Zustand mindestens 95% Sequenzidentität mit SEQ ID NO:92 aufweist, und/oder eine Mutationsanalyse von *IDH1* und/oder *IDH2.* Insbesondere kann die Mutationsanalyse dazu ausgelegt sein, eine Mutation in zumindest einem Teil einer Sequenz zu bestimmen, welche im nicht-umgewandelten wildtypischen Zustand mindestens 95% Sequenzidentität mit SEQ ID NO:84 und/oder SEQ ID NO:85 aufweist.

### Beispiel 10: Verwendung des Verfahrens zur Prognose und/oder Prädiktion

Die Messung von prognostischen Biomarkern ermöglicht die Bestimmung der Agressivität einer malignen Erkrankung, beispielsweise eines Tumors.

Es gehört zu den Alleinstellungsmerkmalen der vorliegenden Erfindung, dass durch die simultane Mutations- und Methylierungsanalyse ein molekulardiagnostisches Gesamtbild geschaffen werden kann, in welches prognostische Biomarker wie zum Beispiel der Methylierungszustand eines Gens und prädiktive Biomarker wie zum Beispiel eine Mutation in einem therapeutisch relevanten Gen gleichermaßen einfließen. Auf diese Weise kann erstmals innerhalb einer einzigen Analyse ein funktioneller Zusammenhang zwischen solchen prognostischen und prädiktiven genetischen Dispositionen hergestellt werden, um Patienten auf diese Weise zielgerichteter und gegebenenfalls dynamischer zu therapieren.

In einer weiteren Anwendung dieser Erfindung werden daher die Methylierungsanalyse eines Gens als prognostischer Biomarker und die Mutationsanalyse eines therapeutisch relevanten Gens als prädiktiver Biomarker einer malignen Erkrankung in einer Analyse zusammengeführt.

Für diese Art der Analyse ist insbesondere vorgesehen, dass die Methylierungsanalyse zumindest einen Teil oder mehrere Teile der prognostischen DNA Methylierungsbiomarker *PITX2,* vorzugsweise zumindest einen Teil einer Sequenz, welche im nicht-umgewandelten wildtypischen Zustand mindestens 95% Sequenzidentität mit SEQ ID NO:90 und/oder SEQ ID NO:91 aufweist, *CDO1, PLAU, POU4F3, TFF1, CXCL12* oder Kombinationen davon umfasst.

### Beispiel 11: Bestimmung des Anteils an DNA einer malignen Erkrankung in genomischer DNA durch kombinierte Mutationsanalyse und Methylierungsanalyse

Im Rahmen der vorliegenden Erfindung wurde auch das Problem erkannt, dass einige maligne Erkrankungen eine insgesamt geringe Methylierung an vielen Genen aufweisen, welche üblicherweise in malignen Erkrankungen aberrant hypermethyliert sind. In solchen Fällen kann es problematisch sein, den Anteil der DNA der malignen Erkrankung in einer Probe genomischer DNA allein anhand der Methylierungsanalyse zu bestimmen, denn es kann DNA der malignen Erkrankung vorliegen, obwohl nur wenig oder keine DNA Methylierung nachgewiesen wird. Auf diese Weise können falsch-negative Diagnosen resultieren.

Diese Problematik wird in einer weiteren Ausgestaltung der Erfindung gelöst, indem die Mutationsanalyse ein Bestimmen von mindestens einer rekurrenten Mutation oder von mindestens einer Mutation in einem rekurrent mutierten Gen umfasst. Insbesondere kann der Anteil der DNA der malignen Erkrankung in der genomischen DNA anhand der mindestens einen rekurrenten Mutation oder mindestens einer Mutation in einem rekurrent mutierten Gen bestimmt werden. Vorzugsweise wird dafür die Mutationsanalyse in Schritt B) unter Bedingungen durchgeführt, welche eine quantitative Bestimmung der mindestens einen rekurrenten Mutation oder der mindestens einen Mutation des rekurrent mutierten Gen erlauben, um den Anteil an DNA der malignen Erkrankung in der genomischen DNA zu quantifizieren.

Soll beispielsweise im Plasma eines Patienten mit Darmkrebs eine erfindungsgemäße Mutationsanalyse von *EGFR* oder *KRAS* durchgeführt werden, dann bietet sich zusätzlich eine Bestimmung weiterer Mutationen an, die bei Darmkrebs sehr häufig sind, um diese alleine oder zusammen mit der Methylierungsanalyse zur Bestimmung des Anteils der DNA der malignen Erkrankung in der genomischen DNA zu verwenden. Bei Darmkrebs kann die Mutationsanalyse zum Beispiel *TP53* und/oder *APC* umfassen, um eine Mutation eines rekurrent mutierten Gens zu bestimmen. Der Anteil an DNA der malignen Erkrankung in der genomischen DNA kann dann allein anhand der Mutation des rekurrent mutierten Gens oder in Kombination mit der Methylierungsanalyse bestimmt werden, um auf diese Weise ein besonders robustes Ergebnis zu erhalten.

Die Bestimmung der rekurrenten Mutation oder der Mutation in dem rekurrent mutierten Gen kann insbesondere davon abhängig gemacht werden, welches Organ beziehungsweise welcher Gewebetyp von der malignen Erkrankung betroffen ist. Nachfolgend sind verschiedene Organ- und Gewebetypen zusammen mit den jeweiligen Genen aufgeführt, welche bevorzugt zumindest teilweise von der Mutationsanalyse zur Bestimmung einer rekurrenten Mutation oder einer Mutation eines rekurrent mutierten Gens umfasst sind. Die Mutationsanalyse kann auch Kombinationen dieser Gene umfassen, um eine oder mehrere rekurrente Mutationen oder eine oder mehrere Mutationen rekurrent mutierter Gene zu bestimmen.

Vulva: *TP53, CDKN2A.* Vagina: *TP53.* Harnwege: *TERT, FGFR3, TP53, STAG2, KDM6A, PIK3CA, CDKN2A, ARID1A, RB1.* Oberer Aeorodigestivtrakt: *TP53, CDKN2A, NOTCH1.* Schilddrüse: *BRAF, RET, TSHR, TERT, NRAS.* Thymus: *ALK*, *TP53, KIT, MEN1, CDKN2A, RET.* Hoden: *CTNNB1, KIT, TP53, KRAS.* Magen: *TP53, ARID1A, CDH1, APC, PIK3CA, KMT2C, TRRAP, CTNNB1.* Weichteile: *KIT, CTNNB1, MED12, NF2, SMARCB1, NF1, PDGFRA, TP53, TERT, CDKN2A, VHL.* Dünndarm: *PDGFRA, KRAS, TP53, CTNNB1, APC, MEN1, SMAD4, GNAS.* Haut: *BRAF, TP53, TERT, CDKN2A, GRIN2A, PTCH1, NRAS, ROS1, FGFR3, KMT2C, HRAS.* Speicheldrüse: *TP53, HRAS, PIK3CA, CDKN2A, CREBBP, KDM6A, CTNNB1.* Prostata: *TP53, PTEN, SPOP, KRAS.* Pleura: *DICER1, CDKN2A, BAP1, NF2, TERT, TP53.* Peritoneum: *GNAS, KRAS, TP53, EGFR, SMAD4.* Pankreas: *KRAS, TP53, GNAS, SMAD4, CDKN2A.* Eierstock: *TP53, FOXL2, KRAS, PIK3CA, ARID1A, BRAF.* Ösophagus: *TP53, CDKN2A, NOTCH1.* Nervensystem: *BRAF, TERT, NRAS, CDKN2A.* Lunge: *TP53, EGFR, KRAS.* Leber: *TP53, CTNNB1, TERT.* Dickdarm: *APC, TP53, KRAS, ATM, PIK3CA, SMAD4, BRAF.* Niere: *VHL, PBRM1, BAP1, SETD2, CTNNB1, AMER1, TP53, WT1.* Leukämien und Lymphome: *JAK2, NPM1, FLT3, MYD88, KIT, CALR, ABL1, TET2, NOTCH1, DNMT3A, ASXL1.* Endometrium: *PTEN, PIK3CA, CTNNB1, TP53, PIK3R1, ARID1A, KRAS.* Gebärmutterhals: *PIK3CA, KMT2C, KMT2D, KRAS.* Zentrales Nervensystem: *IDH1, TERT, TP53, CDKN2A, PTEN, H3F3A.* Brust: *PIK3CA, TP53, CDH1.* Knochen: *IDH1, GNAS, TP53, H3F3B, COL2A1.* Galle: *TP53, KRAS, CDKN2A, KMT2C, IDH1, ARID1A.* Nebennniere: *KCNJ5, TP53, CTNNB1, NF1.*

### Beispiel 12: Nachweis von zirkulierenden Tumorzellen (CTCs)

Die Analyse von zirkulierenden Tumorzellen (CTCs) beinhaltet ein großes Potenzial für die Verbesserung der Behandlung von Patienten mit malignen Erkrankungen. Beispielsweise kann das Vorliegen von CTCs im Blut die Bestimmung des Tumorstadiums ermöglichen, da es bereits frühzeitig auf eine okkulte Fernmetastasierung hinweist. Eine molekulare Analyse von CTCs kann auch eine Aussage über das potenzielle Ansprechen dieser Zellen auf bestimmte Therapien ermöglichen. Liegen beispielsweise in den CTCs von Melanom-Patienten die *BRAF* Mutation V600E vor, dann ist ein Ansprechen dieser Zellen auf eine Behandlung mit Vermurafenib wahrscheinlich.

Der spezifische Nachweis von CTCs ist allerdings problematisch. Herkömmliche Verfahren basieren zum Beispiel auf der Anreicherung von CTCs anhand von epithelialen Oberflächenmarkern. Das epitheliale Zelladhäsionsmolekül EpCAM beispielsweise weist darauf hin, dass die Zelle eine Tumorzelle sein könnte, da diese Zelle epitheliale Charakteristika aufweist, die von anderen zirkulierenden Zellen, die meist hämatopoetischen Ursprungs sind, nicht gezeigt werden. Mithilfe von Antikörpern gegen EpCAM können diese Zellen spezifisch markiert und zum Beispiel über Magnetpartikel aufgereinigt oder bereits im Körper mit einem Katheter, der auf der Oberfläche Antikörper gegen EpCAM trägt, angereichert werden. Dieses Verfahren weist zwei gravierende Probleme auf. Erstens tragen nicht alle Tumorzellen die entsprechenden Proteine wie zum Beispiel EpCAM auf der Oberfläche, so dass die Methode gerade bei geringen Mengen CTCs oft nicht genügend empfindlich für den Nachweis ist. Zweitens finden sich vereinzelt zirkulierenden Zellen, die Charakteristika von Epithelzellen tragen, dabei jedoch nicht tumorösen Ursprungs sind, so dass es der Methode auch an Spezifität mangelt.

Andere bekannte Methoden zur Anreicherung von CTCs basieren auf einer Größenselektion. CTCs sind größer als die meisten anderen zirkulierenden Zellen und können so über Poren einer geeigneten Größe angereichert werden. Auch hier besteht das Problem der Spezifität, da auch andere Zellen nicht-tumorösen Ursprungs angereichert werden können.

Wird nun in den vermeintlich angereicherten CTCs aus den herkömmlichen Verfahren eine Mutationsanalyse durchgeführt, so kann es zu falsch-negativen Ergebnissen kommen, da statt der CTCs zumindest teilweise auch unspezifisch angereicherte gutartige Zellen analysiert werden.

Das erfindungsgemäße Verfahren löst dieses Problem zum Beispiel durch eine kombinierte Mutations- und Methylierungsanalyse in umgewandelter genomischer DNA frei-zirkulierender Zellen. Es ist dann möglich, einen malignen Ursprung der isolierten Zellen anhand der Methylierungsanalyse nachzuwiesen. Auf diese Weise kann die Spezifität der Mutationsanalyse signifikant verbessert werden.

### Beispiel 13: Normalisierte Methylierungsanalyse

In den vorhergehenden Beispielen wurde bereits gezeigt, dass mithilfe des erfindungsgemäßen Verfahrens ein normalisierter Nachweis von Mutationen durch Korrelieren der Mutationsanalyse mit der Methylierungsanalyse möglich ist. Auf diese Weise kann zum Beispiel der Anteil an DNA einer malignen Erkrankung in einer Probe mit genomischer DNA über die Methylierungsanalyse bestimmt werden, um zu vermeiden, dass die Abwesenheit von DNA der malignen Erkrankung in der Probe zu einer falsch-negativen Mutationsanalyse führt. Dadurch wird erfindungsgemäß zum Beispiel eine erhebliche Verbesserung der Sensitivität der Mutationsbestimmung erreicht.

Aber auch der umgekehrte Fall, das heißt eine Normalisierung der Methylierungsanalyse anhand der Mutationsanalyse, ist mit besonderen erfindungsgemäßen Vorteilen verbunden.

Eine maligne Erkrankung, zum Beispiel ein Tumor, ist in der Regel heterogen, das heißt es können verschiedene Subtypen von Zellen vorhanden sein, die mitunter unterschiedliche Eigenschaften haben. Wirkt beispielsweise auf den Tumor eine Therapie, zum Beispiel eine Chemotherapie, eine gerichtete Therapie oder eine Immuntherapie, so sprechen nicht alle Zellen in der gleichen Weise an und oftmals überlebt eine Population von Tumorzellen. Das kann zum Beispiel ein bestimmter Subtyp sein.

Die erfindungsgemäße Methylierungsanalyse ist besonders geeignet, solche Subtypen zu identifizieren. Methylierungsbiomarker sind oftmals nicht in allen Tumorzellen methyliert. Daher ermöglicht die Analyse von Methylierungsbiomarkern die Identifizierung von Subtypen von Zellen, die eine bestimmte Eigenschaft besitzen. Dies kann, wie bereits in den vorhergehenden Beispielen ausgeführt wurde, beispielsweise ein bestimmtes Ansprechen auf eine bestimmte Therapie oder eine bestimmte Aggressivität umfassen.

Wird im Rahmen des erfindungsgemäßen Verfahrens die umgewandelte genomische DNA hinsichtlich solcher prädiktiver und prognostischer Methylierungsbiomarker untersucht, dann ist die Frage von Interesse, wie hoch der Anteil der DNA der malignen Erkrankung in der Probe ist. Umfasst beispielsweise die Methylierungsanalyse *MGMT* in einer Probe mit genomischer DNA eines Glioblastoms um zum Beispiel ein Ansprechen auf Temozolomid vorauszusagen, dann kann von Bedeutung sein, bei wieviel Prozent der Tumorzellen eine *MGMT* Methylierung vorliegt.

Trägt ein Tumor beispielsweise eine Mutation, die mit zur Tumorentstehung geführt hat, dann ist diese klonal in allen Tumorzellen enthalten und das erfindungsgemäße Verfahren ermöglicht die Bestimmung des Anteils an Tumorzellen anhand der Mutationsanalyse. In Bezug auf das Beispiel des Glioblastoms kann beispielsweise eine Mutation von *IDH1* oder *IDH2* bestimmt werden. Anschließend kann ein Verhältnis aus dem Anteil der genomischen DNA, in welcher die Methylierung nachgewiesen wurde, und dem Anteil der genomischen DNA, in welcher die Mutation nachgewiesen wurde, gebildet werden. Dieses Verhältnis kann als Maß für den Anteil des gesuchten Subtyps von Zellen in der malignen Erkrankung dienen. Beispielsweise kann der Anteil von MGMT-methylierten Zellen des Glioblastoms bestimmt werden, welche voraussichtlich auf die Therapie ansprechen.

*PITX2* ist beispielsweise ein sehr leistungsstarker prognostischer Methylierungsmarker für Brust-, Prostata-, Lungen-, und Kopf- und Hals-Tumoren. Hierbei ist die relative Menge an *PITX2* Methylierung im Gewebe prognostisch. In bestimmten Ausgestaltungen umfasst die Methylierungsanalyse des erfindungsgemäßen Verfahrens daher den Methylierungszustand eines oder mehrerer CpG-Dinukleotide in *PITX2.* Vorzugsweise umfasst die Mutationsanalyse in dieser Ausgestaltung die Bestimmung von mindestens einer rekurrenten Mutation oder einer Mutation eines rekurrent mutierten Gens wie beispielsweise in Beispiel 11 beschrieben wurde. Besonders bevorzugt umfasst die Mutationsanalyse zumindest einen Teil von *TP53.*

Eine Normalisierung der *PITX2* Methylierung anhand von rekurrenten Mutationen wie zum Beispiel Mutationen von *TP53* ermöglicht erfindungsgemäß eine genauere Bestimmung von zum Beispiel dem Anteil methylierter Tumor-DNA an der Gesamtheit der Tumor-DNA und damit beispielsweise auch eine differenziertere Diagnose, Prognose und Prädiktion von malignen Erkrankungen.

## Patentansprüche

1. Ein Verfahren zur Diagnose, Prognose, Prädiktion und/oder Verlaufskontrolle einer malignen Erkrankung, umfassend eine Bestimmung mindestens einer Mutation in genomischer DNA, mit den Schritten
A) Umwandeln zumindest eines Teils der in der genomischen DNA enthaltenen Cytosine in Uracil oder eine andere Base mit einem von Cytosin unterscheidbaren Basenpaarungsverhalten und/oder Molekulargewicht,
B) Durchführen einer Mutationsanalyse mit der aus Schritt A) erhaltenen genomischen DNA, um die mindestens eine Mutation zu bestimmen,
C) Durchführen einer Methylierungsanalyse mit der aus Schritt A) erhaltenen genomischen DNA, um einen Methylierungszustand mindestens eines in der genomischen DNA enthaltenden CpG-Dinukleotids zu bestimmen,
D) Korrelieren von An- oder Abwesenheit der Mutation mit dem Methylierungszustand des CpG-Dinukleotids, um die Diagnose, Prognose, Prädiktion und/oder den Verlauf der malignen Erkrankung zu bestimmen,
wobei die Mutationsanalyse zumindest einen Teil des *BRAF* Gens und die Methylierungsanalyse zumindest einen Teil des *SHOX2* Gens umfasst; oder
wobei die Mutationsanalyse zumindest einen Teil des *FGFR3, TERT, PIK3CA, KRAS, TP53, NRAS* und/oder *HRAS* Gens und die Methylierungsanalyse zumindest einen Teils des *ONECUT2, OTX1, SHOX2, SEPT9* und/oder *TWIST1* Gens umfasst; oder
wobei die Mutationsanalyse zumindest einen Teil des *TP53* Gens und/oder eine Insertion viraler DNA, insbesondere zumindest eines Teils oder mehrerer Teile der DNA eines oder mehrerer Humaner Papilloma-Viren (HPV) und die Methylierungsanalyse zumindest einen Teil des *SEPT9* Gens umfasst; oder
wobei die Mutationsanalyse zumindest einen Teil des *BRCA1, BRCA2* und/oder *PALB2* Gens und die Methylierungsanalyse zumindest einen Teil des *BRCA1* Gens umfasst; oder
wobei die Mutationsanalyse zumindest einen Teil des *IDH1, IDH2* und/oder *EGFR* Gens und die Methylierungsanalyse zumindest einen Teil des *MGMT* Gens umfasst; oder wo
bei die Mutationsanalyse zumindest einen Teil des *TP53* Gens und die Methylierungsanalyse zumindest einen Teil des *PITX2* Gens umfasst; oder
wobei die Mutationsanalyse zumindest einen Teil des *AR*, *ESR1, BRCA1, BRCA2, PALB2* und/oder *ERBB2* Gens und die Methylierungsanalyse zumindest einen Teil des *PITX2* Gens umfasst.

2. Das Verfahren nach Anspruch 1, wobei die mindestens eine Mutation eine somatische Mutation umfasst.

3. Das Verfahren nach einem der Ansprüche 1 oder 2, wobei die mindestens eine Mutation eine Insertion viraler DNA umfasst.

4. Das Verfahren nach einem der Ansprüche 1 bis 3, wobei die genomische DNA frei zirkulierende DNA und/oder DNA aus frei zirkulierenden Zellen aus einer Körperflüssigkeit umfasst.

5. Ein Verfahren zur Prognose einer malignen Erkrankung und/oder zur Prädiktion eines Ansprechverhaltens einer malignen Erkrankung auf eine Therapie, umfassend eine Bestimmung mindestens einer Mutation in genomischer DNA, mit den Schritten
A) Umwandeln zumindest eines Teils der in der genomischen DNA enthaltenen Cytosine in Uracil oder eine andere Base mit einem von Cytosin unterscheidbaren Basenpaarungsverhalten und/oder Molekulargewicht,
B) Durchführen einer Mutationsanalyse mit der aus Schritt A) erhaltenen genomischen DNA, um die mindestens eine Mutation zu bestimmen,
C) Durchführen einer Methylierungsanalyse mit der aus Schritt A) erhaltenen genomischen DNA, um einen Methylierungszustand mindestens eines in der genomischen DNA enthaltenden CpG-Dinukleotids zu bestimmen,
D) Korrelieren von An- oder Abwesenheit der Mutation mit dem Methylierungszustand des CpG-Dinukleotids, um die Prognose und/oder das Ansprechverhalten der malignen Erkrankung auf die Therapie zu bestimmen,
wobei die Mutationsanalyse eine Mutation eines Gens ausgewählt aus *BRAF, EGFR, KRAS, NRAS, AKT1, IDH1, IDH2, TSC1, NF1, DNMT3A, EZH2, TERT, SMO, FLT3, JAK2, BCR, SMAD4, MAP2K1, PIK3CA, PTEN, DDR2, NTRK1, RET, KIT, ALK, FGFR1, MET, FGFR1, FGFR2, BCR-ABL1, RET, MEK, mTOR, VEGFR* und beliebige Kombinationen dieser Gene und die Methylierungsanalyse ein oder mehrere CpG-Dinukleotide des *PITX2* Gens umfasst.

6. Ein Verfahren zur Bestimmung mindestens einer Mutation in genomischer DNA, umfassend
A) Umwandeln zumindest eines Teils der in der genomischen DNA enthaltenen Cytosine in Uracil oder eine andere Base mit einem von Cytosin unterscheidbaren Basenpaarungsverhalten und/oder Molekulargewicht,
B) Durchführen einer Mutationsanalyse mit der aus Schritt A) erhaltenen genomischen DNA, um Anwesenheit oder Abwesenheit der mindestens einen Mutation zu bestimmen,
C) Durchführen einer Methylierungsanalyse mit der aus Schritt A) erhaltenen genomischen DNA, um einen Methylierungszustand mindestens eines in der genomischen DNA enthaltenden CpG-Dinukleotids zu bestimmen und anhand des Methylierungszustandes des CpG-Dinukleotids Anwesenheit oder Abwesenheit von DNA einer malignen Erkrankung in der genomischen DNA zu bestimmen,
D) Korrelieren der in Schritt C) bestimmten Anwesenheit oder Abwesenheit von DNA der malignen Erkrankung mit der in Schritt B) bestimmten Anwesenheit oder Abwesenheit der Mutation.

7. Ein Verfahren zur Normalisierung und/oder internen Standardisierung einer Mutationsanalyse und/oder Methylierungsanalyse, umfassend eine Bestimmung mindestens einer Mutation in genomischer DNA, weiter umfassend
A) Umwandeln zumindest eines Teils der in der genomischen DNA enthaltenen Cytosine in Uracil oder eine andere Base mit einem von Cytosin unterscheidbaren Basenpaarungsverhalten und/oder Molekulargewicht,
B) Durchführen einer Mutationsanalyse mit der aus Schritt A) erhaltenen genomischen DNA unter Bedingungen, welche eine quantitative Bestimmung der mindestens einen Mutation erlauben, und Bestimmen eines Anteils der genomischen DNA, welcher die Mutation enthält,
C) Durchführen einer Methylierungsanalyse mit der aus Schritt A) erhaltenen genomischen DNA unter Bedingungen, welche eine quantitative Bestimmung eines Methylierungszustands mindestens eines in der genomischen DNA enthaltenen CpG-Dinukleotids erlauben, und Bestimmen eines Anteils der genomischen DNA, in welchem das CpG-Dinukleotids methyliert vorliegt,
D) Korrelieren des Anteils der genomischen DNA, welcher die Mutation enthält, mit dem Anteil der genomischen DNA, in welchem das CpG-Dinukleotid methyliert vorliegt.

8. Eine Verwendung eines Kits zur Durchführung des Verfahrens nach einem der Ansprüche 1 bis 7, wobei das Kit
a) mindestens ein erstes Oligonukleotid-Paar ausgelegt zur Hybridisierung an die aus Schritt A) erhaltene genomischen DNA, um zumindest einen ersten Teilbereich der umgewandelten genomischen DNA zu amplifizieren, welcher im Verdacht steht, die mindestens eine Mutation zu enthalten
b) mindestens ein zweites Oligonukleotid-Paar ausgelegt zur Hybridisierung an die aus Schritt A) erhaltene genomischen DNA, um zumindest einen zweiten Teilbereich der umgewandelten genomischen DNA zu amplifizieren, welcher das mindestens eine CpG-Dinukleotid beinhaltet, dessen Methylierungszustand zu analysieren ist
umfasst.

## Claims

1. A method for diagnosis, prognosis, prediction and/or monitoring of a malignant disease, comprising determining at least one mutation in genomic DNA, with the steps
A) converting at least a part of the cytosines contained in said genomic DNA to uracil or another base with a base-pairing behavior and/or molecular weight distinguishable from that of cytosine,
B) performing a mutation analysis with said genomic DNA obtained from step A) to determine said at least one mutation,
C) performing a methylation analysis with said genomic DNA obtained from step A) to determine a methylation state of at least one CpG dinucleotide contained in said genomic DNA,
D) correlating presence or absence of said mutation with said methylation state of the CpG dinucleotide to determine the diagnosis, prognosis, prediction and/or monitoring of said malignant disease,
wherein said mutation analysis comprises at least a part of the *BRAF* gene and said methylation analysis comprises at least a part of the *SHOX2* gene; or
wherein said mutation analysis comprises at least a part of the *FGFR3, TERT, PIK3CA, KRAS, TP53, NRAS* and/or *HRAS* gene and said methylation analysis comprises at least a part of the *ONECUT2, OTX1, SHOX2, SEPT9* and/or *TWIST1* gene; or
wherein said mutation analysis comprises at least a part of the *TP53* gene and/or an insertion of viral DNA, in particular at least a part or several parts of DNA of one or more human papillomaviruses (HPV) and said methylation analysis comprises at least a part of the *SEPT9* gene; or wh
erein said mutation analysis comprises at least a part of the *BRCA1, BRCA2* and/or *PALB2* gene and said methylation analysis comprises at least a part of the *BRCA1* gene; or wh
erein said mutation analysis comprises at least a part of the *IDH1, IDH2* and/or *EGFR* gene and said methylation analysis comprises at least a part of the *MGMT* gene; or wh
erein said mutation analysis comprises at least a part of the *TP53* gene and said methylation analysis comprises at least a part of the *PITX2* gene; or
wherein said mutation analysis comprises at least a part of the *AR*, *ESR1, BRCA1, BRCA2, PALB2* and/or *ERBB2* gene and said methylation analysis comprises at least a part of the *PITX2* gene.

2. The method of claim 1, wherein said at least one mutation comprises a somatic mutation.

3. The method of claim 1 or 2, wherein said at least one mutation comprises an insertion of viral DNA.

4. The method of any one of claims 1 to 3, wherein said genomic DNA comprises circulating cell-free DNA and/or DNA from circulating cells from a body fluid.

5. A method for determining prognosis of a malignant disease and/or for predicting response of a malignant disease to therapy, comprising determining at least one mutation in genomic DNA, with the steps
A) converting at least a part of the cytosines contained in said genomic DNA to uracil or another base with a base-pairing behavior and/or molecular weight distinguishable from that of cytosine,
B) performing a mutation analysis with said genomic DNA obtained from step A) to determine said at least one mutation,
C) performing a methylation analysis with said genomic DNA obtained from step A) to determine a methylation state of at least one CpG dinucleotide contained in said genomic DNA,
D) correlating presence or absence of said mutation with said methylation state of the CpG dinucleotide to determine the prognosis and/or to predict the response of said malignant disease to therapy,
wherein said mutation analysis comprises a mutation of a gene selected from *BRAF, EGFR, KRAS, NRAS, AKT1, IDH1, IDH2, TSC1, NF1, DNMT3A, EZH2, TERT, SMO, FLT3, JAK2, BCR, SMAD4, MAP2K1, PIK3CA, PTEN, DDR2, NTRK1, RET, KIT, ALK, FGFR1, MET, FGFR1, FGFR2, BCR-ABL1, RET, MEK, mTOR, VEGFR* and any combination of these genes and said methylation analysis comprises one or more CpG dinucleotides of the *PITX2* gene.

6. A method for determining at least one mutation in genomic DNA, comprising
A) converting at least a part of the cytosines contained in said genomic DNA to uracil or another base with a base-pairing behavior and/or molecular weight distinguishable from that of cytosine,
B) performing a mutation analysis with said genomic DNA obtained from step A) to determine presence or absence of said at least one mutation,
C) performing a methylation analysis with said genomic DNA obtained from step A) to determine a methylation state of at least one CpG dinucleotide contained in said genomic DNA and determining from said methylation state of the CpG dinucleotide presence or absence of DNA of a malignant disease in said genomic DNA,
D) correlating the presence or absence of DNA of the malignant disease determined in step C) with the presence or absence of said mutation determined in step B).

7. A method for normalization and/or internal standardization of a mutation analysis and/or a methylation analysis, comprising determining at least one mutation in genomic DNA, further comprising
A) converting at least a part of the cytosines contained in said genomic DNA to uracil or another base with a base-pairing behavior and/or molecular weight distinguishable from that of cytosine,
B) performing a mutation analysis with said genomic DNA obtained from step A) under conditions which permit a quantitative determination of said at least one mutation, and determining a proportion of said genomic DNA which contains said mutation,
C) performing a methylation analysis with said genomic DNA obtained from step A) under conditions which permit a quantitative determination of a methylation state of at least one CpG dinucleotide contained in said genomic DNA, and determining a proportion of said genomic DNA in which said CpG dinucleotide is methylated,
D) correlating said proportion of the genomic DNA containing said mutation with said proportion of the genomic DNA in which said CpG dinucleotide is methylated.

8. Use of a kit for carrying out said method of any one of claims 1 to 7, wherein the kit comprises
a) at least a first pair of oligonucleotides designed to hybridize to the genomic DNA obtained from step A) for amplifying at least a first part of the converted genomic DNA which is suspected of containing said at least one mutation,
b) at least a second pair of oligonucleotides designed to hybridize to the genomic DNA obtained from step A) for amplifying at least a second part of the converted genomic DNA which contains said at least one CpG dinucleotide whose methylation state is to be analyzed.

## Revendications

1. Procédé pour le diagnostic, le pronostic, la prédiction et/ou la surveillance d'une maladie maligne, comprenant une détermination d'au moins une mutation dans l'ADN génomique, comportant les étapes suivantes :
A) la conversion d'au moins une partie des cytosines contenues dans l'ADN génomique en uracile ou en une autre base avec un comportement d'appariement de bases et/ou un poids moléculaire différent de la cytosine,
B) la réalisation d'une analyse de mutation avec l'ADN génomique obtenu à l'étape A) afin de déterminer l'au moins une mutation,
C) la réalisation d'une analyse de méthylation sur l'ADN génomique obtenu à l'étape A) afin de déterminer un état de méthylation d'au moins un dinucléotide CpG contenu dans l'ADN génomique,
D) la corrélation de la présence ou de l'absence de la mutation avec l'état de méthylation du dinucléotide CpG afin de déterminer le diagnostic, le pronostic, la prédiction et/ou l'évolution de la maladie maligne,
dans lequel l'analyse de mutation comprend au moins une partie du gène *BRAF* et l'analyse de méthylation comprend au moins une partie du gène *SH0X2 ;* ou
dans lequel l'analyse de mutation comprend au moins une partie du gène *FGFR3, TERT, PIK3CA, KRAS, TP53, NRAS* et/ou *HRAS* et l'analyse de méthylation comprend au moins une partie du gène *ONECUT2, 0TX1, SH0X2, SEPT9* et/ou *TWIST1* ; ou dan
s lequel l'analyse de mutation comprend au moins une partie du gène *TP53* et/ou une insertion d'ADN viral, en particulier au moins une partie ou plusieurs parties de l'ADN d'un ou de plusieurs papillomavirus humains (HPV) et l'analyse de méthylation comprend au moins une partie du gène *SEPT9* ; ou
dans lequel l'analyse de mutation comprend au moins une partie du gène *BRCA1, BRCA2* et/ou *PALB2* et l'analyse de méthylation comprend au moins une partie du gène *BRCA1 ;* ou
dans lequel l'analyse de mutation comprend au moins une partie du gène *IDH1, IDH2* et/ou *EGFR* et l'analyse de méthylation comprend au moins une partie du gène *MGMT* ; ou
dans lequel l'analyse de mutation comprend au moins une partie du gène *TP53* et l'analyse de méthylation comprend au moins une partie du gène *PITX2* ; ou
dans lequel l'analyse de mutation comprend au moins une partie du gène *AR*, *ESR1, BRCA1, BRCA2, PALB2* et/ou *ERBB2* et l'analyse de méthylation comprend au moins une partie du gène *PITX2.*

2. Procédé selon la revendication 1, dans lequel l'au moins une mutation comprend une mutation somatique.

3. Procédé selon la revendication 1 ou 2, dans lequel l'au moins une mutation comprend une insertion d'ADN viral.

4. Procédé selon l'une quelconque des revendications 1 à 3, dans lequel l'ADN génomique comprend de l'ADN circulant librement et/ou de l'ADN de cellules circulant librement à partir d'un fluide corporel.

5. Procédé pour le pronostic d'une maladie maligne et/ou pour la prédiction d'une réponse d'une maladie maligne à une thérapie, comprenant une détermination d'au moins une mutation dans l'ADN génomique, comportant les étapes suivantes :
A) la conversion d'au moins une partie des cytosines contenues dans l'ADN génomique en uracile ou en une autre base avec un comportement d'appariement de bases et/ou un poids moléculaire différent de la cytosine,
B) la réalisation d'une analyse de mutation avec l'ADN génomique obtenu à l'étape A) afin de déterminer l'au moins une mutation,
C) la réalisation d'une analyse de méthylation sur l'ADN génomique obtenu à l'étape A) afin de déterminer un état de méthylation d'au moins un dinucléotide CpG contenu dans l'ADN génomique,
D) la corrélation de la présence ou de l'absence de la mutation avec l'état de méthylation du dinucléotide CpG pour déterminer le pronostic et/ou la réponse de la maladie maligne à la thérapie,
dans lequel l'analyse de mutation comprend une mutation d'un gène choisi parmi *BRAF, EGFR, KRAS, NRAS, AKT1, IDH1, IDH2, TSC1, NF1, DNMT3A, EZH2, TERT, SMO, FLT3, JAK2, BCR, SMAD4, MAP2K1, PIK3CA, PTEN, DDR2, NTRK1, RET, KIT, ALK, FGFR1, MET, FGFR1, FGFR2, BCR-ABL1, RET, MEK, mTOR, VEGFR* et toute combinaison de ces gènes et l'analyse de méthylation comprend un ou plusieurs dinucléotides CpG du gène *PITX2.*

6. Procédé de détermination d'au moins une mutation dans l'ADN génomique, comprenant
A) la conversion d'au moins une partie des cytosines contenues dans l'ADN génomique en uracile ou en une autre base avec un comportement d'appariement de bases et/ou un poids moléculaire différent de la cytosine,
B) la réalisation d'une analyse de mutation sur l'ADN génomique obtenu à l'étape A) pour déterminer la présence ou l'absence de l'au moins une mutation,
C) la réalisation d'une analyse de méthylation avec l'ADN génomique obtenu à l'étape A) afin de déterminer un état de méthylation d'au moins un dinucléotide CpG contenu dans l'ADN génomique et de déterminer la présence ou l'absence d'ADN d'une maladie maligne dans l'ADN génomique sur la base de l'état de méthylation du dinucléotide CpG,
D) la corrélation de la présence ou de l'absence d'ADN de la maladie maligne déterminée à l'étape C) avec la présence ou l'absence de la mutation déterminée à l'étape B).

7. Procédé de normalisation et/ou de standardisation interne d'une analyse de mutation et/ou d'une analyse de méthylation, comprenant une détermination d'au moins une mutation dans l'ADN génomique, comprenant en outre
A) la conversion d'au moins une partie des cytosines contenues dans l'ADN génomique en uracile ou en une autre base avec un comportement d'appariement de bases et/ou un poids moléculaire différent de la cytosine,
B) la réalisation d'une analyse de mutation avec l'ADN génomique obtenu à l'étape A) dans des conditions qui permettent une détermination quantitative de l'au moins une mutation, et la détermination d'une proportion de l'ADN génomique qui contient la mutation,
C) la réalisation d'une analyse de méthylation avec l'ADN génomique obtenu à l'étape A) dans des conditions permettant une détermination quantitative d'un état de méthylation d'au moins un dinucléotide CpG contenu dans l'ADN génomique, et la détermination d'une proportion de l'ADN génomique dans laquelle le dinucléotide CpG est méthylé,
D) la corrélation de la proportion de l'ADN génomique contenant la mutation avec la proportion de l'ADN génomique dans laquelle le dinucléotide CpG est méthylé.

8. Utilisation d'un kit pour la réalisation du procédé selon l'une quelconque des revendications 1 à 7, ledit kit comprenant
A) au moins une première paire d'oligonucléotides destinée à l'hybridation à l'ADN génomique obtenu à l'étape A) afin d'amplifier au moins une première région partielle de l'ADN génomique converti, qui est suspectée de contenir l'au moins une mutation,
B) au moins un second couple d'oligonucléotides destiné à l'hybridation à l'ADN génomique obtenu à l'étape A) afin d'amplifier au moins une seconde région partielle de l'ADN génomique converti, qui comprend l'au moins un dinucléotide CpG dont l'état de méthylation est à analyser.
